# EUROPÄISCHE PATENTSCHRIFT

(11) **EP 1 196 545 B1**
(45) Veröffentlichungstag und Bekanntmachung des Hinweises auf die Patenterteilung: **11.10.2006**
(21) Anmeldenummer: 00951460.5
(22) Anmeldetag: 27.07.2000
(51) Int. Cl.: C12N 9/00

(54) **ELEKTRONENDONORSYSTEM FÜR ENZYME UND DESSEN ANWENDUNG BEI DER BIOCHEMISCHEN UMSETZUNG VON SUBSTRATEN**
ELECTRON DONOR SYSTEM FOR ENZYMES AND ITS USE FOR THE BIOCHEMICAL CONVERSION OF SUBSTRATES
SYSTEME DONNEUR D'ELECTRONS POUR ENZYMES ET SON UTILISATION POUR LA TRANSFORMATION BIOCHIMIQUE DE SUBSTRATS

(30) Priorität: 27.07.1999 DE 19935115; 10.03.2000 DE 10011723
(43) Veröffentlichungstag der Anmeldung: 17.04.2002
(73) Patentinhaber: BASF Aktiengesellschaft, 67056 Ludwigshafen (DE)
(72) Erfinder: HAUER, Bernhard, D-67056 Fussgönheim (DE); SCHMID, Rolf, D., D-70569 Stuttgart (DE); SCHWANEBERG, Ulrich, D-71336 Waiblingen (DE)
(74) Vertreter: Kinzebach, Werner
(86) Internationale Anmeldenummer: PCT/EP2000/007251
(87) Internationale Veröffentlichungsnummer: WO 2001/007573

(56) Entgegenhaltungen:
- WO-A-97/49832
- GB-A- 2 312 960
- FAULKNER KEVIN M ET AL: "Electrocatalytically driven omega-hydroxylation of fatty acids using cytochrome P450 4A1." PROCEEDINGS OF THE NATIONAL ACADEMY OF SCIENCES OF THE UNITED STATES, Bd. 92, Nr. 17, 1995, Seiten 7705-7709, XP002156161 1995 ISSN: 0027-8424
- IWUOHA E I ET AL: "DRUG METABOLISM BIOSENSORS: ELECTROCHEMICAL REACTIVITIES OF CYTOCHROME P450CAM IMMOBILISED IN SYNTHETIC VESICULAR SYSTEMS" JOURNAL OF PHARMACEUTICAL AND BIOMEDICAL ANALYSIS,US,NEW YORK, NY, Bd. 17, Nr. 6/07, 1998, Seiten 1101-1110, XP000874063 ISSN: 0731-7085
- SCHWANEBERG ULRICH ET AL: "A continuous spectrophotometric assay for P450 BM-3, a fatty acid hydroxylating enzyme, and its mutant F87A." ANALYTICAL BIOCHEMISTRY, Bd. 269, Nr. 2, 1. Mai 1999 (1999-05-01), Seiten 359-366, XP002156030 ISSN: 0003-2697
- DATABASE BIOSIS [Online] BIOSCIENCES INFORMATION SERVICE, PHILADELPHIA, PA, US; 1996 FANG XIAOQJUN ET AL: "Dithionite-supported hydroxylation of palmitic acid by cytochrome P450BM-3." Database accession no. PREV199799317033 XP002156162 in der Anmeldung erwähnt & DRUG METABOLISM AND DISPOSITION, Bd. 24, Nr. 11, 1996, Seiten 1282-1285, ISSN: 0090-9556
- GRAHAM-LORENCE SANDRA ET AL: "An active site substitution, F87V, converts cytochrome P450 BM-3 into a regio- and stereoselective (14S,15R)-arachidonic acid epoxygenase." JOURNAL OF BIOLOGICAL CHEMISTRY, Bd. 272, Nr. 2, 1997, Seiten 1127-1135, XP002155914 ISSN: 0021-9258
- OLIVER CATHERINE F ET AL: "A single mutation in cytochrome P450 BM3 changes substrate orientation in a catalytic intermediate and the regiospecificity of hydroxylation." BIOCHEMISTRY, Bd. 36, Nr. 7, 1997, Seiten 1567-1572, XP002131694 ISSN: 0006-2960 in der Anmeldung erwähnt
- OLIVER CATHERINE F ET AL: "Engineering the substrate specificity of Bacillus megaterium cytochrome P-450 BM3: Hydroxylation of alkyl trimethylammonium compounds." BIOCHEMICAL JOURNAL, Bd. 327, Nr. 2, 1997, Seiten 537-544, XP002156031 ISSN: 0264-6021

### SEQUENZPROTOKOLL

<110> BASF Aktiengesellschaft
<120> Elektronendonorsystem für Enzyme und dessen Anwendung bei der biochemischen Umsetzung von Substraten
<130> M/40076
<140>
   <141>
<160> 35
<170> PatentIn Ver. 2.1
<210> 1
   <211> 36
   <212> DNA
   <213> Künstliche Sequenz
<220>
   <223> Beschreibung der künstlichen Sequenz: Oligonukleotid für genomische Bacillus megaterium-DNA
<400> 1
   gtgaaagagg gatcccatga caattaaaga aatgcc 36
<210> 2
   <211> 38
   <212> DNA
   <213> Künstliche Sequenz
<220>
   <223> Beschreibung der künstlichen Sequenz: Oligonukleotid für genomische Bacillus megaterium-DNA
<400> 2
   gcctcttgga tccttaccca gcccacacgt cttttgcg 38
<210> 3
   <211> 19
   <212> DNA
   <213> Künstliche Sequenz
<220>
   <223> Beschreibung der künstlichen Sequenz: Primer für Sequenzierung
<400> 3
   gtacgtgatt ttgcaggag 19
<210> 4
   <211> 20
   <212> DNA
   <213> Künstliche Sequenz
<220>
   <223> Beschreibung der künstlichen Sequenz: Primer für Sequenzierung
<400> 4
   ggctatcatg cgatgatggt 20
<210> 5
   <211> 19
   <212> DNA
   <213> Künstliche Sequenz
<220>
   <223> Beschreibung der künstlichen Sequenz: Primer für Sequenzierung
<400> 5
   cccagcttat gatgaaaac 19
<210> 6
   <211> 20
   <212> DNA
   <213> Künstliche Sequenz
<220>
   <223> Beschreibung der künstlichen Sequenz: Primer für Sequenzierung
<400> 6
   ggaaaagatc cagaaacggg 20
<210> 7
   <211> 19
   <212> DNA
   <213> Künstliche Sequenz
<220>
   <223> Beschreibung der künstlichen Sequenz: Primer für Sequenzierung
<400> 7
   gtcggcatgg tcttaaacg 19
<210> 8
   <211> 20
   <212> DNA
   <213> Künstliche Sequenz
<220>
   <223> Beschreibung der künstlichen Sequenz: Primer für Sequenzierung
<400> 8
   attcctcagc ttcaccgtga 20
<210> 9
   <211> 19
   <212> DNA
   <213> Künstliche Sequenz
<220>
   <223> Beschreibung der künstlichen Sequenz: Primer für Sequenzierung
<400> 9
   cttggcggta ttccttcac 19
<210> 10
   <211> 20
   <212> DNA
   <213> Künstliche Sequenz
<220>
   <223> Beschreibung der künstlichen Sequenz: Primer für Sequenzierung
<400> 10
   atttgcaccg caggtcgcaa 20
<210> 11
   <211> 17
   <212> DNA
   <213> Künstliche Sequenz
<220>
   <223> Beschreibung der künstlichen Sequenz: Primer für Sequenzierung
<400> 11
   ctgggctact acgtatc 17
<210> 12
   <211> 20
   <212> DNA
   <213> Künstliche Sequenz
<220>
   <223> Beschreibung der künstlichen Sequenz: Primer für Sequenzierung
<400> 12
   ttcaatttgt cgacagcgcc 20
<210> 13
   <211> 19
   <212> DNA
   <213> Künstliche Sequenz
<220>
   <223> Beschreibung der künstlichen Sequenz: Primer für Sequenzierung
<400> 13
   gaaggagatc atttaggtg 19
<210> 14
   <211> 20
   <212> DNA
   <213> Künstliche Sequenz
<220>
   <223> Beschreibung der künstlichen Sequenz: Primer für Sequenzierung
<400> 14
   tcgcgcaatg gctgctaaaa 20
<210> 15
   <211> 18
   <212> DNA
   <213> Künstliche Sequenz
<220>
   <223> Beschreibung der künstlichen Sequenz: Primer für Sequenzierung
<400> 15
   cgatttcttc atcacctc 18
<210> 16
   <211> 20
   <212> DNA
   <213> Künstliche Sequenz
<220>
   <223> Beschreibung der künstlichen Sequenz: Primer für Sequenzierung
<400> 16
   ctgccaaaag accctgaaac 20
<210> 17
   <211> 20
   <212> DNA
   <213> Künstliche Sequenz
<220>
   <223> Beschreibung der künstlichen Sequenz: Primer für Sequenzierung
<400> 17
   accatcatcg catgatagcc 20
<210> 18
   <211> 20
   <212> DNA
   <213> Künstliche Sequenz
<220>
   <223> Beschreibung der künstlichen Sequenz: Primer für Sequenzierung
<400> 18
   cccgtttctg gatcttttcc 20
<210> 19
   <211> 20
   <212> DNA
   <213> Künstliche Sequenz
<220>
   <223> Beschreibung der künstlichen Sequenz: Primer für Sequenzierung
<400> 19
   tcacggtgaa gctgaggaat 20
<210> 20
   <211> 20
   <212> DNA
   <213> Künstliche Sequenz
<220>
   <223> Beschreibung der künstlichen Sequenz: Primer für Sequenzierung
<400> 20
   ttgcgacctg cggtgcaaat 20
<210> 21
   <211> 20
   <212> DNA
   <213> Künstliche Sequenz
<220>
   <223> Beschreibung der künstlichen Sequenz: Primer für Sequenzierung
<400> 21
   ggcgctgtcg acaaattgaa 20
<210> 22
   <211> 20
   <212> DNA
   <213> Künstliche Sequenz
<220>
   <223> Beschreibung der künstlichen Sequenz: Primer für Sequenzierung
<400> 22
   ttttagcagc cattgcgcga 20
<210> 23
   <211> 20
   <212> DNA
   <213> Künstliche Sequenz
<220>
   <223> Beschreibung der künstlichen Sequenz: Primer für Sequenzierung
<400> 23
   gtttcagggt cttttggcag 20
<210> 24
   <211> 36
   <212> DNA
   <213> Künstliche Sequenz
<220>
   <223> Beschreibung der künstlichen Sequenz: Oligonukleotid für Tag am C-Terminus
<400> 24
   gtgaaagagg gatcccatga caattaaaga aatgcc 36
<210> 25
   <211> 42
   <212> DNA
   <213> Künstliche Sequenz
<220>
   <223> Beschreibung der künstlichen Sequenz: Oligonukleotid für Tag am C-Terminus
<400> 25
   cggaattctt aacgacgacg acgacgacgc ccagcccaca cg 42
<210> 26
   <211> 36
   <212> DNA
   <213> Künstliche Sequenz
<220>
   <223> Beschreibung der künstlichen Sequenz: Oligonukleotid für Tag am C-Terminus
<400> 26
   gtgaaagagg gatcccatga caattaaaga aatgcc 36
<210> 27
   <211> 42
   <212> DNA
   <213> Künstliche Sequenz
<220>
   <223> Beschreibung der künstlichen Sequenz: Oligonukleotid für Tag am C-Terminus
<400> 27
   cggaattctt attcttcttc ttcttcttcc ccagcccaca cg 42
<210> 28
   <211> 36
   <212> DNA
   <213> Künstliche Sequenz
<220>
   <223> Beschreibung der künstlichen Sequenz: Oligonukleotid für Tag am C-Terminus
<400> 28
   gtgaaagagg gatcccatga caattaaaga aatgcc 36
<210> 29
   <211> 42
   <212> DNA
   <213> Künstliche Sequenz
<220>
   <223> Beschreibung der künstlichen Sequenz: Oligonukleotid für Tag am C-Terminus
<400> 29
   cgcaattctt aatgatgatg atgatgatgc ccagcccaca cg 42
<210> 30
   <211> 38
   <212> DNA
   <213> Künstliche Sequenz
<220>
   <223> Beschreibung der künstlichen Sequenz: Oligonukleotid für Tag am C-Terminus
<400> 30
   gtctcagcgt gagaccccca gcccacacgt cttttgcc 38
<210> 31
   <211> 36
   <212> DNA
   <213> Künstliche Sequenz
<220>
   <223> Beschreibung der künstlichen Sequenz: Oligonukleotid für Tag am C-Terminus
<400> 31
   gtgaaagagg tctccaatga caattaaaga aatgcc 36
<210> 32
   <211> 34
   <212> DNA
   <213> Künstliche Sequenz
<220>
   <223> Beschreibung der künstlichen Sequenz: Primer für P450 BM-3 Punktmutante F87A
<400> 32
   gcaggagacg ggttggccac aagctggacg catg 34
<210> 33
   <211> 34
   <212> DNA
   <213> Künstliche Sequenz
<220>
   <223> Beschreibung der künstlichen Sequenz: Primer für P450 BM-3 Punktmutante F87A
<400> 33
   catgcgtcca gcttgtggcc aacccgtctc ctgc 34
<210> 34
   <211> 3150
   <212> DNA
   <213> Bacillus megaterium
<220>
   <221> CDS
   <222> (4)..(3150)
<400> 34
   atg aca att aaa gaa atg cct cag cca aaa acg ttt gga gag ctt aaa    48
       Thr Ile Lys Glu Met Pro Gln Pro Lys Thr Phe Gly Glu Leu Lys
         1               5                  10                  15
   aat tta ccg tta tta aac aca gat aaa ccg gtt caa gct ttg atg aaa    96
   Asn Leu Pro Leu Leu Asn Thr Asp Lys Pro Val Gln Ala Leu Met Lys
                    20                  25                  30
   att gcg gat gaa tta gga gaa atc ttt aaa ttc gag gcg cct ggt cgt   144
   Ile Ala Asp Glu Leu Gly Glu Ile Phe Lys Phe Glu Ala Pro Gly Arg
                   35                  40                  45
   gta acg cgc tac tta tca agt cag cgt cta att aaa gaa gca tgc gat      192
   Val Thr Arg Tyr Leu Ser Ser Gln Arg Leu Ile Lys Glu Ala Cys Asp
               50                  55                  60
   gaa tca cgc ttt gat aaa aac tta agt caa gcg ctt aaa ttt gta cgt      240
   Glu Ser Arg Phe Asp Lys Asn Leu Ser Gln Ala Leu Lys Phe Val Arg
           65                  70                  75
   gat ttt gca gga gac ggg tta ttt aca agc tgg acg cat gaa aaa aat      288
   Asp Phe Ala Gly Asp Gly Leu Phe Thr Ser Trp Thr His Glu Lys Asn
    80                  85                  90                  95
   tgg aaa aaa gcg cat aat atc tta ctt cca agc ttc agt cag cag gca      336
   Trp Lys Lys Ala His Asn Ile Leu Leu Pro Ser Phe Ser Gln Gln Ala
                   100                 105                 110
   atg aaa ggc tat cat gcg atg atg gtc gat atc gcc gtg cag ctt gtt      384
   Met Lys Gly Tyr His Ala Met Met Val Asp Ile Ala Val Gln Leu Val
               115                 120                 125
   caa aag tgg gag cgt cta aat gca gat gag cat att gaa gta ccg gaa      432
   Gln Lys Trp Glu Arg Leu Asn Ala Asp Glu His Ile Glu Val Pro Glu
           130                 135                 140
   gac atg aca cgt tta acg ctt gat aca att ggt ctt tgc ggc ttt aac      480
   Asp Met Thr Arg Leu Thr Leu Asp Thr Ile Gly Leu Cys Gly Phe Asn
       145                 150                 155
   tat cgc ttt aac agc ttt tac cga gat cag cct cat cca ttt att aca      528
   Tyr Arg Phe Asn Ser Phe Tyr Arg Asp Gln Pro His Pro Phe Ile Thr
   160                 165                 170                 175
   agt atg gtc cgt gca ctg gat gaa gca atg aac aag ctg cag cga gca      576
   Ser Met Val Arg Ala Leu Asp Glu Ala Met Asn Lys Leu Gln Arg Ala
                   180                 185                 190
   aat cca gac gac cca gct tat gat gaa aac aag cgc cag ttt caa gaa      624
   Asn Pro Asp Asp Pro Ala Tyr Asp Glu Asn Lys Arg Gln Phe Gln Glu
               195                 200                 205
   gat atc aag gtg atg aac gac cta gta gat aaa att att gca gat cgc      672
   Asp Ile Lys Val Met Asn Asp Leu Val Asp Lys Ile Ile Ala Asp Arg
           210                 215                 220
   aaa gca agc ggt gaa caa agc gat gat tta tta acg cat atg cta aac      720
   Lys Ala Ser Gly Glu Gln Ser Asp Asp Leu Leu Thr His Met Leu Asn
       225                 230                 235
   gga aaa gat cca gaa acg ggt gag ccg ctt gat gac gag aac att cgc      768
   Gly Lys Asp Pro Glu Thr Gly Glu Pro Leu Asp Asp Glu Asn Ile Arg
   240                 245                 250                 255
   tat caa att att aca ttc tta att gcg gga cac gaa aca aca agt ggt     816
   Tyr Gln Ile Ile Thr Phe Leu Ile Ala Gly His Glu Thr Thr Ser Gly
                   260                 265                 270
   ctt tta tca ttt gcg ctg tat ttc tta gtg aaa aat cca cat gta tta     864
   Leu Leu Ser Phe Ala Leu Tyr Phe Leu Val Lys Asn Pro His Val Leu
               275                 280                 285
   caa aaa gca gca gaa gaa gca gca cga gtt cta gta gat cct gtt cca     912
   Gln Lys Ala Ala Glu Glu Ala Ala Arg Val Leu Val Asp Pro Val Pro
           290                 295                 300
   agc tac aaa caa gtc aaa cag ctt aaa tat gtc ggc atg gtc tta aac     960
   Ser Tyr Lys Gln Val Lys Gln Leu Lys Tyr Val Gly Met Val Leu Asn
       305                 310                 315
   gaa gcg ctg cgc tta tgg cca act gct cct gcg ttt tcc cta tat gca    1008
   Glu Ala Leu Arg Leu Trp Pro Thr Ala Pro Ala Phe Ser Leu Tyr Ala
   320                 325                 330                 335
   aaa gaa gat acg gtg ctt gga gga gaa tat cct tta gaa aaa ggc gac    1056
   Lys Glu Asp Thr Val Leu Gly Gly Glu Tyr Pro Leu Glu Lys Gly Asp
                   340                 345                 350
   gaa cta atg gtt ctg att cct cag ctt cac cgt gat aaa aca att tgg    1104
   Glu Leu Met Val Leu Ile Pro Gln Leu His Arg Asp Lys Thr Ile Trp
               355                 360                 365
   gga gac gat gtg gaa gag ttc cgt cca gag cgt ttt gaa aat cca agt    1152
   Gly Asp Asp Val Glu Glu Phe Arg Pro Glu Arg Phe Glu Asn Pro Ser
           370                 375                 380
   gcg att ccg cag cat gcg ttt aaa ccg ttt gga aac ggt cag cgt gcg    1200
   Ala Ile Pro Gln His Ala Phe Lys Pro Phe Gly Asn Gly Gln Arg Ala
       385                 390                 395
   tgt atc ggt cag cag ttc gct ctt cat gaa gca acg ctg gta ctt ggt    1248
   Cys Ile Gly Gln Gln Phe Ala Leu His Glu Ala Thr Leu Val Leu Gly
   400                 405                 410                 415
   atg atg cta aaa cac ttt gac ttt gaa gat cat aca aac tac gag ctg    1296
   Met Met Leu Lys His Phe Asp Phe Glu Asp His Thr Asn Tyr Glu Leu
                   420                 425                 430
   gat att aaa gaa act tta acg tta aaa cct gaa ggc ttt gtg gta aaa    1344
   Asp Ile Lys Glu Thr Leu Thr Leu Lys Pro Glu Gly Phe Val Val Lys
               435                 440                 445
   gca aaa tcg aaa aaa att ccg ctt ggc ggt att cct tca cct agc act    1392
   Ala Lys Ser Lys Lys Ile Pro Leu Gly Gly Ile Pro Ser Pro Ser Thr
           450                 455                 460
   gaa cag tct gct aaa aaa gta cgc aaa aag gca gaa aac gct cat aat     1440
   Glu Gln Ser Ala Lys Lys Val Arg Lys Lys Ala Glu Asn Ala His Asn
       465                 470                 475
   acg ccg ctg ctt gtg cta tac ggt tca aat atg gga aca gct gaa gga     1488
   Thr Pro Leu Leu Val Leu Tyr Gly Ser Asn Met Gly Thr Ala Glu Gly
   480                 485                 490                 495
   acg gcg cgt gat tta gca gat att gca atg agc aaa gga ttt gca ccg     1536
   Thr Ala Arg Asp Leu Ala Asp Ile Ala Met Ser Lys Gly Phe Ala Pro
                   500                 505                 510
   cag gtc gca acg ctt gat tca cac gcc gga aat ctt ccg cgc gaa gga     1584
   Gln Val Ala Thr Leu Asp Ser His Ala Gly Asn Leu Pro Arg Glu Gly
               515                 520                 525
   gct gta tta att gta acg gcg tct tat aac ggt cat ccg cct gat aac     1632
   Ala Val Leu Ile Val Thr Ala Ser Tyr Asn Gly His Pro Pro Asp Asn
           530                 535                 540
   gca aag caa ttt gtc gac tgg tta gac caa gcg tct gct gat gaa gta     1680
   Ala Lys Gln Phe Val Asp Trp Leu Asp Gln Ala Ser Ala Asp Glu Val
       545                 550                 555
   aaa ggc gtt cgc tac tcc gta ttt gga tgc ggc gat aaa aac tgg gct     1728
   Lys Gly Val Arg Tyr Ser Val Phe Gly Cys Gly Asp Lys Asn Trp Ala
   560                 565                 570                 575
   act acg tat caa aaa gtg cct gct ttt atc gat gaa acg ctt gcc gct     1776
   Thr Thr Tyr Gln Lys Val Pro Ala Phe Ile Asp Glu Thr Leu Ala Ala
                   580                 585                 590
   aaa ggg gca gaa aac atc gct gac cgc ggt gaa gca gat gca agc gac     1824
   Lys Gly Ala Glu Asn Ile Ala Asp Arg Gly Glu Ala Asp Ala Ser Asp
               595                 600                 605
   gac ttt gaa ggc aca tat gaa gaa tgg cgt gaa cat atg tgg agt gac     1872
   Asp Phe Glu Gly Thr Tyr Glu Glu Trp Arg Glu His Met Trp Ser Asp
           610                 615                 620
   gta gca gcc tac ttt aac ctc gac att gaa aac agt gaa gat aat aaa     1920
   Val Ala Ala Tyr Phe Asn Leu Asp Ile Glu Asn Ser Glu Asp Asn Lys
       625                 630                 635
   tct act ctt tca ctt caa ttt gtc gac agc gcc gcg gat atg ccg ctt     1968
   Ser Thr Leu Ser Leu Gln Phe Val Asp Ser Ala Ala Asp Met Pro Leu
   640                 645                 650                 655
   gcg aaa atg cac ggt gcg ttt tca acg aac gtc gta gca agc aaa gaa     2016
   Ala Lys Met His Gly Ala Phe Ser Thr Asn Val Val Ala Ser Lys Glu
                   660                 665                 670
   ctt caa cag cca ggc agt gca cga agc acg cga cat ctt gaa att gaa    2064
   Leu Gln Gln Pro Gly Ser Ala Arg Ser Thr Arg His Leu Glu Ile Glu
               675                 680                 685
   ctt cca aaa gaa gct tct tat caa gaa gga gat cat tta ggt gtt att    2112
   Leu Pro Lys Glu Ala Ser Tyr Gln Glu Gly Asp His Leu Gly Val Ile
           690                 695                 700
   cct cgc aac tat gaa gga ata gta aac cgt gta aca gca agg ttc ggc    2160
   Pro Arg Asn Tyr Glu Gly Ile Val Asn Arg Val Thr Ala Arg Phe Gly
       705                 710                 715
   cta gat gca tca cag caa atc cgt ctg gaa gca gaa gaa gaa aaa tta    2208
   Leu Asp Ala Ser Gln Gln Ile Arg Leu Glu Ala Glu Glu Glu Lys Leu
   720                 725                 730                 735
   gct cat ttg cca ctc gct aaa aca gta tcc gta gaa gag ctt ctg caa    2256
   Ala His Leu Pro Leu Ala Lys Thr Val Ser Val Glu Glu Leu Leu Gln
                   740                 745                 750
   tac gtg gag ctt caa gat cct gtt acg cgc acg cag ctt cgc gca atg    2304
   Tyr Val Glu Leu Gln Asp Pro Val Thr Arg Thr Gln Leu Arg Ala Met
               755                 760                 765
   gct gct aaa acg gtc tgc ccg ccg cat aaa gta gag ctt gaa gcc ttg    2352
   Ala Ala Lys Thr Val Cys Pro Pro His Lys Val Glu Leu Glu Ala Leu
           770                 775                 780
   ctt gaa aag caa gcc tac aaa gaa caa gtg ctg gca aaa cgt tta aca    2400
   Leu Glu Lys Gln Ala Tyr Lys Glu Gln Val Leu Ala Lys Arg Leu Thr
       785                 790                 795
   atg ctt gaa ctg ctt gaa aaa tac ccg gcg tgt gaa atg aaa ttc agc    2448
   Met Leu Glu Leu Leu Glu Lys Tyr Pro Ala Cys Glu Met Lys Phe Ser
   800                 805                 810                 815
   gaa ttt atc gcc ctt ctg cca agc ata cgc ccg cgc tat tac tcg att    2496
   Glu Phe Ile Ala Leu Leu Pro Ser Ile Arg Pro Arg Tyr Tyr Ser Ile
                   820                 825                 830
   tct tca tca cct cgt gtc gat gaa aaa caa gca agc atc acg gtc agc    2544
   Ser Ser Ser Pro Arg Val Asp Glu Lys Gln Ala Ser Ile Thr Val Ser
               835                 840                 845
   gtt gtc tca gga gaa gcg tgg agc gga tat gga gaa tat aaa gga att    2592
   Val Val Ser Gly Glu Ala Trp Ser Gly Tyr Gly Glu Tyr Lys Gly Ile
           850                 855                 860
   gcg tcg aac tat ctt gcc gag ctg caa gaa gga gat acg att acg tgc    2640
   Ala Ser Asn Tyr Leu Ala Glu Leu Gln Glu Gly Asp Thr Ile Thr Cys
       865                 870                 875
   ttt att tcc aca ccg cag tca gaa ttt acg ctg cca aaa gac cct gaa      2688
   Phe Ile Ser Thr Pro Gln Ser Glu Phe Thr Leu Pro Lys Asp Pro Glu
   880                 885                 890                 895
   acg ccg ctt atc atg gtc gga ccg gga aca ggc gtc gcg ccg ttt aga      2736
   Thr Pro Leu Ile Met Val Gly Pro Gly Thr Gly Val Ala Pro Phe Arg
                   900                 905                 910
   ggc ttt gtg cag gcg cgc aaa cag cta aaa gaa caa gga cag tca ctt      2784
   Gly Phe Val Gln Ala Arg Lys Gln Leu Lys Glu Gln Gly Gln Ser Leu
               915                 920                 925
   gga gaa gca cat tta tac ttc ggc tgc cgt tca cct cat gaa gac tat      2832
   Gly Glu Ala His Leu Tyr Phe Gly Cys Arg Ser Pro His Glu Asp Tyr
           930                 935                 940
   ctg tat caa gaa gag ctt gaa aac gcc caa agc gaa ggc atc att acg      2880
   Leu Tyr Gln Glu Glu Leu Glu Asn Ala Gln Ser Glu Gly Ile Ile Thr
       945                 950                 955
   ctt cat acc gct ttt tct cgc atg cca aat cag ccg aaa aca tac gtt      2928
   Leu His Thr Ala Phe Ser Arg Met Pro Asn Gln Pro Lys Thr Tyr Val
   960                 965                 970                 975
   cag cac gta atg gaa caa gac ggc aag aaa ttg att gaa ctt ctt gat      2976
   Gln His Val Met Glu Gln Asp Gly Lys Lys Leu Ile Glu Leu Leu Asp
                   980                 985                 990
   caa gga gcg cac ttc tat att tgc gga gac gga agc caa atg gca cct      3024
   Gln Gly Ala His Phe Tyr Ile Cys Gly Asp Gly Ser Gln Met Ala Pro
               995                 1000                1005
   gcc gtt gaa gca acg ctt atg aaa agc tat gct gac gtt cac caa gtg      3072
   Ala Val Glu Ala Thr Leu Met Lys Ser Tyr Ala Asp Val His Gln Val
           1010                1015                1020
   agt gaa gca gac gct cgc tta tgg ctg cag cag cta gaa gaa aaa ggc      3120
   Ser Glu Ala Asp Ala Arg Leu Trp Leu Gln Gln Leu Glu Glu Lys Gly
       1025                1030                1035
   cga tac gca aaa gac gtg tgg gct ggg taa                              3150
   Arg Tyr Ala Lys Asp Val Trp Ala Gly
   1040                1045
<210> 35
   <211> 1048
   <212> PRT
   <213> Bacillus megaterium
<400> 35
   Thr Ile Lys Glu Met Pro Gln Pro Lys Thr Phe Gly Glu Leu Lys Asn
    1               5                  10                  15
   Leu Pro Leu Leu Asn Thr Asp Lys Pro Val Gln Ala Leu Met Lys Ile
               20                  25                  30
   Ala Asp Glu Leu Gly Glu Ile Phe Lys Phe Glu Ala Pro Gly Arg Val
           35                  40                  45
   Thr Arg Tyr Leu Ser Ser Gln Arg Leu Ile Lys Glu Ala Cys Asp Glu
       50                  55                  60
   Ser Arg Phe Asp Lys Asn Leu Ser Gln Ala Leu Lys Phe Val Arg Asp
   65                  70                  75                  80
   Phe Ala Gly Asp Gly Leu Phe Thr Ser Trp Thr His Glu Lys Asn Trp
                   85                  90                  95
   Lys Lys Ala His Asn Ile Leu Leu Pro Ser Phe Ser Gln Gln Ala Met
               100                 105                 110
   Lys Gly Tyr His Ala Met Met Val Asp Ile Ala Val Gln Leu Val Gln
           115                 120                 125
   Lys Trp Glu Arg Leu Asn Ala Asp Glu His Ile Glu Val Pro Glu Asp
       130                 135                 140
   Met Thr Arg Leu Thr Leu Asp Thr Ile Gly Leu Cys Gly Phe Asn Tyr
   145                 150                 155                 160
   Arg Phe Asn Ser Phe Tyr Arg Asp Gln Pro His Pro Phe Ile Thr Ser
                   165                 170                 175
   Met Val Arg Ala Leu Asp Glu Ala Met Asn Lys Leu Gln Arg Ala Asn
               180                 185                 190
   Pro Asp Asp Pro Ala Tyr Asp Glu Asn Lys Arg Gln Phe Gln Glu Asp
           195                 200                 205
   Ile Lys Val Met Asn Asp Leu Val Asp Lys Ile Ile Ala Asp Arg Lys
       210                 215                 220
   Ala Ser Gly Glu Gln Ser Asp Asp Leu Leu Thr His Met Leu Asn Gly
   225                 230                 235                 240
   Lys Asp Pro Glu Thr Gly Glu Pro Leu Asp Asp Glu Asn Ile Arg Tyr
                   245                 250                 255
   Gln Ile Ile Thr Phe Leu Ile Ala Gly His Glu Thr Thr Ser Gly Leu
               260                 265                 270
   Leu Ser Phe Ala Leu Tyr Phe Leu Val Lys Asn Pro His Val Leu Gln
   275 280 285
   Lys Ala Ala Glu Glu Ala Ala Arg Val Leu Val Asp Pro Val Pro Ser
   290 295 300
   Tyr Lys Gln Val Lys Gln Leu Lys Tyr Val Gly Met Val Leu Asn Glu
   305 310 315 320
   Ala Leu Arg Leu Trp Pro Thr Ala Pro Ala Phe Ser Leu Tyr Ala Lys
   325 330 335
   Glu Asp Thr Val Leu Gly Gly Glu Tyr Pro Leu Glu Lys Gly Asp Glu
   340 345 350
   Leu Met Val Leu Ile Pro Gln Leu His Arg Asp Lys Thr Ile Trp Gly
   355 360 365
   Asp Asp Val Glu Glu Phe Arg Pro Glu Arg Phe Glu Asn Pro Ser Ala
   370 375 380
   Ile Pro Gln His Ala Phe Lys Pro Phe Gly Asn Gly Gln Arg Ala Cys
   385 390 395 400
   Ile Gly Gln Gln Phe Ala Leu His Glu Ala Thr Leu Val Leu Gly Met
   405 410 415
   Met Leu Lys His Phe Asp Phe Glu Asp His Thr Asn Tyr Glu Leu Asp
   420 425 430
   Ile Lys Glu Thr Leu Thr Leu Lys Pro Glu Gly Phe Val Val Lys Ala
   435 440 445
   Lys Ser Lys Lys Ile Pro Leu Gly Gly Ile Pro Ser Pro Ser Thr Glu
   450 455 460
   Gln Ser Ala Lys Lys Val Arg Lys Lys Ala Glu Asn Ala His Asn Thr
   465 470 475 480
   Pro Leu Leu Val Leu Tyr Gly Ser Asn Met Gly Thr Ala Glu Gly Thr
   485 490 495
   Ala Arg Asp Leu Ala Asp Ile Ala Met Ser Lys Gly Phe Ala Pro Gln
   500 505 510
   Val Ala Thr Leu Asp Ser His Ala Gly Asn Leu Pro Arg Glu Gly Ala
   515 520 525
   Val Leu Ile Val Thr Ala Ser Tyr Asn Gly His Pro Pro Asp Asn Ala
   530 535 540
   Lys Gln Phe Val Asp Trp Leu Asp Gln Ala Ser Ala Asp Glu Val Lys
   545 550 555 560
   Gly Val Arg Tyr Ser Val Phe Gly Cys Gly Asp Lys Asn Trp Ala Thr
   565 570 575
   Thr Tyr Gln Lys Val Pro Ala Phe Ile Asp Glu Thr Leu Ala Ala Lys
   580 585 590
   Gly Ala Glu Asn Ile Ala Asp Arg Gly Glu Ala Asp Ala Ser Asp Asp
   595 600 605
   Phe Glu Gly Thr Tyr Glu Glu Trp Arg Glu His Met Trp Ser Asp Val
   610 615 620
   Ala Ala Tyr Phe Asn Leu Asp Ile Glu Asn Ser Glu Asp Asn Lys Ser
   625 630 635 640
   Thr Leu Ser Leu Gln Phe Val Asp Ser Ala Ala Asp Met Pro Leu Ala
   645 650 655
   Lys Met His Gly Ala Phe Ser Thr Asn Val Val Ala Ser Lys Glu Leu
   660 665 670
   Gln Gln Pro Gly Ser Ala Arg Ser Thr Arg His Leu Glu Ile Glu Leu
   675 680 685
   Pro Lys Glu Ala Ser Tyr Gln Glu Gly Asp His Leu Gly Val Ile Pro
   690 695 700
   Arg Asn Tyr Glu Gly Ile Val Asn Arg Val Thr Ala Arg Phe Gly Leu
   705 710 715 720
   Asp Ala Ser Gln Gln Ile Arg Leu Glu Ala Glu Glu Glu Lys Leu Ala
   725 730 735
   His Leu Pro Leu Ala Lys Thr Val Ser Val Glu Glu Leu Leu Gln Tyr
   740 745 750
   Val Glu Leu Gln Asp Pro Val Thr Arg Thr Gln Leu Arg Ala Met Ala
   755 760 765
   Ala Lys Thr Val Cys Pro Pro His Lys Val Glu Leu Glu Ala Leu Leu
   770 775 780
   Glu Lys Gln Ala Tyr Lys Glu Gln Val Leu Ala Lys Arg Leu Thr Met
   785 790 795 800
   Leu Glu Leu Leu Glu Lys Tyr Pro Ala Cys Glu Met Lys Phe Ser Glu
   805 810 815
   Phe Ile Ala Leu Leu Pro Ser Ile Arg Pro Arg Tyr Tyr Ser Ile Ser
           820                 825                 830
   Ser Ser Pro Arg Val Asp Glu Lys Gln Ala Ser Ile Thr Val Ser Val
           835                 840                 845
   Val Ser Gly Glu Ala Trp Ser Gly Tyr Gly Glu Tyr Lys Gly Ile Ala
       850                 855                 860
   Ser Asn Tyr Leu Ala Glu Leu Gln Glu Gly Asp Thr Ile Thr Cys Phe
   865                 870                 875                 880
   Ile Ser Thr Pro Gln Ser Glu Phe Thr Leu Pro Lys Asp Pro Glu Thr
                   885                 890                 895
   Pro Leu Ile Met Val Gly Pro Gly Thr Gly Val Ala Pro Phe Arg Gly
               900                 905                 910
   Phe Val Gln Ala Arg Lys Gln Leu Lys Glu Gln Gly Gln Ser Leu Gly
           915                 920                 925
   Glu Ala His Leu Tyr Phe Gly Cys Arg Ser Pro His Glu Asp Tyr Leu
       930                 935                 940
   Tyr Gln Glu Glu Leu Glu Asn Ala Gln Ser Glu Gly Ile Ile Thr Leu
   945                 950                 955                 960
   His Thr Ala Phe Ser Arg Met Pro Asn Gln Pro Lys Thr Tyr Val Gln
                   965                 970                 975
   His Val Met Glu Gln Asp Gly Lys Lys Leu Ile Glu Leu Leu Asp Gln
               980                 985                 990
   Gly Ala His Phe Tyr Ile Cys Gly Asp Gly Ser Gln Met Ala Pro Ala
           995                1000                1005
   Val Glu Ala Thr Leu Met Lys Ser Tyr Ala Asp Val His Gln Val Ser
      1010                1015                1020
   Glu Ala Asp Ala Arg Leu Trp Leu Gln Gln Leu Glu Glu Lys Gly Arg
   025                1030                1035                1040
   Tyr Ala Lys Asp Val Trp Ala Gly
                  1045

## Beschreibung

Die Erfindung betrifft ein neuartiges Elektronendonorsystem für Cytochrom P450-haltige Enzyme mit Redoxeigenschaften, umfassend eine anorganische, nicht elektrodengebundene Elektronenquelle und einen zur Übertragung von Elektronen von der Elektronenquelle auf die Enzyme befähigten Mediator, wobei die Elektronenquelle ein Metall mit einem niedrigeren Standard-Normalpotential als der Mediator ist, und dessen Verwendung in enzymkatalysierten Oxidationsreaktionen, wie insbesondere der Herstellung ω-hydroxylierter Fettsäuren. Die Erfindung betrifft außerdem ein verbessertes Nachweisverfahren für Fettsäuremonooxygenasen, Bioreaktoren sowie Testkits, worin das Elektronendonorsystem vorteilhaft einsetzbar ist.

Die biotechnologische Nutzung von Enzymen mit Redoxeigenschaften, wie z.B. von Monooxygenasen, in zellfreien Reaktionssystemen ist grundsätzlich mit dem Problem behaftet, daß die Verwendung natürlicher Cofaktoren zur Bereitstellung der erforderlichen Redox-Äquivalente (wie z.B. von NADH oder NADPH) mit unvertretbar hohen Kosten verbunden ist.

Dies gilt auch für die biotechnologische Nutzung von Cytochrom P450-haltigen Monooxygenasen. Funktionell ist allen P450-Enzymen gemeinsam, Sauerstoffatome auf nicht aktivierte aliphatische oder aromatische X-H (X = -C, -N, -S) Bindungen zu übertragen. Darüber hinaus vermögen P450-Enzyme -C=C- Doppelbindung zu epoxidieren. Für diese Oxygenierungsreaktionen benötigen die meisten P450-Systeme Cofaktoren, wie NADPH oder NADH, als Elektronenquelle. Entsprechend der Realisierung dieses Elektronen-Transfersystems (Reduktasesystems) unterteilt man P450-Systeme in vier Klassen. Klasse I P450-Enzyme enthalten als Reduktase eine FAD-Domäne und ein weiteres Fe-S-Protein (zumeist mitochondriale und bakterielle P450-Enzyme), Klasse II P450-Enzyme besitzen eine FAD/FMN-Reduktase (meist ER-P450-Enzyme) und Klasse III P450-Enzyme benötigen keine weiteren Reduktionsäquivalente, sie setzen peroxygenierte Substrate um, die den Sauerstoff bereits enthalten. Das einzige P450-Enzym der Klasse IV erhält seine Elektronen direkt ohne Transfersystem von NADH.

Die funktionelle Vielfalt der durch P450-Systeme katalysierten Monooxygenierungen auf chemischem Wege häufig nur schwer zugänglicher Verbindungen bietet ein enormes biotechnologisches, pharmakologisches und toxikologisches Potential. Eine wichtige Voraussetzung zur *in vitro* Nutzung dieser Potentiale ist die Entwicklung geeigneter Expressions-, Reinigungs- und vor allem Aktivitätsnachweissysteme, die erlauben, P450-Systeme zu charakterisieren und Enzymvarianten mit "verbesserten" Eigenschaften aufzufinden.

Weitere wichtige Voraussetzung für die Nutzung dieser P450-Monooxygenasen, insbesondere der Klassen I und II, wäre die Verfügbarkeit eines kostengünstigen Elektronendonorsystems.

Ein NADPH-Cofaktor-Recycling-System wurde von Deffner et al., Ann. N.Y. Acad. Sci.(1987) 501, 171 vorgestellt. Dieses stellt aber bei Anwendung im präparativen Maßstab ein Kostenproblem dar und gestaltet Reaktionsführungen in Enzym-Membran-Reaktoren komplizierter. Deshalb wurde nach alternativen Elektronendonoren gesucht. Ein vielversprechenden Weg stellt die elektrochemische Reduktion von P450-Enzymen dar. Estabrook et al.(Methods Enzymol. (1996) 272, 44) konnten mittels eines Co(III)sepulchrat-Mediatorsystems und Pt-Elektroden für sechs verschiedene P450-Enzyme Umsätze bestimmen. Die Aktivitäten waren jedoch etwa 8-fach geringer als beim Reduktionsäquivalent NADPH. Statt des Mediators wurde in Natriumdithionit (Fang et al., Drug. Metab. Dispos. (1996) 24(11): 1282) eine Verbindung gefunden, die P450-Enzyme direkt reduziert. Im Falle von P450 BM-3 war die Aktivität mit Natriumdithionit gegenüber NADPH um Faktor 8150 reduziert; vermutlich wird P450 BM-3 durch Natriumdithionit dreifach reduziert und damit großteils inaktiviert.

Eine erste Aufgabe der Erfindung war es daher, ein kostengünstiges, effizientes, alternatives Elektronendonorsystem für Enzyme mit Redoxeigenschaften bereitzustellen. Insbesondere sollte dieses System in biochemischen Umsetzungen verwendbar sein, an welchen Cytochrom P450-haltige Enzyme beteiligt sind. Außerdem sollte ein verbessertes Nachweisverfahren für p450-Enzyme bereitgestellt werden. Eine weitere Aufgabe der Erfindung bestand in der Bereitstellung eines verbesserten biotechnologischen Verfahrens zur enzymatischen Übertragung von Sauerstoff auf organische Moleküle und insbesondere zur terminalen oder subterminalen Hydroxylierung von Hydrocarbylverbindungen mit polarem Gruppe, wie insbesondere Fettsäuren.

Diese Aufgaben wurden überraschenderweise gelöst durch Bereitstellung eines Elektronendonorsystems für die Übertragung von Elektronen auf Cytochrom P450-haltige Enzyme mit Redox-Eigenschaften, das dadurch gekennzeichnet ist, daß das System eine anorganische, nicht elektrodengebundene Elektronenquelle und einen Mediator umfaßt, der zur Übertragung von Elektronen von der Elektronenquelle auf das Enzym befähigt ist, wobei die Elektronenquelle ein Metall mit einem nierigeren Standard-Normalpotential als der Mediator ist. Dabei können die Komponenten des Systems einzeln oder im Gemisch, in geträgerter oder vorzugsweise ungeträgerter Form vorliegen.

Eine "nicht elektrodengebundene Elektronenquelle" im Sinne der Erfindung ist ohne Anlegen einer externen Spannung zwischen einem Elektrodenpaar zur Abgabe von Elektronen an den Mediator befähigt. Insbesondere wird dabei der Mediator durch die Elektronenquelle über eine chemische Redoxreaktion reduziert.

Das erfindungsgemäß bereitgestellte Elektronendonorsystem ist für Cytochrom P450-haltige Enzyme der in der großen Familie der Monooxygenasen (E.C. 1.14.-.-) zusammengefassten Enzyme, anwendbar. Als nicht limitierendes Beispiel sei hier die aus dem Bacillus megaterium isolierbare Cytochrom P450-Monooxygenase BM-3 genannt.

In einer bevorzugten Ausführungsform des Elektronendonorsystems umfaßt dieses einen Mediator mit einem Standard-Normalpotential (z.B. gemessen gegen Kalomel-Elektrode; unter Standardbedingungen; vgl. unten Caesar et al.) im Bereich von weniger als etwa -0,4 V. Nichtlimitierende Beispiele für erfindungsgemäß brauchbare Mediatoren sind Kobalt(III)sepulchrat, Methylviologen, Neutralrot, Riboflavin, Rutheniumtriacetat, FMN und FAD, wobei Kobalt(III)sepulchrat bevorzugt ist. Kobalt(III)sepulchrat besitzt beispielsweise unter Standardbedingungen ein Normalpotenial von -0,54 V (vgl. Caesar et al. (1977), J. Am. Chem. Soc., 99, 3181).

Das erfindungsgemäße Elektronendonorsystem umfaßt als Elektronenquelle ein Metall mit einem niedrigeren Standard-Normalpotential als der Mediator. Als nichtlimitierendes Beispiel für die Elektronenquelle ist metallisches Zink zu nennen. Das Metall liegt dabei vorzugsweise in einer reaktiven Form, d.h. mit großer Oberfläche, wie z.B. pulverförmig, vor.

Bevorzugte erfindungsgemäße Elektronendonorsysteme umfassen folgende Kombinationen von Elektronenquelle und Mediator:
- Zn/Kobalt(III)sepulchrat oder
- Zn/Neutralrot.

Ein weiterer Gegenstand der Erfindung betrifft ein Verfahren zur enzymatischen Oxidation von, d.h. Übertragung von Sauerstoff auf ein Kohlenwasserstoff-haltiges Wasserstoffdonor-Molekül (d.h. eine oxidierbare Verbindung), das dadurch gekennzeichnet ist, daß man das Wasserstoff-Donormolekül in einem Reaktionsmedium, umfassend das Sauerstoff-übertragende Enzym und ein Elektronendonorsystem gemäß obiger Definition, in Gegenwart von Sauerstoff unter Reaktionsbedingungen inkubiert.

Eine "Oxidation" im Sinne der vorliegenden Erfindung betrifft enzymkatalysierte Oxidationsreaktionen, welche von Enzymen aus der Klasse der Monooxygenasen (E.C.1.14.-.-) und vor allem von Cytochrom P450-Enzymen katalysiert werden. Diese Oxidationsreaktionen umfassen:
a) Kohlenstoff-Oxidationen an gesättigten oder ungesättigten, aliphatischen oder aromatischen Kohlenstoffatomen, wie z.B. Hydroxylierungen und Epoxidierungen;
b) Schwefel-Oxidationen;
c) Stickstoff-Oxidationen;
d) oxidative Dealkylierungen; und
e) oxidative Dehalogenierungen.

Dabei ist das Wasserstoff-Donormolekül vorzugsweise ausgewählt unter Verbindungen der Formel

R-X

worin
- R: für einen geradkettigen oder verzweigten, vorzugsweise geradkettigen, Alkylrest mit 8 oder mehr Kohlenstoffatomen, wie z.B. 10 bis 30 Kohlenstoffatomen, steht, und
- X: für eine polare, zur Ausbildung von Wasserstoffbrücken befähigte Gruppe, vorzugsweise eine Carboxy-, Amid-, Nitril, Sulfat-, Sulfon-, Amin- oder Hydroxygruppe, steht.

Eine bevorzugte Variante obigen Verfahrens betrifft die enzymatische Herstellung terminal oder subterminal (d.h. in Position ω-1 bis ω-4) hydroxylierter Fettsäuren, das dadurch gekennzeichnet ist, daß man
a) eine hydroxylierbare Fettsäure oder ein hydroxylierbares Fettsäurederivat in Gegenwart eines Elektronendonorsystems gemäß obiger Definition mit einer Cytochrom P450 Monooxygenase und Sauerstoff umsetzt; und
b) das (die) hydroxylierte(n) Produkt(e) isoliert.

Die ω-hydroxylierbare (d.h. terminal oder subterminal hydroxylierbare) Fettsäure bzw. deren Derivat ist erfindungsgemäß bevorzugt ausgewählt unter bzw. abgeleitet von terminal gesättigten, verzweigten oder unverzweigten Fettsäuren mit 8 oder mehr, wie z.B. mehr als 10 Kohlenstoffatomen, insbesondere C₁₂ - C₃₀-Fettsäuren. Als nichtlimitierende Beispiele für erfindungsgemäß hydroxylierbare Fettsäuren sind diese zu nennen: Capryl-, Pelargon-, Undecan-, Laurin-, Tridecan-, Myristin-, Pentadecan-, Palmitin-, Margarin-, Stearin-, Nonadecan-, Arachin-, Behen-, Lignocerin-, Cerotin- und Melissinsäure.

Beispiele für geeignete Fettsäurederivate sind C₁-C₄-Alkylester, Amide oder Anhydride mit vorzugsweise kurzkettigen, insbesondere C₁-C₄-Carbonsäuren.

In den oben beschriebenen erfindungsgemäßen Verfahren verwendet man als Enzym eine Cytochrom P450 Monooxygenase, die ausgewählt ist unter:
- Cytochrom P450 Monooxygenasen der Enzymklasse E.C. 1.14.-.-, insbesondere aus der P450-Familie CYP102, eukaryotischen oder prokaryotischen, insbesondere bakteriellen Ursprungs, und vorzugsweise dem aus Bacillus megaterium (DSM 32T) isolierbaren Wildtypenzym; oder
- einer Mutante davon.

Eine bevorzugte Gruppe von Mutanten ist abgeleitet von Cytochrom P450 Monooxygenase BM-3 aus *Bacillus megaterium* mit einer Aminosäuresequenz gemäß SEQ ID NO:35, welche wenigstens eine funktionale Mutation in einem der folgenden Aminosäuresequenzbereiche aufweist: 24-28, 45-51, 70-72, 73-82 (helix 5), 86-88 (helix 6), 172-224 (F/G-loop) und 352-356 (β-strand 8); sowie funktionale Äquivalente dieser Mutanten.

Eine "funktionale Mutation" im Sinne der vorliegenden Erfindung umfasst einen Aminosäureaustausch in den genannten Sequenzbereichen, welcher zu einer Mutante führt, die weiterhin zzur Katalyse einer der oben genannten Oxidationsreaktionen wie z.B. Hydroxylierungen befähigt ist.

Besonders bevorzugt sind erfindungsgemäß P450 BM-3-Monooxygenase-Mutanten, welche wenigstens je eine funktionale Mutation in den Aminosäuresequenzbereichen 86-88, 172-224, 73-82, 45-51, 24-28 70-72 und 352-356 auf (gemäß SEQ ID NO: 35) einzeln oder in Kombination umfassen.

Insbesondere kann eine Aminosäuresubstitution in wenigstens einer der Positionen 26, 47, 72, 74, 87, 188 und 354 gemäß SEQ ID NO: 35 des Wildtypenzyms vorliegen.

So kann beispielsweise Phe87 ersetzt sein durch eine Aminosäure mit aliphatischer Seitenkette, wie z. B. Ala, Val, Leu, insbesondere Val oder Ala; Leu188 kann ersetzt sein durch eine Aminosäure mit Amin- oder Amid-Seitenkette, wie z. B. Asn, Gln, Arg oder insbesondere Lys, oder Aminosäuren wie Ala, Gly, Ser und Trp; Ala74 kann ersetzt sein durch eine andere Aminosäure mit aliphatischer Seitenkette, wie z. B. Val und insbesondere Gly; Arg47 kann ersetzt sein durch eine Aminosäure mit ringförmiger Seitengruppe, wie z.B. His, Tyr oder insbesondere Phe; und val26 kann ersetzt sein durch eine Aminosäure mit Hydroxyl-Seitengruppe, wie z.B. Ser oder insbesondere Thr. Beispielsweise kann Ser72 ersetzt sein durch eine Aminosäure mit aliphatischer Seitenkette, wie z. B. Ala, Val, Leu, Lle und insbesondere Gly, und Met354 kann ersetzt sein durch eine Aminosäure mit Hydroxyl-Seitengruppe, wie z.B. Ser oder insbesondere Thr.

Beispiele für Mutanten sind:
a) F87V;
b) F87A, L188K;
c) F87V, L188K;
d) F87A, L188K; A74G;
e) F87V, L188K, A74G;
f) F87A, L188K, A74G, R47F;
g) F87V, L188K, A74G, R47F;
h) F87A, L188K, A74G, R47F, V26T; oder
i) F87V, L188K, A74G, R47F, V26T;
k) V26T,
l) R47F,
m) S72G,
n) A74G,
o) F87A
p) L188z, worin z für K, R, W, Q, N, G, A oder S steht, und
q) M354T;
sowie funktionale Äquivalente davon.

Geeignete Mutanten sind beispielsweise auch beschrieben in der älteren DE-A-100 11 723, worauf hiermit Bezug genommen wird

Die eingesetzten Mutanten und funktionalen Äquivalente können dabei im Vergleich zum Wildtyp-Enzym ein "verändertes Substratprofil" zeigen.

Ein "verändertes Substratprofil" bedeutet in Rahmen der vorliegenden Erfindung insbesondere :
a) eine Veränderung der Reaktivität, wie z. B. eine Erhöhung der spezifischen Aktivität (ausgedrückt als nmol umgesetztes Substrat/Minute/nmol P450-Enzym) und/oder wenigstens eines kinetischen Parameters, ausgewählt unter Kcat, Km und Kcat/Km, z.B. um mindestens 1 %, wie z. B. 10 bis 1000 %, 10 bis 500 %, oder 10 bis 100 %, der Mutante gegenüber zumindest einem oxidierbaren Substrat im Vergleich zum Wildtyp-Enzym; und/oder
b) eine Veränderung der Regioselektivität, wie insbesondere eine Verschiebung der bevorzugten Position der oxidationsreaktion; Dabei kann z.B. die bevorzugte terminale oder subterminale (ω-1, ω-2, ω-3, ω-4, insbesondere ω-1 bis ω-3) Hydroxylierungsposition an wenigstens einer hydroxylierbaren Carbonsäure oder einem hydroxylierbaren Derivat einer aliphatischen Carbonsäure verschoben sein. Die Veränderung des Substratprofils kann dabei über den gesamten Größenbereich (d. h. C₈-C₃₀) oder nur in Teilbereichen, z. B. bei C₈-C₁₂-, C₁₀-C₁₂-, C₁₂-C₃₀-, C₁₂-C₂₅- oder C₁₂-C₂₀-Carbonsäuren oder bei einzelnen Carbonsäuren aus diesen Teilbereichen ausgeprägt sein.

Unter "funktionalen Äquivalenten" versteht man erfindungsgemäß auch Mutanten, welche in wenigstens einer der oben genannten Sequenzpositionen eine andere als die konkret genannte Aminosäuresubstitution aufweisen, aber trotzdem zu einer Mutante führen, die ebenso wie die konkret genannte Mutante eine der oben definierten Oxidationsreaktionen katalysiert.

Funktionale Äquivalenz ist insbesondere auch dann gegeben, wenn die Veränderungen im Reaktivitätsmuster qualitativ übereinstimmen.

"Funktionale Äquivalente" umfassen natürlich auch P450-Monooxygenase-Mutanten, welche in gleicher Weise wie die konkret genannten P450 BM3-Mutanten durch Mutation von P450-Enzymen aus anderen Organismen zugänglich sind. Beispielsweise lassen sich durch Sequenzvergleich Bereiche homologer Sequenzregionen festlegen. Mit den modernen Methoden des Molecular Modeling können dann in Anlehnung an die konkreten Vorgaben der Erfindung äquivalente, das Reaktionsmuster beeinflussende Mutationen vorgenommen werden.

"Funktionale Äquivalente" umfassen ebenso die durch eine oder mehrere zusätzliche Aminosäure-Additionen, -Substituenten, -Deletionen und/oder -Inversionen erhältlichen Mutanten, wobei die genannten zusätzlichen Veränderungen in jeglicher Sequenzposition auftreten können, solange sie zu einer Mutante mit "verändertem Substratprofil" im obigen Sinne führen.

Bevorzugt eingesetzte Mutanten umfassen eine Mutation in Position 87 und sind ausgewählt unter den Mutationen F87A und F87V die gegebenenfalls wenigstens eine weitere der folgenden Mutationen aufweist: L188K, A74G, R47F und V26T. Besonders bevorzugt ist die Einfachmutante F87A und die Fünffachmutante F87A, L188K, A74G, R47F, V26T (Angabe der Aminosäuren im Einbuchstaben-Code; ursprüngliche Aminosäure links von der Positionsangabe; neue Aminosäure rechts von der Positionsangabe).

Vorzugsweise werden obige Verfahren unter Verwendung des Elektronendonorsystem Zink/Co(III)sepulchrat durchgeführt.

In einer bevorzugten Ausführungsform des Verfahrens führte man die Umsetzung in Gegenwart von Chloridionen durch.

Außerdem kann die Gegenwart eines Wasserstoffperoxyd-spaltenden Enzyms, wie z.B. Katalase, vorteilhaft sein.

Eine weiterer Gegenstand der Erfindung betrifft einen Bioreaktor zur Verwendung bei der enzymatischen Übertragung von Sauerstoff auf ein Kohlenwasserstoff-haltiges Wasserstoffdonor-Molekül, und insbesondere zur Verwendung bei der Herstellung ω-hydroxylierter Fettsäuren, der gekennzeichnet ist durch eine immobilisierte Monooxygenase (z.B. immobilisiert an einem Trägermaterial , wie z.B. DEAE 650M oder Super Q650M) und ein Elektronendonorsystem gemäß obiger Definition im flüssigen Reaktionsmedium. Die spezielle Ausgestaltung des Reaktors erfolgt in Anlehnung an üblichem Fachwissen.

Ein weiterer Gegenstand der Erfindung betrifft ein Nachweisverfahren für Fettsäure-Monooxygenasen, das dadurch gekennzeichnet ist, daß man
a) einen Analyten, in dem man Enzymaktivität vermutet, mit einer ω-hydroxylierbaren Fettsäure oder Fettsäurederivat, welche(s) einen terminalen, abspaltbaren Fluorophors oder Chromophor trägt, in Gegenwart eines Elektronendonorsystems gemäß obiger Definition inkubiert; und
b) die Abspaltung des Fluorophors oder Chromophors qualitativ oder quantitativ bestimmt.

Als Analyt kommt beispielsweise ein Zellhomogenat, z.B. eines Bakterienstammes, in Betracht. Mit Hilfe des erfindungsgemäßen Nachweisverfahrens können so z.B. Stammsammlungen auf Enzymaktivität durchmustert werden.

Die Reaktion führt man dabei bevorzugt in Gegenwart eines Wasserstoffperoxyd-spaltenden Enzyms und gegebenenfalls in Gegenwart von Chloridionen durch.

Beispiele für geeignete Fluorophore und Chromophore sind Phenole, Catechole, Hydroxycoumarine, wie Umbelliferon, Phenoxazine und insbesondere Resorufin.

Die Einstellung der optimalen Verfahrensparameter für die oben beschriebenen enzymatischen Umsetzungen und Nachweisreaktionen ist unter Berücksichtigung der Anweisungen im folgenden experimentellen Teil ohne weiteres möglich. So ist z.B., ohne darauf beschränkt zu sein, eine Cobalt(III)sepulchrat-Konzentration von mehr als 0,1 mM, wie z.B. 0,5 bis 1,0 mM besonders vorteilhaft; ebenso wie ein pH-Wert etwa 8 bis 8,5; eine Zink-Konzentration von mehr als etwa 2mg/ml wie z.B. etwa 5 bis 50 mg/ml. Das Wasserstoffperoxid-spaltende Enzym kann z.B. in Mengen von etwa 10 bis 5000 U/ml eingesetzt werden.

Schließlich betrifft die Erfindung als weiteren Gegenstand einen Testkit, insbesondere zur Durchführung des obigen Nachweisverfahrens für Carbonsäure-Monoxygenasen, umfassend ein Elektronendonorsystem nach obiger Definition.

Die vielfältigen Anwendungsmöglichkeiten der vorliegenden Erfindung werden in den folgenden Abschnitten weiter erläutert.

### Katalysierte Reaktionen von P450-Systemen und Beispiele industrieller Anwendungen in der Feinchemikaliensynthese:

In Organismen dienen Cytochrom P450-Enzyme unter anderem der Ergosterol-Synthese, der Biosynthese von Insekten- und Phytohormonen, der Ausbildung von Fruchtreife, Geruch und Farbe bei Pflanzen, der Biosynthese von Gluko- und Mineralkortikoiden, der Aromatisierung von Androgenen zu Östrogenen, dem Metabolismus von Retinoiden zur Regulation von normalem und epithelialem Wachstum/Differenzierung, Arachidonsäurestoffwechsel zur Bildung von Prostaglandinen, Leukotrienen und Thromboxanen und der Bildung vasoaktiver Produkte. Darüber hinaus dienen sie zur Aktivierung und Detoxifizierung xenobiotischer Stoffe.

P450-Enzyme gehören zu den sogenannten Phase I Enzymen, die mittels Oxygenierungen wasserunlösliche oder schwer lösliche Verbindungen zur weiteren Metabolisierung vorbereiten. Dazu dienen Phase II Enzyme wie Glutathion-Transferasen, N-Acetyltransferase oder Sulfotransferase, die eine polare Gruppe an jene hydroxylierten oder epoxidierten Verbindungen addieren. Dadurch werden diese Metabolite wasserlöslich und somit bioverfügbar. Die Vielfalt von Reaktionen, die durch Cytochrom P450-Enzyme katalysiert werden, sind im folgenden zusammengefaßt und hinsichtlich ihrer wirtschaftlichen und funktionellen Bedeutung diskutiert. Eine Übersicht, aufgeschlüsselt anhand ausgewählter Familien findet sich in Tabelle 1.

**Tabelle 1**

| P450-Familie | Reaktionen von P450 Enzymen |
|---|---|
| CYP1-3 | Metabolisierung xenobiotischer Stoffe; |
| | CYP1: PCB, PAH, Dioxine, Aflatoxine; |
| | CYP2: Pflanzentoxine, Pestizide; |
| | CYP3: Cyclosporine, Erythromycin, ferner beteiligt am Steroidmetabolismus |
| CYP4, CYP52, CYP102 | CYP4, CYP52, CYP102: terminale und/oder subterminale Oxidation von Fettsäuren |
| | CYP4: Oxidation von Eicosanoiden |
| | (Prostaglandinen, Leukotrienen und Thromboxanen) |
| CYP11, CYP17, CYP21 | Biosynthese von Gluko- und Mineralcorticoiden; |
| | CYP11: 11β-Hydroxylasen, Hydroxylierung von Deoxycorticosteron oder Deoxycortisol; |
| | CYP17: Steroid-17α-hydroxylase; |
| | CYP21: Steroid-21-hydroxylase |
| CYP19 | Umsetzung von Steroiden zu Östrogenen; Aromatase |
| CYP27 | Cholesterol-27-hydroxylase |

Die P450-Enzyme der Enzymfamilien CYP1-3 sind hauptverantwortlich für die Metabolisierung xenobiotischer Stoffe; die dabei auftretenden oxidativen Reaktionen sind im folgenden unterteilt in C-, S-, N-Oxidation, Dealkylierung und Dehalogenierung.

### a) C-Oxidationen

P450-Enzyme hydroxylieren oder epoxidieren nichtaktivierte C-H-Bindungen und/oder C=C-Doppelbindungen in aliphatischen und aromatischen Systemen . Die gebildeten Metabolite sind häufig toxisch, wie das Nervengift 2,5-Hexandion oder karzinogen wie DNA-alkylierende Epoxide (z. B. Styroloxid). Ferner wirken durch P450-Enzyme C-oxidierte Nitrosamine und Benzpyrene karzinogen.

Arzneimittel, wie Barbiturate und Phenobarbitale, werden ebenfalls durch P450-Enzyme in ihre Wirkform überführt. Die Hydroxylierung nicht aktivierter -C-H Bindungen ist wegen der seltenen Konkurrenz durch chemische Verfahren eine der nützlichsten Biotransformationsreaktionen. Im Gegensatz zu Radikalreaktionen zeigen Bio-Hydroxylierungsreaktionen an C-Atomen folgende Aktivitätsreihe: sekundär> tertiär> primär. Bekannte industriell durchgeführte Verfahren sind die Progesteron-Hydroxylierung an 11α-Position mittels *Aspergillus* niger, die etwa die Hälfte der 37 konventionellen Schritte erübrigte, und die 7β-Hydroxylierung von 3α-Hydroxy-5β-cholsäure durch *Fusarium equiseti*. Das Produkt Ursodesoxycholsäure vermag Cholesterin aufzulösen und wird in der Behandlung von Gallensteinen eingesetzt. In der asymmetrischen Synthese werden Biotransformationen unter anderem zur Synthese von β-Hydroxybuttersäure, einem Ausgangsmaterial für Vitamin-synthesen (α-Tocopherol), von Geschmacksstoffen, von Antibiotika (Calcimycin) und von chiralen verzweigten epoxidierten Alkenen verwendet.

### b) N-Oxidation / S-Oxidation

P450-Enzyme hydroxylieren Aryl- und Acetylamine wie Benzidin, 4-Biphenylamin und 2-Acetaminofluoren und vergleichbare Heterozyklen, die beim Anbrennen von Nahrungsmitteln entstehen, am N-Atom. Nach weiterer Acetylierung oder Sulfonierung der Hydroxygruppen entstehen elektrophile, stark karzinogen wirkende Substanzen.

Aryl- und Alkylthioether werden durch P450-Monooxygenasen direkt am S-Atom zu Sulfoxiden oxidiert. Die Oxidation bleibt jedoch häufig nicht auf der Stufe des Sulfoxids stehen; viele Sulfoxide werden zu den entsprechenden Sulfonverbindungen weiteroxidiert, die häufig zelltoxisch wirken. Mikrobielle Oxidationen von chiralen Dithioacetalen gelingen mit hohem Enantiomerenüberschuß mit *Corynebacterium equi* und *Helminthosporium sp.*

### c) Oxidative Dealkylierungen

P450-Enzyme katalysieren auch die oxidative O-, N- und S-Dealkylierung. Beispiele dieser wichtigen Reaktionen sind die O-Dealkylierung von Codein, Mescalin, Phenacetin, die N-Dealkylierung von Ephedrin, Methamphetamin, Aminopurin und die S-Dealkylierung von 6-Methylthiopurin zu 6-Methylpurin.

### d) Oxidative Deaminierung

P450-Enzyme katalysieren auch die oxidative Deaminierung von Aminen. Diese Reaktion hat primär eine Entgiftungsfunktion für den Organismus. Histamin, Noradrenalin und Mescalin können unter anderem auf diese Weise deaminiert werden.

### e) Oxidative Dehalogenierung

Viele halogenierte Alkane und Alkene bilden nach Hydroxylierung an einer -C-H Bindung ein instabiles Intermediat, das in ein Aldehyd oder Keton und H-Hal dissoziert. Beispiele sind die Oxidation von Ethylendibromid zu 2-Bromacetaldehyd und die Oxidation von Chloroform zu Phosgen. Mono- und dihalogeniertes Methan bildet im Vergleich zu dreifach halogeniertem Chloroform nur schwach zelltoxische Metabolite.

Eine mechanistische Beschreibung der vorgestellten P450-Reaktionstypen findet sich in dem Übersichtsartikel von Koymans et al., in Xenobiotica,(1993) 23(6):633.

### In vivo / in vitro Nutzung von P450-Monooxygenasen zur Feinchemikaliensynthese:

Im Gegensatz zur mikrobiellen Biotransformation beschränkt sich die Verwendung rekombinant exprimierter P450-Monooxygenasen zur Feinchemikaliensynthese trotz enormer Potentiale auf wenige *in vivo* Verfahren wie die Herstellung von Dicarbonsäuren als Zwischenstufen für Geruchsstoffe, Steroiden wie Progesteron und subterminal hydroxylierter gesättigter und ungesättigter Fettsäuren als Vorstufen für Riechstoffe (Laktone), Polymere und Darreichungsformen für Medikamente.

Die Verwendung von P450 Monooxygenaseenzymen *in vitro* zur Feinchemikaliensynthese ist bisher noch nicht literaturbekannt. Um P450-Monooxygenasen zur Feinchemikaliensynthese zu verwenden, müssen Expressionssysteme vorhanden sein, die es erlauben, funktionell aktives P450 über Artgrenzen hinweg in hohen Ausbeuten zu exprimieren. Ferner muß das Enzym eine ausreichend hohe Aktivität und Stabilität (Temperaturstabilität, Lösungsmittelstabilität, Oxidationsstabilität) besitzen. Die produzierten Metabolite sollten für das Protein und den Wirt nicht toxisch sein. Für Klasse I und Klasse II sollte ferner ein geeignetes Cofaktor-Recyclingsystem vorhanden sein. Die vorliegende Erfindung schafft hierzu die Voraussetzungen.

### Das P450 BM-3 Monooxygenasesystem:

P450 BM-3 (CYP102) wurde erstmals im Jahre 1986 als dritte P450-Monooxygenase aus *Bacillus megaterium* in E. coli funktionell aktiv exprimiert und charakterisiert. Mit einem Molekulargewicht von 118 kDa war P450 BM-3 das erste bekannte wasserlösliche natürliche Fusionsprotein, das alle drei Domänen (FAD, FMN, P450) in einer einzigen Polypeptidkette enthält. Im Jahre 1993 gelang es, die P450-Domäne ohne Substrate (Ravichandran et al., Science (1993) 261, 731) und im Jahre 1997 mit Substrat (Li et al. Nature Structural Biology (1997), 4(2): 140) zu kristallisieren. P450 BM-3 dient aufgrund seiner Sequenzhomologie zu eukaryontischen P450 und mangels eukaryontischer P450-Kristallstrukturen als bevorzugtes Strukturmodell für diese P450-Enzyme.

P450 BM-3 ist eine Fettsäurehydroxylase, die Carbonsäuren, Alkohole, Amide, Alkylammoniumverbindungen ab Kettenlänge C12 bis zu C22 subterminal hydroxyliert. Die Regiospezifität der Hydroxylierung hängt, wie in Tabelle 2 gezeigt, stark von der Kettenlänge der Fettsäure ab.

**Tabelle 2**

| Substrate | ω-Hydroxy-lierung [%] | ω-1 Hydroxy-lierung [%] | ω-2 Hydroxy-lierung [%] | ω-3 Hydroxy-lierung [%] |
|---|---|---|---|---|
| Laurinsäure (12:0) | - | **48** | 26 | 26 |
| Myristinsäure (14:0) | - | **58** | 21 | 21 |
| Palmitinsäure (16:0) | - | 20 | **48** | 31 |

Die Erfindung wird nun unter Bezugnahme auf die beiliegenden Figuren und die folgenden Ausführungsbeispiele, welche die Anwendung des erfindungsgemäßen Donorsystems auf die Hydroxylierung von Fettsäuren mit Hilfe von P450 Enzymen betreffen, näher erlautert. Dabei zeigt
- Figur 1: das P450 BM3-Klonierungsschema am Beispiel von pCYTEXP1;
- Figur 2: das Prinzip des erfindungsgemäßen spektroskopischen P450-Nachweises;
- Figur 3: das Absorptionsspektrum für die Reduktion von Cobalt(III)sepulchrat mit Zn-Pulver in Tris/HCl, 0,2 M, pH 8,2; gepunktete Linie Co(III)sepulchrat oxidiert; durchgezogenen Linie Zn/Co(III)sepulchrat reduziert;
- Figur 4: die Absorptionsänderung bei Umsatz von 12-pNCA durch P450 BM-3 F87A mit Zn/Co(III)sepulchrat in Tris/HCl, 50 mM, pH 8,2, 0,25 M KCl;
- Figur 5: eine Skizze des Elektronentransferwegs von Zn/Co(III)sepulchrat auf P450 mit "shunt"-Reaktionsweg und pNCA als Substrat;
- Figur 6: den Umsatz von 12-PCA durch P450 BM-3 F87A und Zn/Co(III)sepulchrat in Tris/HCl, 50 mM, pH 8,2, 0,25 M KCl mit und ohne Zugabe von Katalase;
- Figur 7: den Einfluß der Tris/HCl- und Kaliumphosphat-Puffermischung auf die reduktion von p-Nitrophenolat in Gegenwart von P450 BM-3 F87A und Zn/Co(III)sepulchrat;
- Figur 8: (A) 12-pNCA Umsatz durch P450 BM-3 F87A als Funktion der Co(III)sepulchrat-Konzentration; (B) den Einfluß von Co(III)sepulchrat auf die Aktivität von P450 BM-3 F87A in Gegenwart von Substrat;
- Figur 9: den 12-pNCA Umsatz als Funktion der eingesetzten zinkpulvermenge in Gegenwart von 0,5 mM Co(III) sepulchrat;
- Figur 10: die P450 BM-3 F87A Restaktivität nach 5 min Inkubation bei verschiedenen Wasserstoffperoxidkonzentrationen ohne Substrat.

### Referenzbeispiel 1: Mikrobiologische Methoden

### 1. Mikroorganismen und Plasmide, Chemikalien und Enzyme

Das Bakterium *Bacillus megaterium* wurde bei DSMZ-Stammsammlung (32^{T}, Braunschweig, Deutschland), die *E.coli-*Stämme DH5α, JM105 und JM109 bei Clontech (Heidelberg, Deutschland) und XL1-Blue bei Stratagene (Heidelberg, Deutschland) erworben. Den Stamm W3110 erhielten wir vom Institut für Bioverfahrenstechnik (Stuttgart, Deutschland). Der *E.coli*-Stamm DH5α (supE44, lacU169 [80lacZ M15] hsdR17 recA1 endA1 gyrA96 thi-1 relA1) wurde, sofern nicht anders erwähnt, für die folgenden beschriebenen Klonierungsarbeiten verwendet. Als Plasmide fanden pCYTEXP1 mit dem temperaturinduzierbaren P_{R}P_{L}-Promotorsystem des Bakteriophagen λ (Belev T.N., et al., Plasmid (1991) 26:147) und pASK-IBA1CA mit Tetrazyklin-Promotor (Schmidt, T.G.M., et al., J. Chromatogr. A (1994) 676:337; Schmidt, T.G.M. et al., J.Mol.Biol. (1996) 255:753) Verwendung. Die im Rahmen dieser Arbeit zur heterologen Expression von P450 BM-3 verwendeten Stämme und Plasmide sind in Tabelle 3 zusammengefasst, wobei pT die Herkunft der P450 BM-3 Konstrukte vom pCYTEXP1-Plasmid und pA die Herkunft vom pASK-IBA1CA-Plasmid kennzeichnet.

**Tabelle 3**

| E.coli-Stamm/Plasmid-Insert-Konstrukt | exprimiertes Protein |
|---|---|
| DH5α/pT-USC0BM3,W3110/pT-USC0BM3 | P450 BM-3 |
| DHSα/pT-USC1BM3, JM105/pT-USC1BM3, | P450 BM-3His₆ |
| JM109/pT-USC1BM3, W3110/pT-USC1BM3 | |
| DH5α/pT-USC1BM3F87A, JM105/pT-USC1BM3F87A, | P450 BM-3His₆ |
| JM109/pT-USC1BM3F87A, W3110/pT-USC1BM3F87A | F87A Mutante |
| XL1-Blue/pT-USC1BM3F87A | |
| DH5α/pT-USC2BM3, W3110/pT-USC2BM3 | P450 BM-3Glu₆ |
| DH5α/pT-USC3BM3, W3110/pT-USC3MB3 | P450 BM-3Arg₆ |
| DH5α/pA-USC4BM3, JM105/pA-USC4BM3, | P450 BM-3Strep |
| JM109/pA-USC4BM3, W3110/pA-USC4BM3 | |

Sofern nichts anderes erwähnt ist, wurden alle Chemikalien von Fluka Chemie (Buchs, Schweiz) und alle Enzyme von New England Biolabs (Beverly, USA) oder Boehringer Mannheim (Penzberg, Deutschland) bezogen.

### 2. Kulturmedien

Alle Komplexmedien stammen von Difco (Augsburg, Deutschland).
a) Nutrient-Medium (Vollmedium für *Bacillus megaterium*)

| | |
|---|---|
| Peptone | 5,0 g |
| Bacto beef | 3,0 g |
| Bacto peptone | 5,0 g |
| Agar | 15 g (nur für Plattenkultur) |
| MnSO₄*H₂O | 10 mg |
| H₂O | ad 1000 ml |
| | |

| Glycerinkultur | |
|---|---|
| Glycerin (86 %) | 500 µl |
| Kultur(OD₅₇₈= 1,0) | 500 µl |

b) Luria-Bertani-Amp (LB-Amp, Vollmedium für E. coli)

| | |
|---|---|
| Tryptone | 10,0 g |
| NaCl | 5,0 g |
| Yeast extract | 5,0 g |
| Agar | 20,0 g (nur für Plattenkultur) |
| Ampicillin | 100 g/ml |
| H₂O | ad 1000 ml |

### 3. Kultivierung der E. coli-Stämme und von Bacillus megaterium

### a) Schüttelkolbenversuche

Die Kultivierung von *Bacillus megaterium* erfolgte im Nutrient-Medium bei 30°C, die rekombinanten *E. coli*-Stämme (DH5α, JM105, JM109, XL1-Blue, W3110) wurden in LB-Amp Medium bei 37°C kultiviert. Dazu wurde jeweils eine Kolonie mittels Impföse von einer Agarplatte in 5 ml LB-Amp überführt. Nach ca. 18 h Stunden Kultivierung bei 30°C (*Bacillus megaterium*) und 37°C (*E. coli*) bei einer Schüttelfrequenz von 220 Upm wurden 400 ml Medium in einem 2-l-Kolben mit 4 ml Kultur inokuliert. Die Induktion der P450-Expression in *E. coli* erfolgte nach Erreichen eines OD₅₇₈-Wertes zwischen 0,8 und 1,0 durch eine drei- bis vierstündige Hitzeschockinduktion bei 42°C im Falles der pT-Plasmid-Konstrukte oder durch Zugabe von Anhydrotetracyclin (Endkonzentration 0,4 mg/l) beim pA-USC4BM3-Plasmid und einer 14 stündigen Inkubation bei 30°C.

### b) 30-l-Batch-Fermentationen

Zur präparativen P450 BM-3 Produktion wurde die Expressionskassette pT-USC1BM3, der Wirt DH5α und ein Bioengineering Fermenter Typ LP351 bei einer Belüftung von 3,5 l/min, einer Rührgeschwindigkeit von 300 Upm bei 37°C in LB-Amp-Medium bei einem Start-pH-Wert von 7,5 eingesetzt. Um Schaumbildung zu vermeiden und Ausbeuten zu steigern wurden 2,0 ml steriles Contraspum 210 (ZSchimmer & Schwarz, Lahnstein, Deutschland) und 0,1 mg/l FeCl₃ vor Inokulation dem LB-Amp-Medium zugegeben (Nishihara et al. 1997). Inokuliert wurde durch Zugabe zweier 400 ml Schüttelkolben-Kulturen (OD₅₇₈= 0,8-1,0; Abschnitt a)). Nach Erreichen eines OD₅₇₈-Wertes von 1,0 im Fermenter erfolgte die P450-Produktion mittels Hitzeschockinduktion durch Erhöhen der Temperatur für drei bis fünf Stunden von 37°C auf 42°C. P450 BM-3 oder die Mutante F87A wurden in *E. coli* DH5α in Ausbeuten von 36 bis 48 mg/l Fermenterbrühe exprimiert. Die Aufkonzentrierung der *E*. *coli* Kultur von 30 Liter auf 2 Liter wurde durch Querstromfiltration mit einer Millipore Applikation (Eschborn, Deutschland) mit einer Filtron (Dreieich, Deutschland) Centrasette OMEGA (0.3 µm) Membran durchgeführt. Nach Zentrifugation bei 9200 g für 20 min wurden die Zellen in Kaliumphosphatpuffer (50 mM, pH 7,5) oder Tris/HCl-Puffer (50 mM, pH 7,5) resuspendiert, in zwölf 50 ml Falcon-Tubes (Greiner) aliquotiert und bis zur weiteren Verwendung bei -20°C eingefroren.

### Referenzbeispiel 2: Synthetische Oligonukleotide

Alle Primer wurden von ARK Scientific GmbH Biosystems (Düsseldorf, Deutschland) hergestellt. Nur die Primer für die Punktmutation und für die Isolierung des P450 BM-3 Gens aus der genomischen *Bacillus megaterium* DNA waren HPLC gereinigt.
a) Oligonukleotide für genomische Bacillus megaterium-DNA
B1) 5'-GTGAAAGAGGGATCCCATGACAATTAAAGAAATGCC-3' (SEQ ID NO: 1)
B2) 5'-GCCTCTTGGATCCTTACCCAGCCCACACGTCTTTTGCG-3' (SEQ ID NO: 2)

b) Primer für Sequenzierung

| | |
|---|---|
| R0_5 | 5'-GTACGTGATTTTGCAGGAG-3' (SEQ ID NO: 3) |
| R1 | 5'-GGCTATCATGCGATGATGGT-3' (SEQ ID NO: 4) |
| R1_5 | 5'-CCCAGCTTATGATGAAAAC-3' (SEQ ID NO: 5) |
| R2 | 5'-GGAAAAGATCCAGAAACGGG-3' (SEQ ID NO: 6) |
| R2_5 | 5'-GTCGGCATGGTCTTAAACG-3' (SEQ ID NO: 7) |
| R3 | 5'-ATTCCTCAGCTTCACCGTGA-3' (SEQ ID NO: 8) |
| R3_5 | 5'-CTTGGCGGTATTCCTTCAC-3' (SEQ ID NO: 9) |
| R4 | 5'-ATTTGCACCGCAGGTCGCAA-3' (SEQ ID NO: 10) |
| R4_5 | 5'-CTGGGCTACTACGTATC-3' (SEQ ID NO: 11) |
| R5 | 5'-TTCAATTTGTCGACAGCGCC-3' (SEQ ID NO: 12) |
| R5_5 | 5'-GAAGGAGATCATTTAGGTG-3' (SEQ ID NO: 13) |
| R6 | 5'-TCGCGCAATGGCTGCTAAAA-3' (SEQ ID NO: 14) |
| R6_5 | 5'-CGATTTCTTCATCACCTC-3' (SEQ ID NO: 15) |
| R7 | 5'-CTGCCAAAAGACCCTGAAAC-3' (SEQ ID NO: 16) |
| L1 | 5'-ACCATCATCGCATGATAGCC-3' (SEQ ID NO: 17) |
| L2 | 5'-CCCGTTTCTGGATCTTTTCC-3' (SEQ ID NO: 18) |
| L3 | 5'-TCACGGTGAAGCTGAGGAAT-3' (SEQ ID NO: 19) |
| L4 | 5'-TTGCGACCTGCGGTGCAAAT-3' (SEQ ID NO: 20) |
| L5 | 5'-GGCGCTGTCGACAAATTGAA-3' (SEQ ID NO: 21) |
| L6 | 5'-TTTTAGCAGCCATTGCGCGA-3' (SEQ ID NO: 22) |
| L7 | 5'-GTTTCAGGGTCTTTTGGCAG-3' (SEQ ID NO: 23) |

c) Oligonukleotide für Tags am C-Terminus

| | |
|---|---|
| A1) | 5'-GTGAAAGAGGGATCCCATGACAATTAAAGAAATGCC-3' |
| | (SEQ ID NO: 24) |
| A2) | 5'-CGGAATTCTTAACGACGACGACGACGACGCCCAGCCCACACG-3' |
| | (SEQ ID NO: 25) |
| G1) | 5'-GTGAAAGAGGGATCCCATGACAATTAAAGAAATGCC-3' |
| | (SEQ ID NO: 26) |
| G2) | 5'-CGGAATTCTTATTCTTCTTCTTCTTCTTCCCCAGCCCACACG-3' |
| | (SEQ ID NO: 27) |
| H1) | 5'-GTGAAAGAGGGATCCCATGACAATTAAAGAAATGCC-3' |
| | (SEQ ID NO: 28) |
| H2) | 5'-CGCAATTCTTAATGATGATGATGATGATGCCCAGCCCACACG-3' |
| | (SEQ ID NO: 29) |
| S1) | 5'-GTCTCAGCGTGAGACCCCCAGCCCACACGTCTTTTGCC-3' |
| | (SEQ ID NO: 30) |
| S2) | 5'-GTGAAAGAGGTCTCCAATGACAATTAAAGAAATGCC-3' |
| | (SEQ ID NO: 31) |

d) Primer für P450 BM-3 Punktmutante F87A

| | |
|---|---|
| F87A1 | 5'-GCAGGAGACGGGTTGGCCACAAGCTGGACGCATG-3' |
| | (SEQ ID NO: 32) |
| F87A2 | 5'-CATGCGTCCAGCTTGTGGCCAACCCGTCTCCTGC-3' |
| | (SEQ ID NO: 33) |

### Referenzbeispiel 3: Gentechnische Methoden

### 1. Isolierung und Präzipitation genomischer Bacillus-DNA und Plasmid-DNA aus E. coli

Zur Isolation der genomischen DNA aus *Bacillus megaterium* mittels Phenol-Chloroformextraktion wurde das Zellpellet aus 200 ml Kulturüberstand (OD₅₇₈= 1,2) in 20 ml Lysozymmix (18 mg Lysozym, 50 mM EDTA, 50 mM NaCl, 30 mM Tris/HCl, pH 8,0) resuspendiert und bei 37°C und 30 Minuten lang bei 220 UpM geschüttelt. Nach Zugabe von 2 ml SDS (10 % (m/v)) wurde weitere 60 Minuten bei 37°C und 220 UpM inkubiert. Zur Entfernung von Zelltrümmern wurde dreimal mit einer Mischung aus Chloroform/Isoamylalkohol (Roth, Karlsruhe, Deutschland) und einmal mit Phenol extrahiert. Die genomische DNA wird durch Zugabe von 10 % (v/v) 3 M NaAc, pH 4,8 und 60 % (v/v) Isopropanol gefällt, mittels Glasstab aufgerollt, in ein Corex-Röhrchen überführt und bei 32 570 g zentrifugiert. Nach dreimaligem Waschen mit 70 % Ethanol wird die DNA am "Hausvakuum" getrocknet und in 3 ml TE-Puffer (10 mM Tris/HCl, 1 mM EDTA, pH 7,5) gelöst. Die Reinheit der DNA wurde spektroskopisch durch Quotientenbildung von 260 nm zu 280 nm zu 2,2 bestimmt.

Die Isolation der Plasmid-DNA aus *E. coli* erfolgte mit dem auf alkalischer Lyse der Zellen basierenden QIAprep-Spin-Miniprep-Kit exakt nach den Angaben des Herstellers (Qiagen).

### 2. Polymerase-Ketten-Reaktion (PCR)

### a) PCR zur Isolierung des P450 BM-3 Gens aus genomischer Bacillus megaterium-DNA

**PCR-Standardprotokoll**

| Wiederholungen des Programmschritts | Denaturierung | DNA_Annealing | Elongation/DNA-Synthese |
|---|---|---|---|
| 1 Mal | 95°C, 7 min | | |
| 30 Mal | 95°C, 1,5 min | 50°C, 1,5 min | 72°C, 2 min |
| 1 Mal | | | 72°C, 7 min |

### Standard-Ansatz

8 µl dNTP-Mix (200µM), 10 µl Taq-Polymerase-Puffer (10 x) ohne MgCl₂, 8µl MgCl₂ (25mM), 1 µl Primer B1 (0,1 µM), 1 µl Primer B2 (0,1 µM), 1µl genomische *Bacillus megaterium-*DNA*,* 2,5 U Taq-Polymerase (MBI Fermentas, Vilnius, Litauen), ad 100 µl demineralisiertes Wasser.

### b) Einfügen von Tag-Sequenzen am C-Terminus

Um die Proteinreinigung zu vereinfachen und eine gerichtete Immobilisierung von P450 BM-3 zu ermöglichen wurde ein jeweils sechs Aminosäuren langer C-terminaler-Tag an das P450 BM-3 Gen angefügt. Als Templat fungierte genomische *Bacillus megaterium*-DNA. Als überhängende Restriktionsenden wurden für die His-, Arg- und Glu-Tags *Bam*HI und *Eco*RI gewählt, um anschließend die modifizierten P450-BM-3-Gene in das Plasmid pCYTEXP1 zu klonieren. Für den Strep-Tag wurden für das 5'- und 3'-Ende *Bsa*I gewählt; dies ermöglicht eine Klonierung in das Plasmid pASK-IBA1CA. *Bsa*I schneidet erst nach der Erkennungssequenz, wodurch die überhängenden Restriktionsenden eine unterschiedliche Sequenz aufweisen und somit eine gerichtete Ligation erlauben.

Für die PCR wurden das Standard-PCR Programm und der Standard-Ansatz verwendet. Im Unterschied zum Standard-Ansatz wurden für den His₆-Tag die Primer H1 und H2, für den Arg₆-Tag die Primer A1 und A2, für den Glu₆-Tag die Primer G1 und G2 sowie für den Strep-Tag die Primer S1 und S2 verwendet.

### 3. Restriktionsspaltung, Elektrophoretische Trennung und Reinigung von DNA-Fragmenten

Restriktionsendonukleasen schneiden DNA sequenzspezifisch. Die Restriktionsspaltung von DNA erfolgte in einem Reaktionsvolumen von 10 µl in Gegenwart von 2-3 µl DNA (Spin-Präp (Qiagen)), 1-2 U Restriktionsenzym, 1 µl Restriktionspuffer (10x) und ad 10 µl ddH₂O durch ein- bis zweistündige Inkubation der Reaktionslösung bei 37°C (außer *Bsa*I) entsprechend der Herstellervorschrift. Die Reinigung der DNA-Fragmente wurde mittels Agarose-Gelelektrophorese nach der Vorschrift von Sambrook et al. 1989 durchgeführt. Abhängig von der Größe der aufzutrennenden DNA-Fragmente wurden 0,8-2%ige Agarosegele verwendet. Als Elektrophorese-Puffer diente TAE-Puffer (40 mM Tris/HCl, 20 mM Essigsäure, 2 mM EDTA, pH 8,3). Angelegt wurde aufgrund der Wärmeentwicklung bei bis zu einprozentigen Agarose-Gelen eine Spannung von 120 V und bei bis zu zweiprozentigen Agarose-Gelen von 100 V. Interkalierendes Ethidiumbromid (Endkonzentration 0,25 µM) wurde zur Visualisierung der DNA im UV-Durchlicht eines Transilluminators (312 nm) verwendet.

Die Isolierung von DNA-Fragmenten aus Agarose-Gelen wurde unter Verwendung des QIAquick Gel Extraktion Kits (Qiagen) durchgeführt. Dabei wird die DNA durch Binden an eine Silika-Gel enthaltene Säule immobilisiert und mittels verschiedener Waschschritte von Verunreinigungen befreit. Durch anschließende Elution mit demineralisiertem Wasser können mit dieser Methode bis zu 8 µg DNA (100 bp-10 kb) erhalten werden.

### 4. Ligation und Transformation in E. coli

Zur Ligation von DNA wurde die T4-DNA-Ligase (New England Biolabs, Beverly, USA) verwendet, die die Bildung von Phosphodiesterbindungen zwischen 5'-Phosphat- und 3'-Hydroxy-Enden der DNA katalysiert und somit zwei lineare DNA-Moleküle verbindet oder die Zyklisierung eines linearen Moleküls ermöglicht.

Zur Ligation wurden Agarose-Gel gereinigte Vektor- und Insertfragmente (vergleiche Punkt 3.) im molaren Verhältnis 1 zu 3 bis 1 zu 5 zugunsten des Inserts in ein Eppendorf-Reaktionsgefäß gegeben. Vor Zugabe von 2 µl Ligase-Puffer (10x) und 2-10 U T4-DNA-Ligase wurde demineralisiertes Wasser hinzugefügt (Gesamtvolumen 20 µl). Die Reaktionsansätze wurden eine Stunde bei Raumtemperatur oder über Nacht bei 7°C inkubiert.

Zur Herstellung kompetenter Zellen für die Transformation wurden 500 µl einer *E*. *coli*-Übernachtkultur zum Animpfen eines 250 ml-Schüttelkolbens, der 50 ml LB-Medium enthielt, verwendet. Nach ca. dreistündiger Inkubation bei 37°C und 220 UpM, wird die Kultur nach Erreichen eines OD₆₀₀-Wertes bei 0,4-0,6 bei 5500 g 3 Minuten bei 4°C zentrifugiert. Das Zellpellet wird in 2 ml TSS-Puffer (10 g PEG6000, 5 ml DMSO, 0,6 g MgSO₄, ad 100 ml LB) resuspendiert, auf Eis inkubiert und in 200 µl Aliquots aufgeteilt und nach Schockgefrieren in flüssigem Stickstoff bei -80°C gelagert.

Zur Transformation werden zu 200 µl der kompetenten Zellen 5 bis 15 µl des Ligationsansatzes gegeben. Nach 30minütiger Inkubation auf Eis wird der Transformationsansatz 30 s bei 42°C im Thermomixer inkubiert und 800 µl auf 37°C erwärmtes LB-Medium zugegeben. Die Ansätze werden eine Stunde lang im Inkubator bei 37°C geschüttelt. Die Zellen wurden anschließend bei 5500 g zentrifugiert (3 Minuten), im rücklaufenden Tropfen LB-Medium aufgenommen und auf LB-Amp-Agarplatten ausplattiert. Über Nacht wurden die Agarplatten bei 37°C im Brutschrank inkubiert.

### 5. DNA-Sequenzierung

Die DNA-Sequenzierung wurde unter Verwendung des Applied Biosystems DNA Sequenzierautomats 373A und des Dye Terminator Cycle Sequencing-Kits mit AmpliTaq-DNA-Polymerase durchgeführt. Die Sequenzierung erfolgt nach der Didesoxy-Methode von Sanger, jedoch werden statt radioaktiv markierter dNTPs, dNTPs mit Fluoreszenzmarkern eingesetzt, die im Verlauf der Sequenzierreaktion in die synthetisierten DNA-Fragmente eingebaut werden.

Die Reaktionsprodukte wurden anschließend mit 100 prozentigem auf -20°C gekühltem Ethanol gefällt. Nach Trocknung der DNA und Aufnahme in 4 µl einer 5:1-Mischung aus Formamid und 25 mM EDTA, pH 8,0 erfolgte die Denaturierung eines jeden PCR-Ansatzes bei 95°C für 5 Minuten und sofortiger Überführung der Proben auf Eis.

Die mit Fluoreszenzfarbstoffen markierten DNA-Fragmente durchlaufen während der Gelelektrophorese einen Laserstrahl. Das senkrecht zu diesem Strahl emittierte Fluoreszenzlicht wird von Fotodioden hinter dem Gel gemessen und von der Software in ein Kurvendiagramm umgewandelt.

### Sequenzier-Ansatz

8 µl Terminator Ready Reaction Mix; 3,2 pmol Primer; 300-500 ng Template DNA;
H₂O ad 20 µl.

**PCR-Programm**

| Wiederholungen des Programmschritts | Denaturierung | DNA-Annealing | Elongation/DNA-Synthese |
|---|---|---|---|
| 1 Mal | 96°C, 6 min | | |
| 25 Mal | 96°C, 40 s | 50°C, 30 s | 60°C, 4 min |
| 1 Mal | | | 60°C, 4 min |

### Sequenzier-Gel-Zusammensetzung:

Harnstoff 30 g (Roth); Rotiphorese NF-10x-TBE-Puffer 6 ml (Roth); Acrylamid/Bis-Lösung 40 % (29:1) 9 ml (Roth); ddH₂O demineralisiert 23,5 ml.

Die fertige Lösung wird filtriert und entgast. Die Polymerisation wird durch Zugabe von 24 µl TEMED und 180 µl 10 % (m/v) APS gestartet. Für Einzelheiten dazu, zur Auswertung der Sequenzdaten und zu den dNTP-Markierungen, sei auf das Handbuch zum Sequenzierautomaten verwiesen.

### 6. Punktmutation F87A mittels OuikChange Kit (Stratagene)

Zur Erhöhung des Sensitivität des pNCA-Assaysystems, wurde an Position 87 die Aminosäure Phenylalanin durch ein Alanin ersetzt. Um die Punktmutante leichter identifizieren zu können, wurde, ohne weitere Änderung der Aminosäuresequenz, eine zusätzliche *Eae*I-Restriktionsschnittstelle unter Einbeziehung der Mutationsposition, eingefügt.

Im Gegensatz zum Standard-PCR-Programm erfolgte hierbei nur eine vierminütige DNA-Denaturierung und der Sequenzierreaktionsschritt wurde bei einer Annealing-Temperatur von 52,3°C, und 17-minütigem Elongationsschritt nur 16 Mal durchlaufen. Auf den dritten Programmschritt wurde verzichtet.

### Ansatz für PCR:

1,2 µl dNTP-Mix (200µM), 5 µl Pfu-Polymerase-Puffer (10x), 2,5 U Pfu-Polymerase, 2,5 µl Primer F87A1 (5nM), 2,5 µl Primer F87A2 (5nM), 1 µl pT-USC1BM3 (aus Mini-Präparation 1:20 mit demineralisiertem Wasser verdünnt), ad 50 µl demineralisiertes Wasser.

Die "Annealing"-Temperatur von 52,3°C zeigte nach obiger PCR auf einem einprozentigen Agarose-Gel eine scharfe Bande mit erwarteter Fragmentgröße.

Nach Abtrennen des Mineralöls vom Reaktionsgemisch wurde der PCR-Ansatz mit 1µl *Dpn*I (10 U/µl) versetzt und die Lösung vorsichtig durch Auf- und Abpipettieren gemischt. Es wurde kurz zentrifugiert und inkubiert bei 37°C für eine Stunde. Während des Verdaus wurden die ultrakompetenten *E. coli* XL1-Blue auf Eis aufgetaut und in 50 µl Aliquots auf 15 ml Falcon-Tubes verteilt. Nach Zugabe von 1 µl β-Mercaptoethanol, 4 µl des verdauten PCR-Reaktionsansatzes und 30minütiger Inkubation auf Eis folgte ein 30 s langer Hitzeschock bei 42°C und eine zweiminütige Abkühlung auf Eis. Zum Transformationsansatz wurden 450 µl erwärmtes (Ölbadtemperatur 42°C) NZY-Medium (12 g NZamine, 5 g Hefeextrakt, 5 g NaCl, pH 7,5) zugegeben. Nach einstündiger Inkubation bei 37°C und 220 UpM und Zentrifugation (5500 g, 3 min) wurde das Zellpellet in der Restflüssigkeit resuspendiert und auf LB-Amp-Platten ausgestrichen.

### Referenzbeispiel 4: Präparative Methoden

### 1. Zellaufschluß

Zellpellets mit einer Biofeuchtmasse von bis zu 15 g *E. coli* DH5α/pT-USC1BM-3 oder DH5α/pT-USC1BM-3F87A wurden auf Eis aufgetaut und in 25 ml Kaliumphosphat-Puffer (50 mM, pH 7,5, 1 mM EDTA) oder Tris/HCl Puffer (50 mM, pH 7,5, 1 mM EDTA) suspendiert. Mittels dreiminütiger Ultraschallbehandlung (Branson Sonifier W250, (Dietzenbach, Deutschland), Leistungsabgabe 80 W, Arbeitsintervall 20 %) wurde die auf Eis gekühlte *E. coli*-Zellsuspension aufgeschlossen. Vor der Proteinreinigung wurde die Zellsuspension für 20 min bei 32 500 g zentrifugiert und durch einen 0,22 µm Sterivex-GP-Filter (Millipore) filtriert. Diese klare Lösung wird im folgenden als Rohextrakt bezeichnet.

### 2. Chromatographische P450 BM-3-Reinigung mittels Anionenaustauschern

Zur Proteinreinigung wurde ein ÄKTAexplorer System (Amersham Pharmacia Biotech) mit einer auf Personal-Computern basierenden OS/2-UNICORN Steuersoftware v2.1 und einem Frac-900 Fraktionssammler verwendet, das die gleichzeitige Messung der Gesamtproteinelution bei 280 nm und P450 BM-3 Elution bei 417 nm erlaubt.

Alle analytischen P450 BM-3 Proteinreinigungen mittels Anionenaustauscherchromatographie wurden bei 200 cm/h, Raumtemperatur und einer Beladung von 6-10 mg P450 BM-3 pro 100 ml Matrix durchgeführt. Alle Chromatographiematerialien wurden von TosoHaas (Stuttgart, Deutschland) bezogen.

### a) Packung und Charakterisierung der Chromatographiesäulen

Zur Entwicklung eines P450 BM-3 Reinigungsprotokolls wurden vom Hersteller gepackte MPD-DEAE 650S (7,5 mm x 85 mm, Partikeldurchmesser 35 µm, Af=1,1, 4100 TP/m) und selbst gepackte XK16/20 (16 mm x 100 mm, APB) Chromatographiesäulen verwendet, die Toyopearl DEAE 650M (Af= 1,1, 1610 Tp/m), SuperQ 650M, (Af= 1,2, 1840 Tp/m) und QAE 550M (Af= 1,0, 1780 Tp/m) mit einem Partikeldurchmesser von 65 µm enthielten. Für die präparativen Chromatographiereinigungen wurde eine INDEX200 (APB) Glassäule (20 cm x 19 cm, Af= 1,4, 3800 Tp/m), sechs Liter Toyopearl DEAE 650M und eine Zahnradpumpe (ISMATEC, PB, USA) benutzt. Im Gegensatz zu den analytischen P450 BM-3 Chromatographiereinigungen wurde das ÄKTA*explorer* System über einen 1/20 aufgeteilten Fluß als Monitor verwendet, und die Puffer vor den präparativen Reinigungen manuell hergestellt. Die Puffer bestanden aus Tris/HCl (0,1 M, pH 7,8) mit 1 mM EDTA sowie 150 mM NaCl für die erste, 250 mM NaCl für die zweite und 1 M NaCl für die dritte Salzstufe.

### b) Wahl der pH-, Elutions- und Pufferbedingungen

Voruntersuchungen auf der Grundlage von Aktivitätsmessungen über NADPH-Verbrauch ergaben einen geeigneten pH-Bereich zwischen 6,8 und 8,0 zur P450 BM-3 Reinigung. Spätere Untersuchungen zur pH-Stabilität mit dem pNCA-Nachweissystem bestätigten diesen pH-Bereich.
Anionenaustauscherchromatographien mit linearen NaCl-Gradienten zeigten, daß ein Tris/HCl-Puffer (0,1 M, pH 7,8) mit 1 mM EDTA einem Phosphatpuffer (0,1 M, pH 7,0 bis pH 7,5) mit 1 mM EDTA in Bezug auf Bindungskapazität und Auflösungsvermögen für P450 BM-3 deutlich überlegen ist. Aus diesen Gründen wurde das Tris/HCl-Puffersystem für alle weiteren Reinigungsoptimierungen verwendet.

### 3. Immobilisierung der P450 BM-3 F87A Mutante

### a) Vorexperimente zur Auswahl der Trägermaterialien

Zur Immobilisierung von P450 BM-3 F87A wurden in Vorexperimenten verschiedene adsorptive und kovalente Methoden untersucht. Für alle Vorexperimente wurden 100 mg Trägermaterial (Ausnahme: EP-100, 50 mg) und 5 nmol P450 BM-3 F87A (aus Rohextrakt) verwendet.

Zur Auswahl eines geeigneten Trägermaterials wurde bei den adsorptiven Methoden 5 nmol P450 BM-3 F87A zur KₓPO₄-Puffer-(20 mM, pH 7,5, ad 1600 µl) Trägermaterial-Suspension in ein 2 ml Eppendorf-Reakionsgefäß gegeben. Die Reaktionsgefäße wurden nach Anbringen mittels Klebeband an die Außenseite eines 250 ml Rundkolbens eine Stunde am Rotationsverdampfer langsam zur Proteinimmobilisation über Kopf gedreht. Zur Bestimmung der Adsorptionskapazitäten wurden die Immobilisate eine Minute bei 9000 g zentrifugiert und 200 µl des Überstandes zur Aktivitätsmessung entnommen.

### b) Optimierte Immobilisierungsvorschrift für DEAE 650M und Super Q650M

Bis zu 81 nmol eines P450 BM-3 F87A-Rohextrakts oder Lyophilisats können pro ml sedimentiertem DEAE 650M Adsorptionsmaterial in 5 ml Tris/HCl-Puffer (pH 7,5) immobilisiert werden. Bei dem SuperQ 650M Adsorptionsmaterial sind es bis zu 102 nmol eines P450 BM-3 F87A-Rohextrakts. Zur Immobilisierung wurde das Lyophilisat in 4 ml dH₂O in einem 15 ml Falcon-Reaktionsgefäß aufgenommen und mit einer Endkonzentration von 0,1 mM 12-pNCA versetzt. Nach fünfminütiger Inkubation bei Raumtemperatur wurde 1 ml der mit 5 ml Wasser zweimal gewaschenen und zentrifugierten (9000 g, 1 min) Adsorptionsmatix zugegeben und eine halbe Stunde analog zu den im vorherigen Abschnitt beschriebenen Eppendorf-Reaktionsgefäßen über Kopf gedreht. Nach Zentrifugationen und Abnahme des Überstandes wurde das Immobilisat mit 5 ml Tris/HCl-Puffer (pH 7,5, 0,02 mM) versetzt, vorsichtig resuspendiert und erneut zentrifugiert (9000 g, 1min). Dieser Waschvorgang wurde zwei Mal wiederholt. Nach Zugabe von Tris/HCl-Puffer (0,1 M, pH 7,5) auf ein Endvolumen von 2,0 ml zum Immobilisat wurde dieses resuspendiert und in 200 µl Aliquots auf 1,5 ml Eppendorf-Reaktionsgefäße verteilt, die bereits 495 µl dieses Puffers enthielten. Nach Zugabe von 5 µl 12-pNCA (15 mM) und fünfminütiger Inkubationszeit wurde die Reaktion in einer Eppendorf-Schüttelapparatur (Ika-Vibrax, mit Janke und Kunkel Aufsatz VX2E) durch Zugabe von 100 µl NADPH (1 mM) gestartet und nach variierten Reaktionszeiten mit 100 µl 6 M KOH gestoppt. Nach Zentrifugation bei 9000 g (1 min) wurde der Überstand abgenommen und die Absorption bei 410 nm bestimmt.

Zur kovalenten Immobilisierung von P450 BM-3 F87A mittels Glutardialdehyd wurde P450 BM-3 F87A in einem 20 mM Kaliumphosphatpuffer (pH 7,5) aufgeschlossen. 5 g aminomodifizierter Trisopor-Gläser (Schueller, Durchmesser 500-800 µm) wurden bei Raumtemperatur zwischen drei und zwölf Stunden in einer geschlossenen Apparatur mit Glutardialdehyd bedampft. Die mit Glutardialdehyd aminomodifizierten Gläser zeigten eine rot-violette Farbe, die auch nach zweimaligem Waschen mit 100 ml Kaliumcarbonatlösung (50 mM, pH 7,8) über eine Fritte erhalten bleibt. 50 bis 150 mg dieser rot-violetten Gläser wurden mit 0,5-0,8 nmol P450 BM-3 F87A versetzt und bis zur Entfärbung der Lösung in 2 ml Eppendorf-Reaktionsgefäßen in der Retsch-Kugelmühle bei niedrigster Stufe und 6°C eine Stunde lang geschüttelt.

### 4. Enzym-Membranreaktor

zur Überprüfung des Enzym-Membran-Reaktorkonzepts wurden 25 nmol P450 BM-3 F87A auf 4 ml SuperQ 650M immobilisiert und in das Reaktorreaktionsgefäß zu 200 ml Tris/HCl Puffer (pH 7,8) gegeben. Nach Zugabe von 1,2 µmol 12-pNCA bei einer Flußgeschwindigkeit von 40 ml/min wurde die Lösung 20 Minuten lang ohne Gegendruck auf das Filtrationsmodul durchmischt. Bei einem Gegendruck von 2 psi wurde durch Zugabe von 2 ml wäßriger NADPH-Lösung (1 mM) die Reaktion gestartet und der Reaktionsablauf durch die Bestimmung der Wellenlängen bei 410 nm verfolgt. Während des Reaktionsablaufs wurde NADPH-Lösung in 1 ml Schritten bis zur vollständigen 12-pNCA-Umsetzung zugegeben.

### Referenzbeispiel 5: Chemische Synthesen

Alle ¹H-NMR-Spektren wurden mit einem 500 MHz-NMR-Gerät, alle ¹³C-NMR mit einem 125 MHz-NMR-Gerät und CDCl₃ als Lösungsmittel aufgenommen.

### 1. Herstellung von ω-Phenoxycarbonsäuren (PCA)

Reaktionsschema A zeigt zur direkten PCA-Synthese aus ω-Bromo-carbonsäuren.

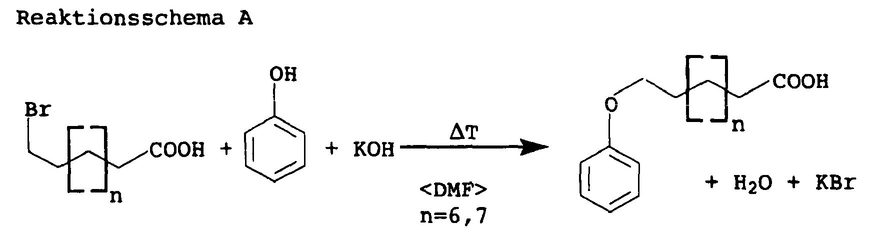

### Ansätze

| | | |
|---|---|---|
| 1,01 g | (3,77 mmol) | Bromundekansäure oder |
| 1,05 g | (3,77 mmol) | Bromdodekansäure |
| 355 mg | (3,77 mmol) | Phenol |
| 417 mg | (7,44 mmol) | Kaliumhydroxid |
| 50 ml | | DMF |

### Allgemeine Reaktionsvorschrift:

Zur in DMF gelösten Bromfettsäure gibt man Phenol und Kaliumhydroxid. Die Lösung wurde unter Rückfluß 6-7 Stunden lang auf 160°C erhitzt und die Reaktion mittels Dünnschichtchromatographie (DC, Laufmittel Petrolether : Diethylether 1:1) verfolgt. Nach Entfernen des Lösungsmittels wurde der braune Niederschlag in einem Wasser-Diethylether-Zweiphasensystem (1:1) gelöst und mit verdünnter Salzsäure wurde ein pH-Wert von 2 eingestellt. Nach Extraktion der wäßrigen Phase mit Diethylether wurden am Rotationsverdampfer die vereinigten Diethyletherfraktionen vom Lösungsmittel befreit, in Petrolether:Essig-säureethylester 1:1 aufgenommen und mittels einer DC60-Kieselgelchromatographiesäule (Merck) und dem gleichen Laufmittelgemisch gereinigt.

### Charakterisierung:

ω-Phenoxydodekansäure (12-PCA): Ausbeute: 76 %; mₚ:= 98-99°C berechnet: C 73,93% H 9,65% O 16,41%, gefunden: C 73,92% H 9,67%
¹H-NMR: δ:= 7,26-7,29 (m, 2H, Phenyl-), 6,88-6,94 (m, 3H, Phenyl), 3,94 (t, 2H, Phenyl-O-CH₂-, J= 6,5 Hz), 2,35 (t, 2H, -CH₂-COO, J= 7,5 Hz), 1,74-1,80 (m, 2H, -O-CH₂-CH₂-), 1,59-1,66 (m, 2H, -CH₂-CH₂-COO), 1,42-1,48 (m, 2H, -O-CH₂-CH₂-CH₂-), 1,29-1,35 (m, 12 H, -O-CH₂-CH₂-CH₂-(CH₂)₆-CH₂-).
¹³C-NMR: δ:= 180,4 (-COO), 159,3 (C 1'), 129,8 (C 3'), 120,8 (C 4'), 114,7 (C2') 68,3 (-O-CH₂), 34,5 (-CH₂-COO-), 29,9, 29,8, 29,7, 29,7, 29,5, 29,4, 29,1, 26,5, 25,1 (C-3-C-11).
ω-Phenoxyundekansäure (11-PCA): Ausbeute: 78 %; mₚ:= 95°C berechnet: C 73,35% H 9,41% O 17,24%, gefunden: C 73,32% H 9,44%
¹H-NMR: δ:= 7,18-7,21 (m, 2H, Phenyl-), 6,81-6,86 (m, 3H, Phenyl), 3,87 (t, 2H, Phenyl-O-CH₂-, J= 6,5 Hz), 2,27 (t, 2H, -CH₂-COO, J= 7,5 Hz), 1,67-1,73 (m, 2H, -O-CH₂-CH₂-), 1,53-1,57 (m, 2H, -CH₂-CH₂-COO), 1,35-1,39 (m, 2H, -O-CH₂-CH₂-CH₂-), 1,18-1,30 (m, 10H, -O-CH₂-CH₂-CH₂-(CH₂)₅-CH₂-).
13C-NMR: δ:= 180,5 (-COO), 159,5 (C 1'), 129,8 (C 3'), 120,7 (C 4'), 114,7 (C 2'), 68,2 (-O-CH₂-), 34,5 (-CH₂-COO-), 29,9, 29,7, 29,7, 29,7, 29,6, 29,4, 26,4, 25,0 (C-3-C-10).

### 2. Herstellung von p-Nitrophenoxycarbonsäuren (pNCA)

Im Gegensatz zu den PCA-Synthesen war eine entsprechende Synthese der pNCA-Verbindungen nicht möglich. Die pNCA-Synthese gelang erst, nachdem die ω-Bromcarbonsäuren verestert waren und wasserfrei gearbeitet wurde.

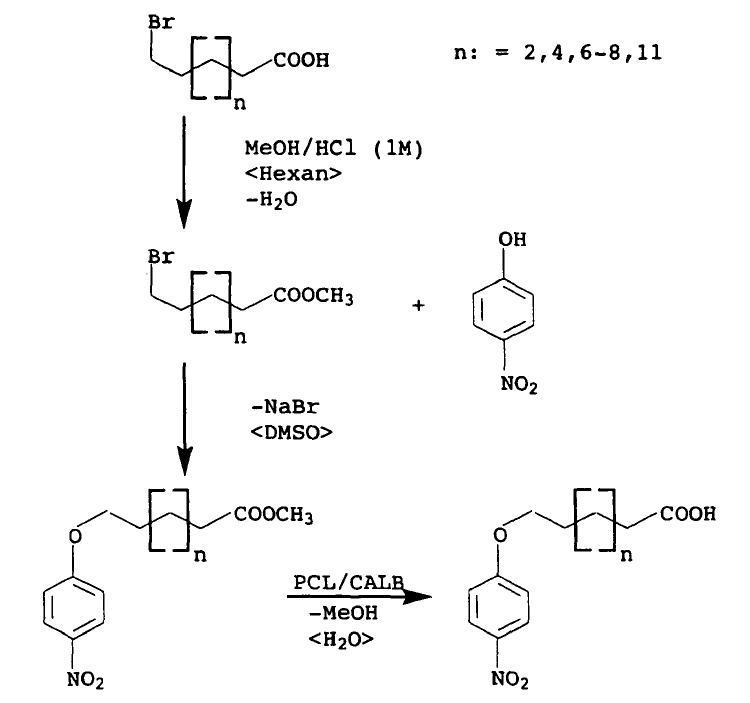

### Allgemeine Vorschrift zur Synthese:

Ausgehend von ω-Bromcarbonsäuren, wurden ω-Bromcarbonsäuremethylester und ω-Brom-carbonsäureethylester in Hexan entsprechend einer Standardmethode (Becker et al. 1993) mit wasserfreier Methanol- oder Ethanollösung, die 1 M HCl enthält, verestert. Der Reaktions-verlauf wurde mittels DC (Laufmittel Hexan : Diethylether : Essigsäure 70:30:1) verfolgt. Die wäßrige Phase wird zwei Mal mit Diethylether extrahiert, zwei Mal mit gesättigter Natriumhydrogencarbonatlösung und einmal mit demineralisiertem Wasser gewaschen, über Nacht mit wasserfreiem Natriumsulfat getrocknet, filtriert und chromatographisch aufgereinigt, wobei als Laufmittel Petrolether : Diethylether im Verhältnis 70:30 verwendet wird. Nachdem das Lösungsmittel am Rotationsverdampfer evaporiert war, wurden die Ester eine Stunde lang ans Hochvakuum angeschlossen. Um die pNCA-Ester darzustellen wurde zu 17 mmol ω-Brom-carbonsäureester, die in 100 ml DMSO gelöst wurden, ein geringer Überschuß von 18,5 nmol p-Nitrophenol gegeben. Die Lösung wurde unter Rückflußkühlung für zwei bis drei Stunden auf 120°C erhitzt. Nach Abkühlen der leicht bräunlichen Lösung auf Raumtemperatur wurde tropfenweise demineralisiertes Wasser zugegeben, um die pNCAester auszufällen. Das Präzipitat wurde filtriert, mit 1,5 Liter eiskaltem Wasser zur Entfernung von überschüssigem p-Nitrophenol gewaschen und in DMSO zu einem weißen Pulver umkristallisiert. Zur Hydrolyse der pNCAester wurde eine Lipasemischung, bestehend aus 100 mg *Pseudomonas cepacia* (PCL) und 100 mg *Candida antarctica* Lipase B von Amano (Nagoya, Japan), zu einer 100 ml Aceton/Wasser Mischung (pH 7,5, 0,2 M Kaliumphosphat, bis zu 50 % Aceton) zugegeben. Nach Zugabe der pNCAester (5 mmol) wurde die Suspension bei Raumtemperatur bis zu 16 h gerührt. Zur Entfernung des Lipaseimmobilisats wurde die klare Suspension filtriert und die pNCAs durch Evaporierung von Aceton kristallisiert. Die pNCA-Verbindungen wurden mit kaltem Wasser gewaschen, in DMSO umkristallisiert und chromatographisch über Silicagel DC60 (Merck, Darmstadt) (Petrolether : Essigsäureethylester 1:1 bis 1:3) gereinigt.

### Charakterisierung der pNCAs:

Die angegebenen Ausbeuten der pNCA beziehen sich immer auf die entsprechenden pNCAester als Edukte, und die Ausbeuten für die pNCAester beziehen sich immer auf die zugehörigen ω-Bromcarbonsäuren.
p-Nitrophenoxyhexansäure (6-pNCA): Ausbeute: 81 %; mₚ:= 94°C
¹H-NMR: δ:= 8,18 (d, 2H, NO₂-Phenyl-, J= 9,3Hz), 6,94 (d, 2H, Phenyl-O-, J= 9,3 Hz), 4,06 (t, 2H, Phenyl-O-CH₂-, J= 6,3 Hz), 2,42 (t, 2H, -CH₂-COO, J= 7,1 Hz), 1,80-1,92 (m, 2H, -O-CH₂-CH₂-), 1,68-1,77 (m, 2H, -CH₂-CH₂-COO), 1,49-1,61 (m, 2H, -O-CH₂-CH₂-).
¹³C-NMR: δ:= 180,0 (-COOH), 164,1 (C-4'), 141,4 (C-1'), 125,9 (C-2'), 114,4 (C-3'), 68,5 (-O-CH₂-), 33,9 (-CH₂-COOH), 28,6, 25,5, 24,3 (C-3, C-5, C-4).
p-Nitrophenoxyhexansäureethylester (6-pNCAEt): Ausbeute: 81 %
¹H-NMR: δ:= 8,09 (d, 2H, NO₂-Phenyl-, J= 9,3Hz), 6,85 (d, 2H, Phenyl-O-, J= 9,3 Hz), 4,05 (q, 2H, -COO-CH₂-), 3,97 (t, 2H, Phenyl-O-CH₂-, J= 6,4 Hz), 2,26 (t, 2H, -CH₂-COO, J= 7,3 Hz), 1,69-1,82 (m, 2H, -O-CH₂-CH₂-), 1,57-1,67 (m, 2H,-CH₂-CH₂-COO), 1,40-1,50 (m, 2H, -O-CH₂-CH₂-), 1,17 (t, 3H, -COOCH₂-CH₃).
¹³C-NMR: δ:= 173,7 (-COO), 164,3 (C 4'), 141,5 (C 1'), 126,0 (C 2'), 114,6 (C 3'), 68,7 (-O-CH₂-), 60,4 (-COO-CH₂-), 34,3 (-CH₂-COO-), 28,8, 25,7, 24,8 (C-3, C-5, C-4), 14,4 (-O-CH₂-CH₃).
p-Nitrophenoxyoktansäure (8-pNCA): Ausbeute: 92 %; mₚ:= 98°C
¹H-NMR: δ:= 8,11 (d, 2H, NO₂-Phenyl-, J= 9,2 Hz), 6,86 (d, 2H, Phenyl-O-, J= 9,3 Hz), 3,97 (t, 2H, Phenyl-O-CH₂-, J= 6,4 Hz), 2,29 (t, 2H, -CH₂-COO, J= 7,4 Hz), 1,69-1,80 (m, 2H, -O-CH₂-CH₂), 1,56-1,61 (m, 2H, -CH₂-CH₂-COO), 1,32-1,43 (m, 6H, -O-CH₂-CH₂-(CH₂)₃-CH₂-).
¹³C-NMR: δ:= 180,2 (-COOH), 164,3 (C 4'), 141,4 (C 1'), 125,9 (C 2'), 114,4 (C 3'), 68,8 (-O-CH₂-), 34,0 (-CH₂-COOH), 28,9, 28,9, 28,9, 25,8, 24,6 (C-3-C-7).
p-Nitrophenoxyoktansäuremethylester (8-pNCAMe): Ausbeute: 79 %
¹H-NMR: δ:= 8,10 (d, 2H, NO₂-Phenyl-, J= 9,2Hz), 6,86 (d, 2H, Phenyl-O-, J= 9,3 Hz), 3,97 (t, 2H, Phenyl-O-CH₂-, J= 6,5 Hz), 3,59 (s, 3H, -COOCH₃), 2,24 (t, 2H, -CH₂-COO, J= 7,4 Hz), 1,69-1,77(m, 2H, -O-CH₂-CH₂-), 1,51-1,59 (m, 2H, -CH₂-CH₂-COO), 1,27-1,43 (m, 6H, -O-(CH₂)₂-(CH₂)₃-).
¹³C-NMR: δ:= 174,2 (-COO), 164,3 (C 4'), 141,4 (C 1'), 125,9 (C 2'), 114,4 (C 3'), 68,8 (-O-CH₂-), 51,5 (-COO-CH₃), 34,0 (-CH₂-COO-), 29,0, 28,9, 28,9, 25,8, 24,9 (C-3-C-7).
p-Nitrophenoxydekansäure (10-pNCA): Ausbeute: 84 %; mₚ:= 101°C
¹H-NMR: δ:= 8,18 (d, 2H, NO₂-Phenyl-, J= 9,2 Hz), 6,93 (d, 2H, Phenyl-O-, J= 9,3 Hz), 4,03 (t, 2H, Phenyl-O-CH₂-, J= 6,5 Hz), 2,35 (t, 2H, -CH₂-COO, J= 7,4 Hz), 1,75-1,86 (m, 2H, -O-CH₂-CH₂-), 1,60-1,63 (m, 2H, -CH₂-CH₂-COO), 1,32-1,45 (m, 10H, -O-CH₂-CH₂-(CH₂)₅-CH₂-).
¹³C-NMR: δ:= 180,4 (-COO), 164,4 (C 4'), 141,5 (C 1'), 126,1 (C 2'), 114,6 (C 3'), 69,1 (-O-CH₂-), 34,2 (-CH₂-COOH), 29,9, 29,4, 29,4, 29,3, 29,2, 26,3, 26,1, 24,8 (C-3-C-9).
p-Nitrophenoxydekansäuremethylester (10-pNCAMe): Ausbeute: 73 %
¹H-NMR: δ:= 8,11 (d, 2H, NO₂-Phenyl-, J= 9,2 Hz), 6,86 (d, 2H, Phenyl-O-, J= 9,3 Hz), 3,97 (t, 2H, Phenyl-O-CH₂-, J= 6,5 Hz), 3,59 (s, 3H, -COOCH₃), 2,23 (t, 2H, -CH₂-COO, J= 7,5 Hz), 1,68-1,77 (m, 2H, -O-CH₂-CH₂-), 1,52-1,57 (m, 2H, -CH₂-CH₂-COO), 1,22-1,45 (m, 10H, -O-CH₂-CH₂-(CH₂)₅-CH₂-).
¹³C-NMR: δ:= 174,2 (-COO), 164,2 (C 4'), 141,2 (C 1'), 125,8 (C 2'), 114,3 (C 3'), 68,8 (-O-CH₂-), 51,3 (-COOCH₃), 34,3 (-CH₂-COO-), 29,2, 29,1, 29,1, 29,0, 28,9, 25,8, 24,8 (C-3-C-9).
p-Nitrophenoxyundekansäure (11-pNCA): Ausbeute: 89 %; mₚ:= 102°C
¹H-NMR: δ:= 8,19 (d, 2H, NO₂-Phenyl-, J=9,2 Hz), 6,94 (d, 2H, Phenyl-O-, J= 9,1 Hz), 4,05 (t, 2H, Phenyl-O-CH₂-, J= 6,5 Hz), 2,35 (t, 2H, -CH₂-COO, J= 7,6 Hz), 1,79-1,84 (m, 2H, -O-CH₂-CH₂-), 1,61-1,66 (m, 2H, -CH₂-CH₂-COO), 1,43-1,49 (m, 2H, -O-CH₂-CH₂-CH₂-), 1,32-1,45 (m, 10H, -O-CH₂-CH₂-CH₂-(CH₂)₅-CH₂-).
¹³C-NMR: δ:= 180,12 (-COO), 164,3 (C 4'), 141,3 (C 1'), 125,9 (C 2'), 114,4 (C 3'), 68,9 (-O-CH₂-), 34,0 (-CH₂-COO-), 29,4, 29,3, 29,3, 29,2, 29,0, 29,0, 25,9, 24,6 (C-3-C-10).
p-Nitrophenoxyundekansäuremethylester (11-pNCAMe): Ausbeute: 80 %
¹H-NMR: δ:= 8,18 (d, 2H, NO₂-Phenyl-, J=9,3 Hz), 6,93 (d, 2H, Phenyl-O-, J= 9,2 Hz), 4,03 (t, 2H, Phenyl-O-CH₂-, J= 6,5 Hz), 3,66 (s, 3H, -COOCH₃), 2,29 (t, 2H, -CH₂-COO, J= 7,5 Hz), 1,75-1,86 (m, 2H, -O-CH₂-CH₂-), 1,58-1,64 (m, 2H, -CH₂-CH₂-COO), 1,29-1,45 (m, 12H, -O-CH₂-CH₂-(CH₂)₆-CH₂-).
¹³C-NMR: δ:= 174,5 (-COO), 164,4 (C 4'), 141,5 (C 1'), 126,1 (C 2'), 114,6 (C 3'), 69,0 (-O-CH₂-), 51,6 (-COOCH₃), 34,2 (-CH₂-COO-), 29,6, 29,5, 29,4, 29,4, 29,3, 29,1, 26,1, 25,1 (C-3-C-10).
p-Nitrophenoxydodekansäure (12-pNCA): Ausbeute: 89 %; mₚ:= 106°C
berechnet: C 64,07% H 8,07% N 4,06%, O 23,71%, gefunden: C 63,99 % H 8,18% N 4,15%
¹H-NMR: δ:= 8,19 (d, 2H, NO₂-Phenyl-, J=9,3 Hz), 6,94 (d, 2H, Phenyl-O-, J= 9,4 Hz), 4,04 (t, 2H, Phenyl-O-CH₂-, J= 6,6 Hz), 2,35 (t, 2H, -CH₂-COO, J= 7,5 Hz), 1,79-1,84 (m, 2H, -O-CH₂-CH₂-), 1,60-1,66 (m, 2H, -CH₂-CH₂-COO), 1,43-1,49 (m, 2H, -O-CH₂-CH₂-CH₂-), 1,29-1,35 (m, 12H, -O-CH₂-CH₂-CH₂-(CH₂)₆-CH₂-).
¹³C-NMR: δ:= 180,4 (-COO), 164,3 (C 4'), 141,3 (C 1'), 125,9 (C 2'), 114,4 (C 3'), 68,9 (-O-CH₂-), 34,1 (-CH₂-COO-), 29,5, 29,4, 29,3, 29,2, 29,2, 29,0, 29,0, 25,9, 24,7 (C-3-C-11).
p-Nitrophenoxydodekansäureethylester (12-pNCAEt): Ausbeute: 73 %
¹H-NMR: δ:= 8,18 (d, 2H, NO₂-Phenyl-, J=9,2 Hz), 6,94 (d, 2H, Phenyl-O-, J= 9,3 Hz), 4,12 (q, 2H, -COO-CH₂-, J= 7,1 Hz) 4,05 (t, 2H, Phenyl-O-CH₂-, J= 6,5 Hz), 2,29 (t, 2H, -CH₂-COO, J= 7,5 Hz), 1,70-1,87 (m, 2H, -O-CH₂-CH₂-), 1,59-1,69 (m, 2H, -CH₂-CH₂-COO), 1,32-1,45 (m, 14H, -O-CH₂-CH₂-(CH₂)₇-CH₂-), 1,26 (t, 3H, -COOCH₂-CH₃, J= 7,2 Hz).
¹³C-NMR: δ:= 174,8 (-COO), 164,4 (C 4'), 141,5 (C 1'), 126,1 (C 2'), 114,6 (C 3'), 69,0 (-O-CH₂-), 60,5 (-COO-CH₂-), 34,2 (-CH₂-COO-), 29,6, 29,5, 29,4, 29,4, 29,3, 29,2, 29,1, 26,1, 25,1 (C-3-C-11), 14,4 (-COO-CH₂-CH₃).
p-Nitrophenoxypentadekansäure (15-pNCA): Ausbeute: 87 %; mₚ:= 115°C
¹H-NMR: δ:= 8,19 (d, 2H, NO₂-Phenyl-, J=9,2 Hz), 6,94 (d, 2H, Phenyl-O-, J= 9,3 Hz), 4,05 (t, 2H, Phenyl-O-CH₂-, J= 6,5 Hz), 2,35 (t, 2H, -CH₂-COO, J= 7,5 Hz), 1,79-1,85 (m, 2H, -O-CH₂-CH₂-), 1,59-1,66 (m, 2H, -CH₂-CH₂-COO), 1,43-1,49 (m, 2H, -O-CH₂-CH₂-CH₂-), 1,26-1,35 (m, 18H, -O-CH₂-CH₂-CH₂-(CH₂)₉-CH₂-).
¹³C-NMR: δ:=180,0 (-COO), 164,3 (C.4'), 141,3 (C 1'), 126,0 (C 2'), 114,4 (C 3'), 68,9 (-O-CH₂-), 34,0 (-CH₂-COO-), 29,6, 29,6, 29,5, 29,4, 29,4, 29,3, 29,3, 29,2, 29,1, 29,0, 25,9, 24,7 (C-3-C-14).
p-Nitrophenoxypentadekansäuremethylester (15-pNCAMe): Ausbeute: 75 %
¹H-NMR: δ:= 8,19 (d, 2H, NO₂-Phenyl-, J=9,2 Hz), 6,94 (d, 2H, Phenyl-O-, J= 9,3 Hz), 4,05 (t, 2H, Phenyl-O-CH₂-, J= 6,5 Hz), 3,66 (s, 3H, -COOCH₃), 2,26 (t, 2H, -CH₂-COO, J= 7,5 Hz), 1,79-1,85 (m, 2H, -O-CH₂-CH₂-), 1,59-1,66 (m, 2H, -CH₂-CH₂-COO), 1,43-1,49 (m, 2H, -O-CH₂-CH₂-CH₂-), 1,26-1,35 (m, 18H, -O-CH₂-CH₂-CH₂-(CH₂)₉-CH₂-).
¹³C-NMR: δ:= 174,6 (-COO), 164,3 (C 4'), 141,3 (C 1'), 126,0 (C 2'), 114,4 (C 3'), 68,9 (-O-CH₂-), 51,7 (-COOCH₃), 34,0 (-CH₂-COO-), 29,7, 29,6, 29,5, 29,5, 29,4, 29,3, 29,3, 29,2, 29,1, 29,1, 26,1, 25,0 (C-3-C-14).

### 3. Herstellung von Resorufinylcarbonsäuren (RCA)

### a) Darstellung von ω-Resorufinyldodekansäuremethylester und ω-Resorufinylundekansäure-methylester

Zur RCA-Synthese wurden die ω-Bromcarbonsäuren, entsprechend dem Vorgehen bei den pNCA-Verbindungen verestert, mit dem Natriumresorufinsalz in DMF umgesetzt und mittels einer PCL/CAL-B-Lipasemischung hydrolysiert.

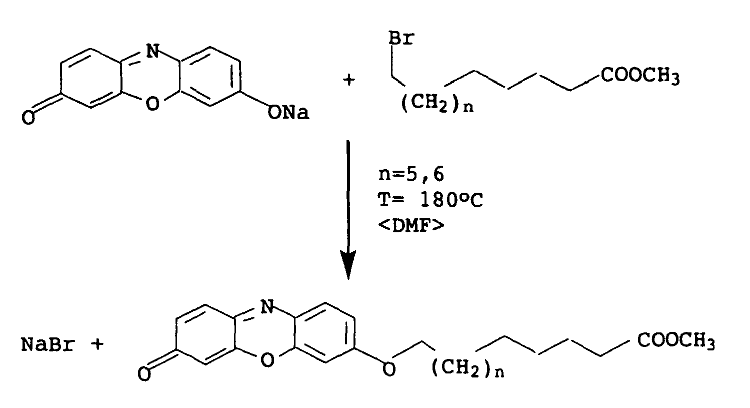

### Ansatz für ω-Resorufinyllaurinsäuremethylester:

| | | |
|---|---|---|
| 0,90 g | (3,1 mmol) | 12-Bromlaurinsäuremethylester |
| 0,72 g | (3,1 mmol) | Resorufin |
| 50 ml | | DMF |

### Ansatz für ω-Resorufinylundekansäuremethylester:

| | | |
|---|---|---|
| 0,75 g | (2,7 mmol) | 11-Bromundekansäuremethylester |
| 0,63 g | (2,7 mmol) | Resorufin |
| 50 ml | | DMF |

### Durchführung:

Zur Lösung des Bromfettsäureesters in DMF gibt man Resorufin, das sich nicht vollständig löst, zu. Die dunkelviolette Suspension wird unter Rückfluß vier Stunden lang erhitzt und die Reaktion mittels DC (Laufmittel Petrolether:Diethylether 1:1) verfolgt. Nach dem Entfernen des Lösungsmittels wird der braune Niederschlag in Petrolether:Essigsäureethylester 1:1 eine halbe Stunde lang gelöst und mittels einer DC60 (Merck)-Kieselgelchromatographiesäule in dem gleichen Laufmittelgemisch gereinigt. Die Säule sollte nicht zu groß gewählt werden, da die Verbindung in geringem Maße auf der Säule zerfällt.
ω-Resorufinyllaurinsäuremethylester: Ausbeute: 17 %
¹H-NMR: δ:= 7,62 (d, 1H, -H-9', J= 8,89 Hz), 7,35 (d, 1H, -H-1', J= 9,79 Hz), 6,85 (dd, 1H, -H-8', J_{8'}, _{9'}= 8,87 Hz, J_{8',6'}= 2,41 Hz), 6,76 (dd, 1H, -H-2', J_{2', 1'}= 9,74 Hz, J_{2',4'}= 1,74 Hz), 6,73 (d, 1H, -H-6', J= 2,40 Hz), 6,25 (d, 1H, -H-4', J= 1,74 Hz), 3,98 (t, 2H, Phenyl-O-CH₂-, J= 6,50 Hz), 3,59 (s, 3H, -COO-CH₃), 2,23 (t, 2H, -CH₂-COO, J= 7,50 Hz), 1,73-1,79 (m, 2H, -O-CH₂-CH₂-), 1,53-1,56 (m, 2H, -CH₂-CH₂-COO), 1,37-1,41 (m, 2H, -O-CH₂-CH₂-CH₂-), 1,22-1,29 (m, 12H, -O-CH₂-CH₂-CH₂-(CH₂)₆-CH₂-).
¹³C-NMR: δ:= 186,7 (-C 3'), 174,7 (-COO), 163,7, 150,3 146,1, 145,7 (C 4a', C-5a', C-9a', C-10a'), 135,1, 134,5, 131,9, 128,7, 114,5, 107,1, 100,8 (C 2', C 4', C 1', C 9', C 6', C 8', C 9'), 69,5 (-O-CH₂-), 51,9 (-COO-CH₃),34,5 (-CH₂-COO-), 30,1, 29,8, 29,7, 29,7, 29,6, 29,5, 29,3, 26,3, 25,3 (C-3-C-11).
ω-Resorufinylundekansäuremethylester: Ausbeute: 15 %;
¹H-NMR: δ:= 7,62 (d, 1H, -H-9', J= 8,91 Hz), 7,35 (d, 1H, -H-1', J= 9,70 Hz), 6,86 (dd, 1H, -H-8', J_{8'}, _{9'}= 8,90 Hz, J_{8',6'}= 2,53 Hz), 6,76 (dd, 1H, -H-2', J_{2'}, _{1'}= 9,72 Hz, J_{2'},_{4'}= 1,95 Hz), 6,73 (d, 1H, -H-6', J= 2,54 Hz), 6,25 (d, 1H, -H-4', J= 1,94 Hz), 3,98 (t, 2H, Phenyl-O-CH₂-, J= 6,49 Hz), 3,60 (s, 3H,-COO-CH₃), 2,23 (t, 2H, -CH₂-COO, J= 7,50 Hz), 1,74-1,79 (m, 2H, -O-CH₂-CH₂-), 1,54-1,57 (m, 2H, -CH₂-CH₂-COO), 1,35-1,42 (m, 2H, -O-CH₂-CH₂-CH₂-), 1,18-1,29 (m, 10H, -O-CH₂-CH₂-CH₂-(CH₂)₅-CH₂-).
¹³C-NMR: δ:= 186,7 (-C 3'), 174,7 (-COO), 163,7, 150,3 146,1, 145,7 (C 4a', C-5a', C-9a', C-10a'), 135,0, 135,0, 132,0, 128,6, 114,5, 107,0, 100,8 (C 2', C 4', C 1', C 9', C 6', C 8', C 9'), 69,5 (-O-CH₂-), 51,8 (-COO-CH₃), 34,5 (-CH₂-COO-), 29,9, 29,8, 29,7, 29,6, 29,5, 29,3, 26,3, 25,3 (C-3-C-10).

### b) Darstellung von ω-Resorufinyldodekansäure und ω-Resorufinylundekansäure

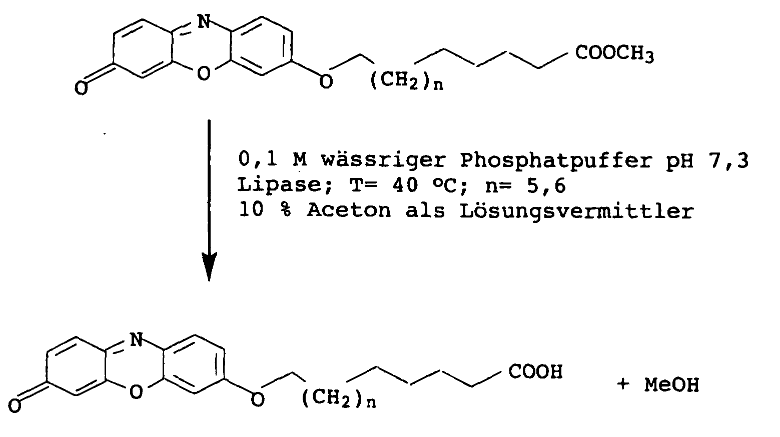

### Vorversuche:

Chemische Hydrolyse mittels NaOH führte zur Spaltung des Esters, jedoch konnte das gewünschte gelb-orange Produkt nur in Ausbeuten von 10 % isoliert werden; die Ursache liegt darin, daß der Resorufinyl-Aromat in stark alkalischen Lösungen (pH>12) unter Farbverlust zerfällt. Auf DC-Plättchen waren fünf Produkte erkennbar. Im alkalischen Medium zeigt der Resorufinylfettsäureester eine grünliche Färbung. Die Spaltung mittels Lipasen verlief nach Optimierung der Aufarbeitung wesentlich erfolgreicher. Zur Wahl einer geeigneten Lipase wurden folgende Lipasen im Thermomixer bei 40° C über Nacht in einem 0,1 M Natriumphosphatpuffer pH 7,5 mit 10 % Aceton als Lösungvermittler getestet: *Candida antarctica Lipase A* und *B*, *Aspergillus niger* Lipase und *Pseudomonas cepacia Lipase* (PCL).

Die *Candida antarctica Lipase B* und die *PCL* spalten die Resorufinylcarbonsäureester, wobei letztendlich die PCL-Lipase zur präparativen Esterspaltung verwendet wurde.

### Ansatz für ω-Resorufinyldodekansäure:

| | | |
|---|---|---|
| 40 mg | (94 µmol) | ω-Resorufinyldodekansäuremethylester |
| 2 ml | | Aceton |
| 18 ml | | Natriumphosphatpuffer, pH 7,3 |
| Spatelspitze | | PCL-Lipase |

### Ansatz für ω-Resorufinylundekansäure:

| | | |
|---|---|---|
| 36 mg | (30 µmol) | ω-Resorufinylundekansäuremethylester |
| 2 ml | | Aceton |
| 18 ml | | Natriumphosphatpuffer, pH 7,3 |
| Spatelspitze | | PCL-Lipase |

### Durchführung:

In einem 50 ml Rundkolben werden die Ester in Aceton gelöst, nach Zugabe von 9 ml Natriumphosphatpuffer, pH 7,3 wird eine Spatelspitze PCL zugegeben und bei 40°C über Nacht inkubiert. Der Reaktionsablauf wird mittels DC (Laufmittel Petrolether : Diethylether 1:1) verfolgt. Nach mehrfacher vorsichtiger Extraktion mittels Chloroform wird die Chloroformphase zwei Mal mit demineralisiertem Wasser gewaschen, über Natriumsulfat vier Stunden getrocknet, filtriert und am Rotationsverdampfer eingeengt.
ω-Resorufinyllaurinsäure: Ausbeute: 73 %
¹H-NMR: δ:= 7,62 (d, 1H, -H-9', J= 8,9 Hz), 7,35 (d, 1H, -H-1', J= 9,8 Hz), 6,85 (dd, 1H, -H-8', J_{8'} , _{9'}= 8,87 Hz, J_{8'} , _{6'}= 2,4 Hz), 6,76 (dd, 1H, -H-2', J_{2'} , _{1'}= 9,7 Hz, J_{2'} ,_{4'}= 1,7 Hz), 6,73 (d, 1H, -H-6', J= 2,4 Hz), 6,25 (d, 1H, -H-4', J= 1,7 Hz), 3,98 (t, 2H, Phenyl-O-CH₂-, J= 6,5 Hz), 2.34 (t, 2H, -CH₂-COO, J= 7,5 Hz), 1.73-1.79 (m, 2H, -O-CH₂-CH₂-), 1.55-1.59 (m, 2H, -CH₂-CH₂-COO), 1.37-1.41 (m, 2H, -O-CH₂-CH₂-CH₂-), 1.22-1.29 (m, 12H, -O-CH₂-CH₂-CH₂-(CH₂)₆-CH₂-).
¹³C-NMR: δ:= 186,6 (-C 3'), 180,4 (-COO), 163,7, 150,3 146,1, 145, 7 (C 4a', C-5a', C-9a', C-10a'), 135,1, 134,5, 131,9, 128,7, 114,5, 107,1, 100,8 (C 2', C 4', C 1', C 9', C 6', C 8', C 9'), 69,5 (-O-CH₂-), 34,2 (-CH₂-COO-), 30,1, 29,8, 29,7, 29,7, 29,6, 29,5, 29,3, 26,3, 25,3 (C-3-C-11).
ω-Resorufinylundekansäure: Ausbeute: 78 %
¹H-NMR: δ:= 7,63 (d, 1H, -H-9', J= 8,9 Hz), 7,35 (d, 1H, -H-1', J= 9,7 Hz), 6,85 (dd, 1H, -H-8', J_{8'}, _{9'}= 8,9 Hz, J_{8',6'}= 2,53 Hz), 6,76 (dd, 1H, -H-2', J_{2', 1'}= 9,7 Hz, J_{2',4'}= 1,9 Hz), 6,73 (d, 1H, -H-6', J= 2,5 Hz), 6,25 (d, 1H, -H-4', J= 1,9 Hz), 3,98 (t, 2H, Phenyl-O-CH₂-, J= 6,5 Hz), 2.35 (t, 2H, -CH₂-COO, J= 7,5 Hz), 1.74-1.79 (m, 2H, -O-CH₂-CH₂-), 1.54-1.59 (m, 2H, -CH₂-CH₂-COO), 1.35-1.42 (m, 2H, -O-CH₂-CH₂-CH₂-), 1.18-1.29 (m, 10H, -O-CH₂-CH₂-CH₂-(CH₂)₅-CH₂-).
¹³C-NMR: δ:= 186,7 (-C 3'), 180,7 (-COO), 163,7, 150,3 146,1, 145,7 (C 4a', C-5a', C-9a', C-10a'), 135,0, 135,0, 132,0, 128,6, 114,5, 107,0, 100,8 (C 2', C 4', C 1', C 9', C 6', C 8', C 9'), 69,5 (-O-CH₂-), 34,1 (-CH₂-COO-), 29.9, 29.8, 29.7, 29,6, 29,5, 29,3, 26,3, 25,3 (C-3-C-10).

### c) Aktivitätstest von P450 BM-3 mit ω-Resorufinyllaurinsäure und ω-Resorufinylundekansäure:

Eine Spatelspitze der 11- oder 12-RCA wurde in 0,5 ml Aceton, Ethanol, THF, Dioxan und 5,5 ml Kaliumphosphatpuffer (pH 7,5, 0,1 M) gelöst. Zu den als Lösungsvermittler fungierenden organischen Lösungsmitteln wurde 5 ml Kaliumphosphatpuffer (pH 7,5, 0,1 M) gegeben. Nach Zugabe von 5 nmol P450 wurde 10 Minuten bei Raumtemperatur inkubiert und das P450 BM-3 durch Zugabe von 200 µl NADPH (50 µM) reduziert. Nach einer Stunde wurden 200 µl der jeweiligen Reaktionsansätze in eine Mikrotiterplatte überführt.
In der zweiten Reihe sind zusätzlich entsprechende Blindproben ohne NADPH-Zugabe pipettiert.

### Referenzbeispiel 6: Analytische Methoden

### 1. Proteintrennung in der SDS-Polyacrylamid-Gelelektrophorese (SDS-PAGE)

Mittels SDS-PAGE können Proteine nach ihrer Größe aufgetrennt werden. Gereinigte Proteinproben wurden vor der Gelelektrophorese 1:1 mit SDS-Probenpuffer (Tris/HCl 500 mM, pH 6,8, Glycerin 10 % (v/v), SDS 20 % (w/v), Mercaptoethanol 2 % (w/v), Bromphenolblau 0,05 %) vermischt und 5 min zur Proteindenaturierung auf 95°C erhitzt. Zur Analyse der Proteinexpression in *E. coli* Zellen wurden nach fünfstündiger Induktionsphase (OD₅₇₈ = 1,5-2,0) 1 ml der Kultur abpipettiert und bei 12000 g zentrifugiert. Das Zellpellet wurde mit 100 µl des Probenpuffers aufgeschlossen und 1 min bei 12000 g zentrifugiert. Nach Abkühlen der Proteinproben auf Eis erfolgte die Proteinauftrennung im Elektrophoresepuffer (9,0 g Tris, 43,2 g Glycerin, 3,0 g SDS, ddH₂O ad 600 ml) bei 25 mA pro Gel.

Als Proteinstandards dienten ein Niedermolekulargewichts-Marker (Phosphorylase B 97,4 kDa, BSA 66,3 kDa, Ovalbumin 45,0 kDa, Carboanhydrase 31,0 kDa, Trypsininhibitor 21,5 kDa, Lysozym 14,4 kDa) und ein Hochmolekulargewichts-Kit (Myosin 200 kDa, β-Galaktosidase 116,25 kDa, Phosphorylase B 97,4 kDa, BSA 66,3 kDa, Ovalbumin 45 kDa) von Bio-Rad (Richmond, USA).

Zur Proteintrennung wurde ein 7,5 prozentiges Trenngel (2,5 ml Tris/HCl (1,5 M, pH 8,8), 100 µl SDS (10% m/v), 2,5 ml Acrylamid : N,N'-Methylenbisacrylamid 30 : 1 (Roth), 50 µl APS, 5 µl TEMED) und 4 prozentiges Sammelgel (1 ml Sammelgelpuffer (12,11 g Tris, 0,8 g SDS, ad H₂O 200 ml, pH 6,8 mit HCl eingestellt), 0,52 µl Acrylamid : N,N'-Methylen-bisacrylamid 30:1 (Roth), 40 µl APS 10 % (w/v), 4 µl TEMED verwendet. Im Anschluß an die Elektrophorese wurden die aufgetrennten Proteine drei Stunden lang unter langsamem Schütteln mit Färbelösung (0,1 % Coomassie Brilliant Blue R250, 10 % (v/v) Essigsäure, 30 % Methanol) blau gefärbt. Die Inkubation in Entfärbelösung (10 % (v/v) Essigsäure, 30 % Methanol) erfolgte bis deutliche Proteinbanden sichtbar wurden. Zur Dokumentation wurde das Gel luftblasenfrei zwischen ein Cellulose-Filterpapier und eine Kopierfolie gelegt und zwei Stunden im Geltrockner (Bio-Rad, Model 583) bei 80°C unter Hausvakuum getrocknet.

### 2. Bestimmung des Proteingehalts mittels BCA-Nachweis

Proteinkonzentrationen wurden mittels des Bicinchoninsäure-(BCA) Protein-Nachweissystems von Pierce (St. Augustin, Deutschland) spektrometrisch bei 562 nm nach Herstellerangaben entsprechend dem Standardprotokoll bestimmt. Kalibriergeraden wurden mit BSA-Verdünnungen aufgenommen.

### 3. Spektroskopische Nachweismethoden

Alle spektroskopischen UV-Vis Nachweise wurden unter aeroben Bedingungen in einem Pharmacia Spectrophotometer, Modell BioChrom4060 (APB, Uppsala, Schweden), mit BioChrom4060 Windows 3.11 Software v2.0 durchgeführt.

### a) Bestimmung der P450 Konzentration

### P450 BM-3 Konzentrationen wurden mittels der

CO-Differenzspektroskopiemethode (Omura et al. 1964) und einem Extinktionskoeffizienten von ε= 91 mM⁻¹ * cm⁻¹ bei einer Vorschubgeschwindigkeit von 125 nm/min in 0,1 nm Intervallen bestimmt. Dazu wurde zu 5 ml phosphatgepuffertem (20 mM, pH 7,3 bis 7,5) P450 BM-3 Rohextrakt eine Spatelspitze Natriumdithionit und 10 µl einer 0,1 prozentigen (w/v) wäßrigen Methylviologen-Lösung gegeben. Die abhängig von der P450 BM-3 Konzentration tiefblaue bis blaugrüne Probe wurde in zwei Fraktionen aufgeteilt. Eine der beiden Fraktionen wurde eine Minute lang mit CO begast, wobei Schaumbildung zu vermeiden ist, die andere dient als Referenz für die differenzspektroskopische Messung.

### b) Messung der Reduktase-Aktivität

Die Messungen wurden bei Raumtemperatur in 0,1 M Kaliumphosphatpuffer pH 7,4 durchgeführt, wobei die Absorptionszunahme bei 550 nm mit ε= 8,9 mM⁻¹ (Fulco et al. 1992) gemessen wurde. 1 ml Probe enthielten 50 nmol Cytochrom c (aus Pferdeherz), 100 nmol NADPH und eine aus Tabelle 6 ersichtlichen Menge P450 BM-3. Als Referenz diente dieselbe Probe vor NADPH-Zugabe. Die Lösungen wurden frisch bereitet und bis zur Benutzung am selben Tag auf Eis gelagert. Zur Messung der Reduktaseaktivität von P450 BM-3His₆ wurde zur Cytochrome-c-Lösung nach fünfminütiger Inkubation die NADPH-Lösung gegeben.

### 4. Protokolle zur Charakterisierung von P450 BM-3 und/oder P450 BM-3 F87A

Zur Messung der kinetischen Konstanten sowie der pH- und Temperaturstabilität wurde gereinigtes P450 BM-3 F87A verwendet. Für alle anderen Bestimmungen wurde zentrifugierter und filtrierter klarer Rohextrakt verwendet. Für alle Experimente wurden pro Ansatz 0,05-0,2 nmol P450 BM-3 oder P450 BM-3 F87A in einem Gesamtvolumen von 1 ml und 8 µl 5-15 mM pNCA-Lösung verwendet. Mit Ausnahme der Lösungsmittelversuche wurden Charakterisierungsexperimente in 1 ml Einweg-Plastikküvetten durchgeführt. Sofern nicht anders beschrieben, wurden die Versuche bei Raumtemperatur durchgeführt.

### a) Bestimmung der kinetischen Konstanten

Die kinetischen Konstanten wurden nach Zugabe von 792 µl Tris/HCl (pH 8,2, 0,2 M) und 100 µl P450 BM-3 zu 8 µl DMSO-Substratlösung unterschiedlicher Substratkonzentration ermittelt. Nach fünfminütiger Inkubation bei Raumtemperatur wurde die Reaktion durch Zugabe von 100 µl 1 mM NADPH-Lösung gestartet.

### b) Lösungsmittelstabilität von P450 BM-3 F87A

Die Messung der Lösungsmittelstabilitäten erfolgte in Glasküvetten (Hellma, Modell 6040), wobei Substrat statt in DMSO in Aceton, Dioxan, THF und Ethanol gelöst wurde. Die Bestimmungen wurden entsprechend der bei den kinetischen Messungen beschriebenen Vorschrift durchgeführt; jedoch wurde in Unterschied dazu der Anteil des Puffers entsprechend der verwendeten Lösungsmittelmenge variiert.

### c) pH-Stabilität und Temperaturstabilität von P450 BM-3 F87A

Die Messung der pH-Stabilität wurden bei 30°C im Stratagene Robocycler (Modell gradient40) in dünnwandigen 0,5 ml PCR-Reaktionsgefäßen (Stratagene) durchgeführt. Zu 0,1-0,2 nmol P450 BM-3 wurden 392 µl phosphatgepufferter Lösung (50 mM, pH 4-10) gegeben. Nach dem Inkubationszeitraum wurde die P450 BM-3 Lösung in eine Küvette überführt und 500 µl Tris/HCl (0,3 M, pH 8,2) und 8 µl 12-pNCA (6 mM) hinzugegeben. Nach fünfminütiger Inkubation wurde die Reaktion mittels 100 µl NADPH-Lösung (1 mM) gestartet. Die Temperaturstabilität wurde bei einem pH-Wert von 7,5 unter den gleichen Bedingungen wie die pH-Stabilität untersucht.

### d) Einfluß von Puffersalzkonzentrationen, Detergenzien, Proteaseinhibitoren, Thio-Verbindungen, Co(III)sepulchrat auf die P450 BM-3 F87A Aktivität

Die Auswirkungen dieser Verbindungen auf die P450 BM-3 F87A Aktivität sind in Analogie zu den kinetischen Untersuchungen durchgeführt worden, wobei ein variierter Teil des Puffers durch die im selben Puffersystem gelöste Untersuchungssubstanz ersetzt wurde.

### e) Automatisierter Aktivitätsnachweis

Für den automatisierten Aktivitätsnachweis von P450 BM-3 F87A wurden Mikrotiterplatten mit 96 Reaktionskammern (Greiner Frickenhausen, Deutschland) gewählt. Das Gesamtreaktionsvolumen betrug 250 µl in einer Tris/HCl-Puffer (0,1 M, pH 8,2) mit 18 nmol 10- und 11-pNCA, 12 nmol 12-pNCA oder 10 nmol 15-pNCA, die in 2,2 µl DMSO gelöst waren. Für die Pipettierarbeiten wurde eine Biomek2000 Pipettierroboter (Beckman Instruments, Fullerton, USA) verwendet. Nach fünfminütiger Inkubationszeit mit 0,02 nmol P450 BM-3 F87A wurde die Reaktion gestartet durch Einspritzen von 25 µl einer wäßrigen NADPH Lösung (1 mM) in jede Reaktionskammer. In einem FluoStar-Mikrotiterplattenleser (BMG LabTechnology, Offenburg, Deutschland) wurde der Reaktionsablauf bei 405 nm absorptiv verfolgt.

### f) Wasserstoffperoxid-Untersuchungen

Für Aktivitätsuntersuchungen wurde verfahren wie für die kinetischen Messungen beschrieben, nur daß statt NADPH variierte wäßrige Wasserstoffperoxidmengen zugegeben wurden.

Bei den Stabilitätsuntersuchungen wurden 0,05-0,1 nmol P450 BM-3 F87A in Abwesenheit von pNCA-Substrat mit variierten Wasserstoffperoxidmengen fünf Minuten inkubiert. Nach Zugabe von Katalase (600 U) und einer weiteren fünfminütigen Inkubationszeit wurden zur Reaktionslösung 60 nmol 12-pNCA-Substrat pipettiert. Die Reaktion wurde fünf Minuten später durch Zugabe von 100 µl NADPH (1 mM) gestartet.

### g) Zn-Mediator-Untersuchungen

Die Reaktionen wurden in einer Schüttelapparatur für Eppendorf-Reaktionsgefäß-Schüttler (IKA-Labortechnik Vibrax mit Janke und Kunkel Aufsatz Typ VX2E) durchgeführt. Für Aktivitätsuntersuchungen wurde verfahren wie für die kinetischen Messungen beschrieben, nur wurden anstelle von NADPH variierte Mengen an Zink/Cobalt(III)sepulchrat benutzt. Nach variierten Zeitintervallen wurden 100 µl Probe der Reaktionssuspension entnommen und in ein weiteres 1,5 ml Eppendorf-Reaktionsgefäß pipettiert, in dem zum Reaktionsstop 10 µl KOH (6 M) vorgelegt waren. Nach einminütiger Zentrifugation bei 12000 g wurde der Überstand abgenommen und die Absorption bei 410 nm gemessen.

### Beispiel 1: Klonierung, Expression und Reinigung von P450 BM-3 und Mutanten im Gramm-Maßstab

### 1. Klonierung des p450 BM-3 Gens und der Mutante p450 BM-3 F87A und deren Expression in 30-l-Fermentationen

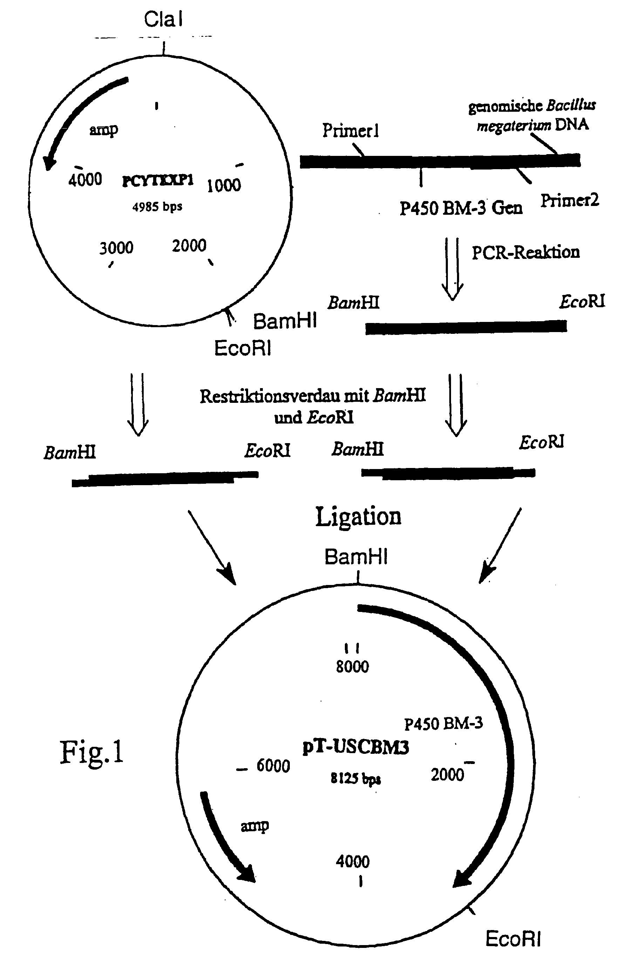

Das P450 BM-3 Gen wurde mittels PCR aus der genomischen DNA von *Bacillus megaterium* isoliert, mit Tags versehen und wie in Figur 1 für die pT-Plasmide gezeigt, in die Expressionsvektoren pCYTEXP1 und pASK-IBA1CA kloniert . Zur Überprüfung der korrekten Insertion des P450 BM-3 Gens, der mit unterschiedlichen Tags versehen P450 BM-3 Varianten und zur Überprüfung der P450 BM-3 F87A Punktmutante wurden die Sequenzierprimer R0_5 bis L7 verwendet. Für die PCR-Reaktionen nach dem Standard-Protokoll wurden für das Wildtyp-Enzym die Primer B1 und B2, für die P450 BM-3His₆ die Primer H1 und H2, für P450 BM-3Glu₆ die Primer G1 und G2, für P450 BM-3Arg₆ die Primer A1 und A2 und für P450 BM-3Strep die Primer S1 und S2 verwendet. Die Sequenzanalyse ergab keinerlei Mutationen für einen aus jeweils drei sequenzierten pT-USCOBM3- und pT-USC1BM3-Plasmiden. Für pT-USC2BM3 und pT-USC3BM3 wurde nur mit Primer R7 die korrekte Insertion des Tags überprüft. Der sequenzierte pA-USC4BM3-Klon zeigte zwei Mutationen im Reduktaseanteil des P450 BM-3 Proteins. Die Expressionsrate des mit einem His₆-Tag versehen P450 BM-3 in DH5α war mit Werten von 300 nmol pro Liter Fermenterbrühe um ca. 20 % höher als beim Wildtyp. Aufgrund dieser höheren Expressionsrate wurde pT-USC1BM3 zur Erhöhung der Sensitivität des pNCA-Tests an Position 87 durch Austausch eines Phenylalanins gegen Alanin mit den Stratagene QuikChange-Kit punktmutiert. Durch die PCR-Reaktion mit den Primern F87A1 und F87A2 enthält pT-USC1BM3F87A an der Mutationsposition ohne weitere Veränderung der Proteinsequenz eine zusätzliche EaeI-Restriktionsschnittstelle, die zur Selektion des zur Sequenzierung ausgewählten Klons verwendet wurde. Diese zusätzliche Restriktionsschnittstelle führt nach Restriktionsspaltung mit *Eae*I zum Auftreten einer neuen Bande bei ca. 800 bp in Spur 2 und einer gegenüber dem Wildtyp-Verdau in Spur 1 deutlich kleineren Bande bei 1,7 kbp. Nach Plasmidisolation wurden acht Klone mit *Eae*I verdaut (2 µl EaeI-Puffer (10x), 1 µl *Eae*I 3,2 µl pT-USC1BM3, ad 20 µl ddH₂O, 1 h bei 37°C). Ein Klon wurde sequenziert. Das pT-USC1BM3F87A Plasmid besitzt in der Linker-Region an Position 470 eine zusätzliche Mutation R470C. Auf eine Rückmutation wurde verzichtet aufgrund der Lokalisation der Mutation R470C am Ende der Linker-Region, die den P450-Anteil des P450 BM-3 mit dem Reduktaseanteil verbindet und keinerlei Einfluß auf die P450 BM-3 Aktivität zeigt.

### 2. Reinigung von P450 BM-3 und P450 BM-3 F87A

Ziel war es, eine kostengünstige, rasche und im präparativen Maßstab durchführbare Methode zur Reinigung von P450 BM-3 und P450 BM-3 F87A zu entwickeln. Dazu wurde ein Arg₆-, His₆-, Glu₆- und Strep-Tag an das C-terminale Ende von P450 BM-3 angefügt. Auf der Basis des His₆- und Strep-Tag sollten Affinitätsreinigungen und auf Basis des Arg₆- und Glu₆-Tags chromatographiesche Reinigungen mit Ionenaustauschern entwickelt werden. Ein Einfügen eines Tags an das N-terminale Ende des P450 BM-3 Gens wurde wegen der Lokalisation des

N-Terminus im Bereich des flexiblen Substrateingangskanals nicht in Betracht gezogen. Eine Modifikation in diesem Bereich würde mit hoher Wahrscheinlichkeit zu einer Änderung der P450 BM-3 Aktivität und Selektivität führen.

### 2.1 Proteinreinigungen mit Affinitätschromatographie

### a) P450 BM-3 mit His₆-Tag

Bei der P450 BM-3His₆-Reinigung über Metallchelatchromatographie binden mehr als 95 % der aufgetragenen 4 mg P450 BM-3 nicht an die 20 ml XK16/20-Affinitätssäule bei einem linearen 0-0,5 molaren Imidazolgradienten (10CV). Die chromatographische Reinigung mit Zn²⁺-Ionen wurde nach dem Pharmacia-Standardprotokoll durchgeführt. Das wenige aufgereinigte P450 BM-3 zeigte im NADPH-Assay keine Aktivität mehr. Die Ursache dieser Inhibierung konnte auf den Einfluß von Imidazol zurückgeführt werden. Imidazol bewirkt ein Ablösen des fünften Cysteinat-Liganden am katalytischen Häm-Zentrum. Diese Abdissoziation des fünften Liganden vom Häm-System führt zur sofortigen P450-Inaktivierung und zeigt sich im CO-Differenzspektrum durch eine charakteristische Verschiebung des Absorptionsmaximums von 448 nm nach 420 nm.

### b) P450 BM-3 mit Strep-Tag

Zur Aufreinigung wurden 8,2 mg P450 BM-3Strep verwendet. Vergleichbar den Ergebnissen der His₆-Tag Reinigungen zeigte P450 BM-3Strep keine Bindung an die Streptavidin-Affinitätsmatrix bei der Reinigung entsprechend dem READY TO USE KIT (IBA, Göttingen).

### c) P450 BM-3 mit Glu₆-Tag

Ein Vergleich der linearen 0-1 M NaCl-Gradienten (12 CV, Tris/HCl Puffer (0,1 M, pH 8,0)) zeigte für P450 BM-3Glu₆ bei Verwendung von Super 650M- und DEAE 650M-Anionenaustauschern keine Unterschiede im Elutionsverhalten zum Wildtyp-Enzym.

### d) Schlußfolgerungen

Die Ergebnisse der Reinigung von P450 BM-3 mit His₆-, Glu₆- und Strep-Tag lassen darauf schließen, daß das C-terminale Ende von P450 BM-3 nicht frei zugänglich an der Proteinoberfläche liegt und somit nicht für Reinigungszwecke verwendbar ist. Ferner verursachte im Falle des His₆-Tags die Elution mittels Imidazol eine rasche Proteininaktivierung durch Verdrängung des fünften Liganden am Porphyrin-System.

### 2.2 Anionenaustauschchromatographie

Um die Kosten zu minimieren, sollte eine effektive, kostengünstige und zeitsparende Reinigungsmethode für P450 BM-3 entwickelt werden, die eine einfache Maßstabsvergrößerung erlaubt. Als Methoden zur P450 BM-3 Reinigung standen nach den Ergebnissen der Reinigung von P450 BM-3 mit Tags somit noch nur HIC-Materialien und Ionenaustauscher zur Verfügung. Die Entscheidung fiel zugunsten der Anionenaustauscher, die in der Regel billiger sind und ein einfacheres Handling bieten.

### a) Linearer und Stufengradient

Um eine Anionenaustauschchromatographiemethode zu entwickeln wurden vier unterschiedlich starke Anionenaustauschermaterialien DEAE 650S (35 µm), DEAE 650M (65 µm), SuperQ 650M (65 µm) und QAE 550M (65 µm) auf ihre Eignung hin untersucht. In ersten Experimenten wurde mit jedem Säulenmaterial ein 10-Säulen-Volumen (CV) langer linearer 0-1 M NaCl-Gradient zur Reinigung von 6-10 mg P450 BM-3 Rohextrakt in einem Tris/HCl-Puffer (0,1 M, pH 7, 8) aufgenommen. In allen Fällen betrug die Ausbeute 80-84 %. Die höchsten Proteinanteile im P450 BM-3 Elutionspeak zeigte das DEAE-Material, dicht gefolgt vom SuperQ- und QAE-Material.

Für die weitere Verbesserung der Auflösung mittels gestufter Salzgradienten war die Fähigkeit des ÄKTA*explorer*-Systems hilfreich, gleichzeitig Leitfähigkeit und Absorption bei 280 nm und 417 nm aufzuzeichnen. Ein optimierter Zwei-Stufen-Salzgradient für die DEAE 650M Chromatographiesäule besitzt eine erste Stufe von 150 mM NaCl und eine zweite mit 250 mM NaCl. Die P450 BM-3 Elution beginnt, wie aus den Leitfähigkeitswerten des linearen NaCl-Salzgradienten ablesbar, bei einer Erhöhung der NaCl-Konzentration von 150 mM auf 170 mM. Die minimale obere Salzstufe wurde zu 250 mM NaCl ermittelt; NaCl-Konzentrationen < 230 mM verursachen eine Verbreiterung des P450 BM-3 Elutionspeaks, und NaCl-Konzentrationen > 300 mM führen zu einem deutlich schlechteren Reinigungseffekt. Die Salzstufen wurden für die Chromatographiematerialien SuperQ 650M und QAE 550M in vergleichbarer Weise optimiert.

Ein Vergleich des linearen DEAE 650M mit dem gestuften NaCl-Gradienten führt zu einer Erhöhung des P450 BM-3 Proteinanteils im Elutionspeak von 39 % beim linearen, auf 84 % beim gestuften Gradienten. Vergleichbare Ergebnisse wurden mit dem SuperQ 650M Chromatographiematerial erreicht; das QAE 550M Chromatographiematerial zeigte deutlich schlechtere Reinigungsergebnisse. Bei den gestuften Reinigungsmethoden lagen die Ausbeuten in allen Fällen bei > 78 %; die höchsten Ausbeuten von 93 % an P450 BM-3 im Elutionspeak wurden mit DEAE 650S erzielt.

### Beispel 2: Entwicklung eines spektroskopischen Aktivitätsnachweises für P450 BM-3 und P450 BM-3 F87A

Ziel war es einen spektrometrischen oder fluorimetrischen Aktivitätsnachweis für P450 BM-3 zu entwickeln. Ein solcher Aktivitätsnachweis kann im Gegensatz zu Standardmethoden wie HPLC oder GC einfach automatisiert und somit zum Auffinden von weiteren P450 BM-3 Enzymvarianten verwendet werden.

Um einen neuen Aktivitätsnachweis für P450 BM-3 zu entwickeln, wurden verschiedene Verbindungen, die am terminalen C-Atom der Carbonsäuren das Chromophor tragen, synthetisiert und auf ihre Eignung untersucht.

Arbeiten von Oliver et al. Biochemistry (1997), 36(7):1567 zeigten für Laurinsäure und Myristinsäure als Substrat eine Verschiebung des Hydroxylierungsprofils von P450 BM-3 von subterminalen Positionen zur terminalen Position, falls Phenylalanin an Position 87 durch Alanin ersetzt wurde. Zur Erhöhung der Sensitivität der pNCA-Nachweismethode wurde diese Punktmutation, wie in Material und Methoden beschrieben, eingefügt und mittels Sequenzanalyse bestätigt.

### 1. Nachweisprinzip

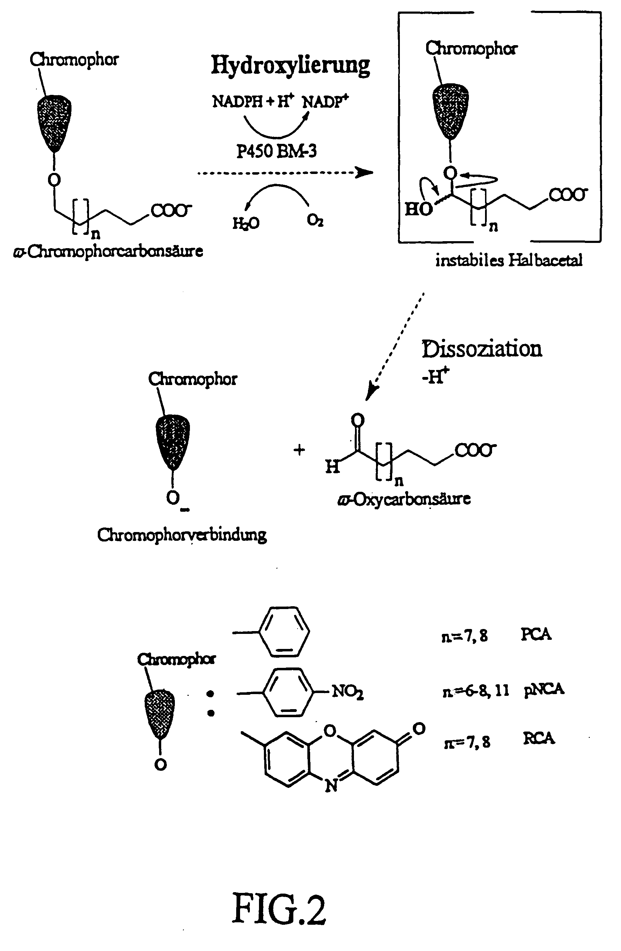

Figur 2 zeigt das Prinzip zu einem spektrometrischen Aktivitätsnachweis für terminal hydroxylierende Fettsäurehydroxylasen. Nach terminaler Hydroxylierung entsteht ein instabiles Halbacetal, das in die ω-Oxycarbonsäure und das spektrometisch detektierbare Chromophor dissoziert.

### 2. Nachweis der P450 BM-3 F87A Aktivität mit PCA

### a) Synthese der Phenoxycarbonsäuren (PCA)

Die PCA-Verbindungen wurden in einer einstufigen Synthese, wie in Referenzbeispiel 5 beschrieben, ausgehend von ω-Bromcarbonsäuren synthetisiert. Die Synthesen gelangen in Gesamtausbeuten zwischen 76 und 78 %. Charakterisiert wurden die PCAs mittels ¹H-NMR, ¹³C-NMR und Schmelzpunktbestimmung.

### b) Wahl der Reaktionsbedingungen

Um die Phenolbildung spektroskopisch nachweisen zu können, bietet es sich an, die Absorption im Wellenlängenbereich zwischen 250 und 280 nm zu messen (Luchter-Wasylewska 1996). Der geringe Extinktionskoeffizient am Absorptionsmaxium bei 274 nm von ε=1090 M⁻¹cm⁻¹, das unterschiedliche Absorptionsverhalten von NADPH und NADP⁺ in diesem Wellenbereich und die Bildung von Wasserstoffperoxid in ungekoppelten Reaktionen verhinderten jedoch eine kontinuierliche spektroskopische Messung der Phenolbildung. Aus diesen Gründen wurde der Merck-Phenol-Nachweis Kit, zur Quantifizierung der Produktbildung verwendet. Mit NADPH als Cofaktor ergaben sich bei Verwendung der P450 BM-3 Mutante F87A für 12-PCA Umsatzgeschwindigkeiten von 640 eq/min und für 11-PCA von 410 eq/min.

### 3. Nachweis der P450 BM-3 und P450 BM-3 F87A Aktivität mit pNCA-Substraten

### a) Synthese der p-Nitrophenoxycarbonsäuren (pNCA)

Die pNCA-Verbindungen wurden in einer neuen, dreistufigen Synthese, wie in Referenzbeispiel 5 beschrieben, ausgehend von ω-Bromcarbonsäuren durch Veresterung, sich anschließender Sₙ2-Reaktion mit dem Natrium-*p*-Nitrophenolat und lipasekatalysierter Hydrolyse der Ester, synthetisiert. Die dreistufigen pNCA-Synthesen gelangen in Gesamtausbeuten zwischen 61 und 69 %. Charakterisiert wurden die pNCAs mittels ¹H-NMR, ¹³C-NMR und Schmelzpunktbestimmung.

### b) Wahl der Reaktionsbedingungen

In Figur 2 ist das Reaktionsprinzip der Umsetzung von pNCA-Substraten durch P450 BM-3 und P450 BM-3 F87A beschrieben. Für die Aktivitätsmessungen mußten pH-Wert und Wellenlänge des spektroskopischen Nachweissystems optimiert werden. Nur deprotoniertes *p*-Nitrophenolat (pKs-Wert 7,1) trägt zur gelben Farbe bei; für die Nachweisempfindlichkeit wäre somit ein pH-Wert >9,1 (>99 % deprotoniert) empfehlenswert. Die pNCA-Verbindungen sind in stark alkalischen Lösungen stabil, im Gegensatz zum P450 BM-3 Protein, das bei pH 10 und 30°C innerhalb von 5 min 80 % seiner Aktivität verliert. Als Kompromiß zwischen Proteinstabilität und Sensitivität der Nachweismethode wurde ein pH-Wert von 8,1 bis 8,2 gewählt. Berechnungen auf der Basis der Henderson-Hasselbalch-Gleichung zeigen, daß in diesem pH-Bereich 90-92 % des umgesetzten *p*-Nitrophenols deprotoniert sind und zur gelben Farbe beitragen. Das Absorptionsmaximum von *p*-Nitro-phenolat liegt bei 400 nm. Bei dieser Wellenlänge absorbieren jedoch auch das Häm-Zentrum und der Cofaktor NADPH. P450 BM-3 besitzt ein starkes Absorptionsverhalten zwischen 350 und 450 nm, das in Abhängigkeit von Substratbindung und Oxidationsstufe des Eisens im katalytischen Zentrum stark schwankt. Das Absorptionsspektrum von NADPH begrenzt den Wellenlängenbereich auf >390 nm. Die Wellenlänge 410 nm wurde aufgrund der Reproduzierbarkeit letztendlich zur Aktivitätsmessung ausgewählt. Falls die Sensitivität des Nachweissystems nicht ausreichend ist, sollte bei Wellenlängen im Bereich von 400 nm gearbeitet werden. Unter den gewählten Nachweisbedingungen (pH 8,2, 410 nm) wurde der Extinktionskoeffizient bestimmt zu ε= 13200 M⁻¹cm⁻¹.

pNCAs besitzen nur eine geringe Wasserlöslichkeit, so daß ein Lösungsvermittler benötigt wird. Dazu kann DMSO ohne Beeinflussung der P450 BM-3 F87A Aktivität bis zu Konzentrationen von 1 % (v/v) zugegeben werden. 0,8 % (v/v) DMSO wurden deshalb als Lösungsvermittler im Aktivitätsnachweissystem verwendet.

Der Zeitbedarf des pNCA-Nachweissystems hängt stark von der verwendeten Enzymmenge ab. Falls nmol-Mengen an P450 BM-3 F87A im pNCA-Aktivitätsnachweis eingesetzt wurde, war 1 min zum Aktivitätsnachweis ausreichend.

### c) Automatisierung des pNCA-Nachweises

Für den Einsatz in einer HTS-Umgebung wurde der pNCA-Aktivitätsnachweis unter Benutzung einer Biomek2000 Arbeitsstation und eines BMG FluoStar Mikrotiterplattenleser automatisiert. Unter Verwendung von P450 BM-3 und der Mutante F87A wurde in jeweils 16 Messungen mit den pNCA-Substraten 10-, 11-, 12- und 15-pNCA die Reproduzierbarkeit des Assaysystems untersucht. Bei den Gesamtabsorptionen gab es aufgrund von Pipettierungenauigkeiten Unterschiede um den Faktor zwei bis vier, die Reaktivitätsunterschiede schwankten jedoch nur zwischen 2 und 6 %. Die beste Reproduzierbarkeit wurde für die P450 BM-3 F87A Mutante erzielt mit Abweichungen - 2 % und die schlechteste für den Wildtyp und 15-pNCA mit Abweichungen von bis zu 6 % vom Mittelwert aller Messungen. Ursächlich für die Genauigkeitsunterschiede sind die unterschiedliche Löslichkeit der pNCA-Substrate und verschiedene Umsätze in Abhängikeit von der Kettenlänge.

Die F87A Mutante setzt 12-pNCA vollständig um, während das Wildtyp-Enzym nach 33 % stoppt. Die Ursache liegt vermutlich in subterminal hydroxylierten 12-pNCA-Verbindungen, die nicht mehr oder nur noch langsam weiter umgesetzt werden. Für P450 BM-3 und P450 BM-3 F87A sind das pNCA-Kettenlängenprofil und der Umsatz aus ω-Hydroxylierung der pNCAs, berechnet aus dem Quotienten von beobachteten zu gemessener Gelbfärbung der Lösung in den folgenden Tabellen 4 und 5 zusammengefaßt. Zusätzlich wurden für P450 F87A die kinetischen Daten aus den Lineweaver-Burk-Diagrammen ermittelt.

**Tabelle 4**

| Kettenlänge der pNCA-Verbindung | P450 BM-3 F87A k_{cat} [eq^{a}/min] | P450 BM-3 F87A ω-Hydroxylierungsprodukte [%] | P450 BM-3 Wildtyp k_{cat} [eq^{a}/min] | P450 BM-3 Wildtyp ω-Hydroxylierungsprodukte [%] |
|---|---|---|---|---|
| 6 | nicht umgesetzt | nicht umgesetzt | nicht umgesetzt | nicht umgesetzt |
| 8 | 1 | <1 | 2 | <1 |
| 10 | 426 | 94 | 322 | 98 |
| 11 | 204 | 64 | 267 | 63 |
| 12 | 574 | 100 | 114 | 33 |
| 15 | 680 | 61 | 246 | 35 |

| | | | | |
|---|---|---|---|---|
| a anstatt des Ausdrucks nmol (Substrat)/nmol (P450) wird Equivalente (eq) verwendet | | | | |

**Tabelle 5**

| Kettenlänge der pNCA-Verbindung | P450 BM-3 F87A k_{cat} [eq^{a}/min] | Kₘ [mM] | k_{cat}/Kₘ [M⁻¹*min⁻¹] |
|---|---|---|---|
| 10 | 426 ± 32 | 42 ± 8 | 1.0*10⁷ |
| 11 | 204 ± 18 | 69 ± 7 | 3.4*10⁵ |
| 12 | 574 ± 47 | 6,2 ± 0,3 | 1.1*10⁴ |
| 15 | 680 | - | - |

| | | | |
|---|---|---|---|
| a anstatt des Ausdrucks nmol (Substrat)/nmol (P450) wird Equivalente (eq) verwendet | | | |

### Beispiel 3: Neues Cofaktor-System für P450 BM-3 und P450 BM-3 F87A

Ein Haupthindernis der industriellen Nutzung von P450-Systemen sind die Kosten, die der Cofaktor NADPH verursacht. Zur Lösung des Kostenproblems wurde ein alternatives Cofaktor-Konzept entwickelt, bei dem NADPH durch Zink und den Mediator Cobalt(III)sepulchrat ersetzt wurde; Zink dient als Elektronenquelle und Cobalt (III) sepulchrat als Elektronentransportsystem vom Zink zum P450 BM-3.

### 1. Nachweis der Reduktion von Cobalt(III)sepulchrat durch Zink

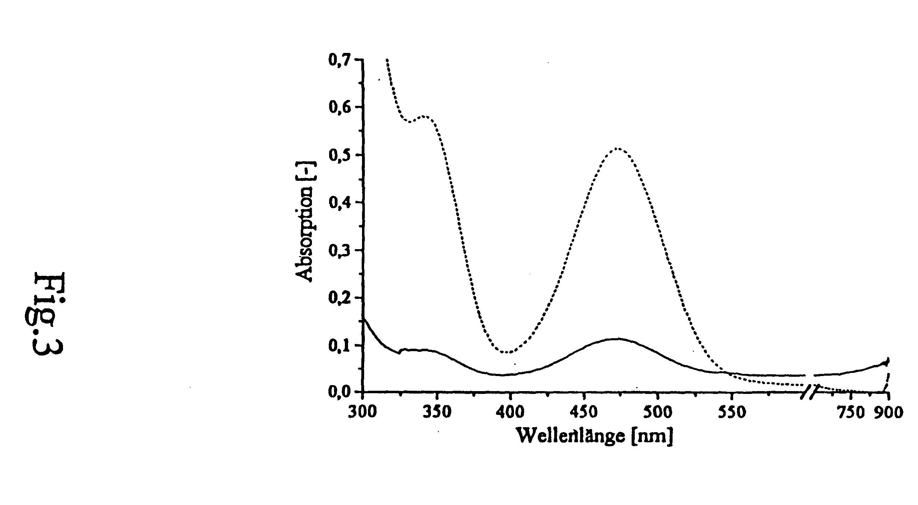

Cobalt(III)sepulchrat besitzt unter Standardbedingungen ein Normalpotential von -0,54 V gegen eine Kalomel-Elektrode (Creaser et al., J. Am. Chem. Soc. (1977) 99: 3181). Es läßt sich, wie in Abbildung 3 gezeigt, mittels Zn-Staub in einem Tris/HCl-Puffer (0,2 M, pH 8,2) innerhalb von Sekunden reduzieren. In Gegenwart von Luftsauerstoff in wäßrigen Lösungen wird es innerhalb weniger Minuten wieder zu Cobalt(III)sepulchrat oxidiert.

### 2. Nachweis der P450 BM-3 F87A Aktivität mit alternativem Cofaktor-System

### 2.1 pNCA-Assay

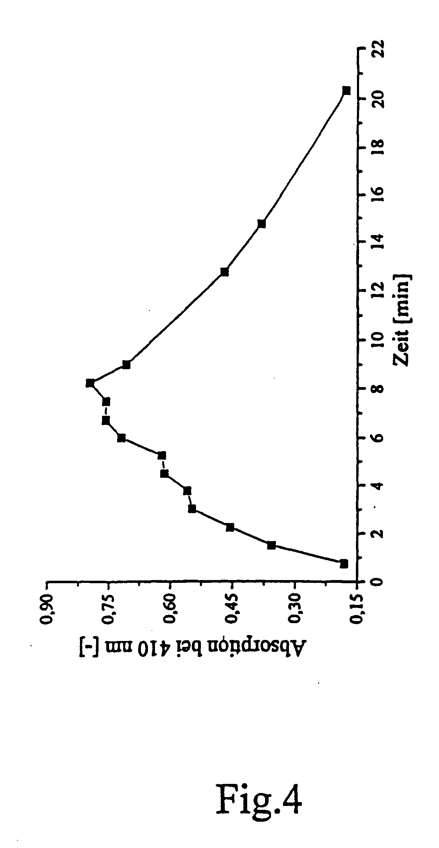

Figur 4 zeigt die Umsetzung von 12-pNCA mittels des Zn/Cobalt(III)sepulchrat/P450 BM-3 F87A Systems. Bei einem Absorptionsmaximum von 0,8 nach 8 min zeigt das Zn/Cobalt(III)sepulchrat/P450 BM-3 F87A System eine gelbe Farbe, die einem 70%igen Umsatz entspricht. Innerhalb von 20 min verschwindet die durch *p*-Nitrophenolat verursachte gelbe Farbe völlig; der Restabsorptionswert von 0,15 stammt vom Mediator. Kontrollexperimente zeigten, daß Zn-Pulver die Nitrogruppe von *p*-Nitrophenolat im Tris/HCl-Puffer (pH 8,2, 50 mM, 0,25 M KCl) reduziert. Weitere Kontrollexperimente zeigten, daß Wasserstoffperoxid keinen oxidativen Einfluß auf *p*-Nitrophenolat und pNCAs hat.

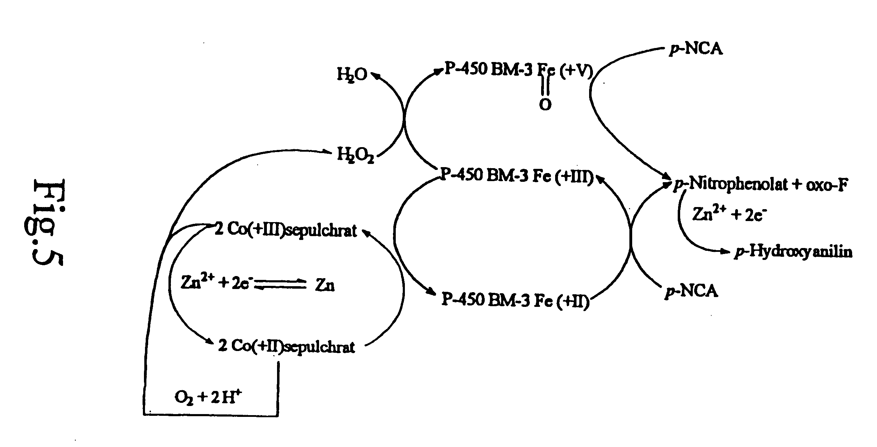

In Figur 5 sind die beschriebenen Elektronentransferwege von Zn über den Mediator zum P450 BM-3 und der Substratumsetzung unter Berücksichtigung des verkürzten Wasserstoffperoxid-Reaktionswegs ("shunt-pathway") dargestellt.

### 2.2 PCA-Assay

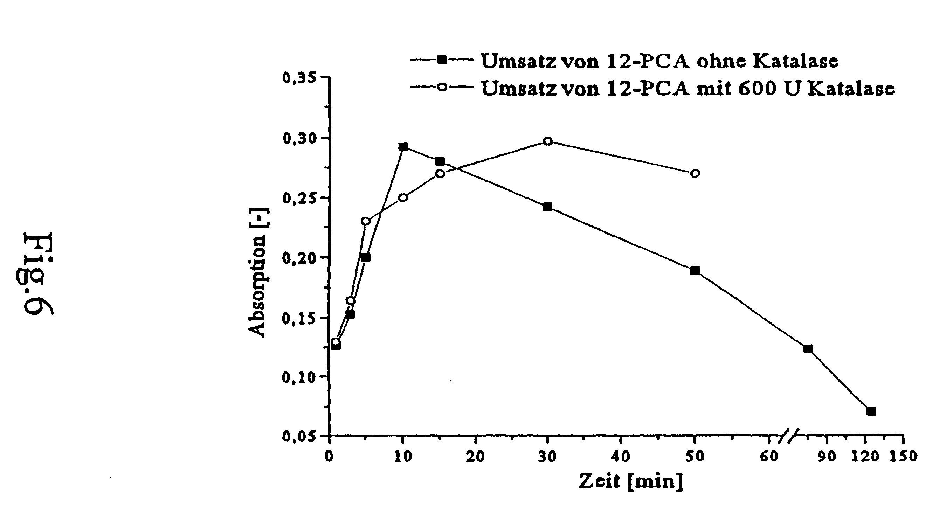

Aufgrund der Reduktion von *p*-Nitrophenol durch Zink in einem Tris/HCl-Puffer wurde 12-pNCA durch 12-PCA ersetzt. Der Phenolnachweis erfolgte mittels des Merck-Phenolnachweis-Kits bei 495 nm. Ein direkter photometrischer Phenolnachweis bei 274 nm ist aufgrund des Absorptionsverhaltens des oxidierten und reduzierten Cobalt(III)sepulchrat nicht möglich. Figur 6 verdeutlicht für 12-PCA als Substrat eine im Vergleich zu 12-pNCA langsame Entfärbung (Figur 4). Als Ursache für die Absorptionsabnahme konnte Wasserstoffperoxid identifiziert werden, das durch reduziertes Cobalt(II)sepulchrat in Nebenreaktionen durch Reduktion von Luftsauerstoff in wäßrigen Lösungen gebildet wird. Ferner tritt Wasserstoffperoxid-Bildung bei P450-Systemen infolge von ungekoppelten Reaktionen als Nebenreaktionsprodukt immer auf. Aus letzterem Grund wurde eine weitere Optimierung der PCA-Reaktionsbedingungen nicht weiter verfolgt.

In Gegenwart von 600 U Katalase erfolgt, wie in Figur 6 gezeigt, die Absorptionsabnahme bei 495 nm deutlich langsamer.

### 3.1 Pufferzusammensetzung

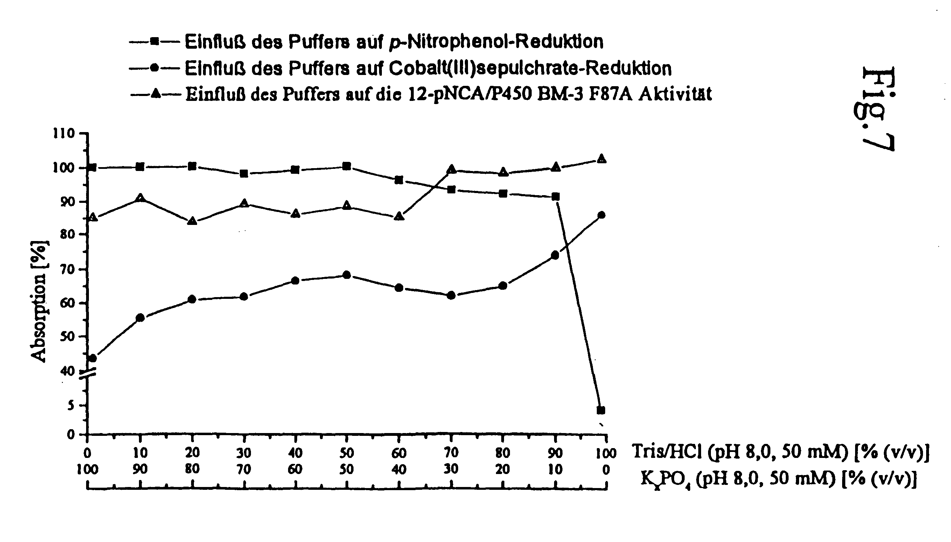

Um die Entfärbung der Lösung durch die Reduktion von p-Nitrophenolat mittels Zink zu vermeiden, wurde zunächst erfolglos die KCl-Konzentration und der pH-Wert im Rahmen der P450 BM-3 F87A Stabilität variiert (6,5-8,5). Erfolgreich, wie in Figur 7 gezeigt, kann die Reduktion von *p*-Nitrophenol durch Zink mittels Zusätzen an Kaliumphosphat-Puffer verhindert werden. Ab einem Kaliumphosphatanteil ≥ 10 % wird *p*-Nitrophenol nur noch in geringem Ausmaß (≤10 %) durch Zn reduziert und bei einer 1:1 Mischung ist selbst nach 14 h keine weitere Reduktion mehr feststellbar (nicht abgebildet). Wie in Figur 7 gezeigt, verringert sich der Anteil an reduziertem Co(II)sepulchrat im Mischungsbereich bis zu 20 % Kaliumphosphat zunächst deutlich um ca. -25 %, um dann zwischen 20 und 70 % (v/v) Kaliumphosphat-Anteil konstant zwischen 60 bis 70 % zu verharren. Die im Vergleich zum Tris/HCl-Puffersystem geringere Verfügbarkeit an reduziertem Cobalt(II)sepulchrat führt zu einem Aktivitätsverlust von ca. 15 %.

Aus obigen Ergebnissen wurde für die weiteren Optimierungen eine 1:1 Mischung beider Pufferlösungen verwendet.

### 3.2 Einfluß der Mediatorkonzentration auf den Umsatz und die P450 BM-3 F87A Aktivität

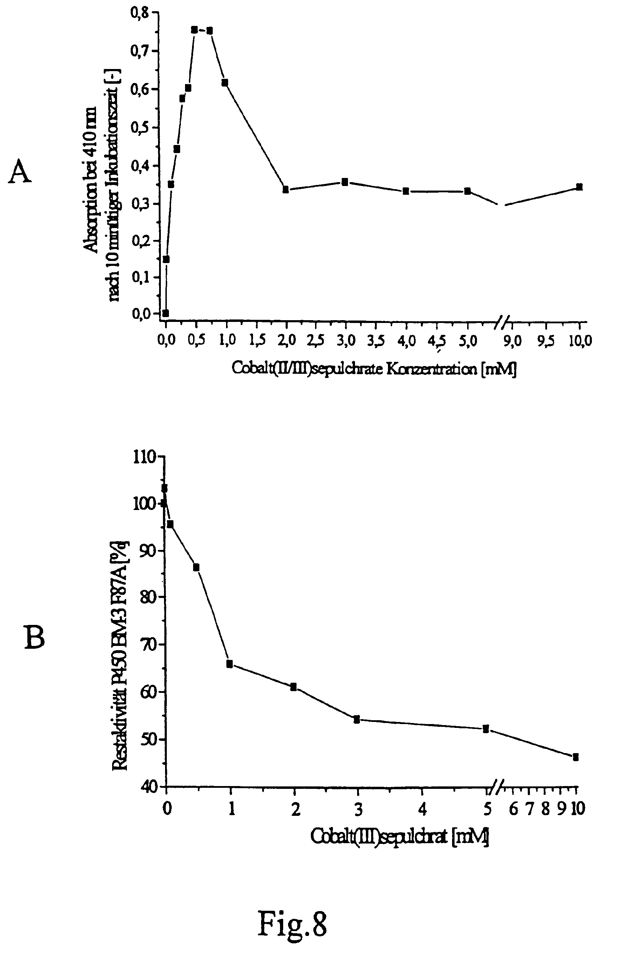

Figur 8A zeigt den Einfluß des Cobalt(III)sepulchrat Mediators auf die P450 BM-3 Aktivität im NADPH-Assay bei variierten Mediatorkonzentrationen. Bei Cobalt(III)sepulchrat-Konzentrationen ≥ 5 mM bildete sich während des Reaktionsablaufs ein kolloidaler Niederschlag, der vor Absorptionsmessung mittels Zentrifugation abgetrennt wurde. Für Umsetzungen mit dem Zink/Cobalt(III)sepulchrat-System ist eine optimale Cobalt(III)-sepulchrat-Konzentration im Bereich von 0,5-0,75 mM pro 0,072 nmol P450 BM-3 F87A vorhanden. Wie in Figur 8B gezeigt, inhibiert Cobalt(III)sepulchrat bis zu Konzentrationen ≤ 0,5 mM P450 BM-3 F87A selbst in Abwesenheit von Substrat nur geringfügig. Der Inhibierungsgrad bleibt bei variierten Inkubationszeiten konstant und hängt nur vom Konzentrationsverhältnis Cobalt(III)sepulchrat zu P450 BM-3 F87A ab.

### 3.3 Zn-Konzentration

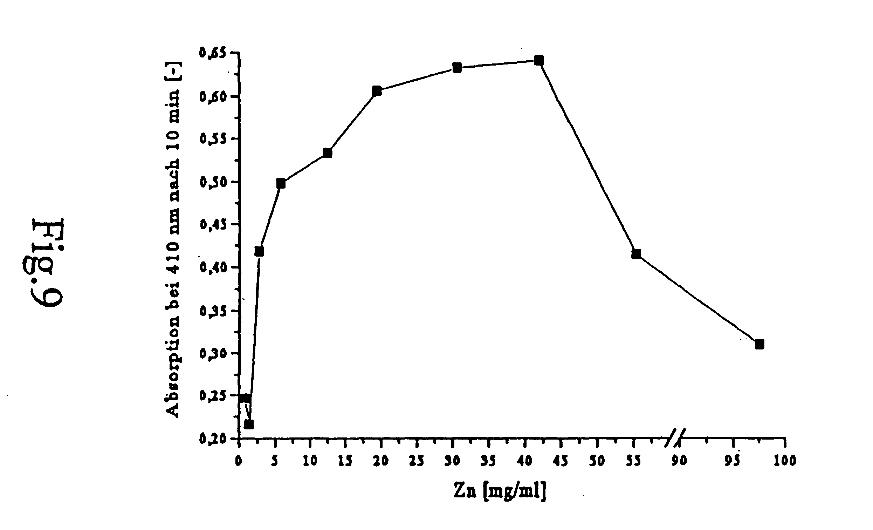

Die Abhängigkeit des Umsatzes von 0,072 nmol P450 BM-3 F87A von der eingesetzten Zinkpulvermenge ist in Figur 9 gezeigt. Ein optimaler Umsatz wurde im Bereich von 20 bis 40 mg Zink pro Milliliter Reaktionslösung erzielt. Für die Umsatzrückgänge bei 50 und100 mg war vermutlich eine abnehmende Sauerstoffkonzentration in der Reaktionslösung durch zunehmende mediatorkatalysierte Wasserstoffperoxidbildung verantwortlich.

Ein Ersatz von Zinkpulver durch Zinkgranalien führte selbst bei Verwendung von 100fach erhöhten Mengen nur zu sehr geringen Umsatzgeschwindigkeiten (Faktor >20 geringer).

### 3.4 Einfluß der H₂O₂-Konzentration

Um den Anteil den Wasserstoffperoxid über den 'shuntA Reaktionsweg zur 12-pNCA- Umsetzung durch P450 BM-3 F87A beiträgt, zu bestimmen, wurden 12-pNCA und P450 BM-3 F87A mit variierten Wasserstoffperoxid-Konzentrationen inkubiert. Im Bereich von 8-20 µM Wasserstoffperoxid wird bei vergleichbaren P450 BM-3 F87A Aktivitäten nach einer Minute bei einem 20-25%igen Umsatz eine Plateau erreicht, unabhängig von der Wasserstoffperoxid-Konzentration. Die weitere Zugabe von NADPH im Überschuß (100 µM) zeigt, daß P450 BM-3 F87A durch Wasserstoffperoxid nicht vollständig inaktiviert wird. Die Restaktivitäten sind bei geringen Wasserstoffperoxid-Konzentration hoch und sinken mit zunehmender Wasserstoffperoxid-Konzentration (nicht gezeigt).

Um die Stabilität von P450 BM-3 gegenüber Wasserstoffperoxid zu bestimmen, wurde P450 BM-3 F87A in Abwesenheit von Substrat (12-pNCA) 5 min mit variierten Wasserstoffperoxid-mengen inkubiert. Nach Zugabe von Katalase (600 U) und 60 nmol Substrat wurden die Umsatzgeschwindigkeiten unter Verwendung von NADPH als Elektronendonor bestimmt.

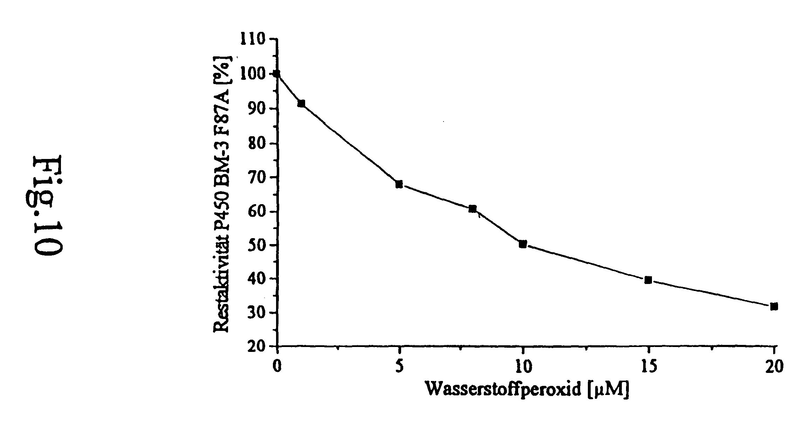

Wie in Figur 10 gezeigt, inhibieren in Abwesenheit von Substrat bereits Wasserstoffperoxid-Konzentrationen im 5 µM-Bereich die P450 BM-3 F87A Aktivität deutlich. Als Konsequenz ergibt sich hieraus für einen P450 BM-3 F87A Enzym-Membran-Reaktor, bei möglichst hohen Substratkonzentrationen zu arbeiten und die wasserstoffperoxidbildung durch Katalase- oder Anti-Oxidant-Zugabe zu minimieren.

### Beispiel 4: Aktivitätstest mit weiteren P450 BM-3 Mutanten

Analog Beispiel 2 wurden weitere BM-3 Mutanten sowie das Wildtyp-Enzym (WT) auf Aktivität gegenüber pNCA-Derivaten verschiedener Kettenlänge getestet. Die Ergebnisse sind in folgender Tabelle 6 zusammengefasst:

**Tabelle 6**

| Spezifische Aktivität einzelner P450 BM-3 Mutanten für pNCAs verschiedener Kettenlänge | | | | | | | | |
|---|---|---|---|---|---|---|---|---|
| | WT | F87A | L188K | V26T | R47F | S72G | A74G | M354T |
| 15-pNCA | 405¹ | 410 | 288 | 519 | 258 | 439 | 474 | 560 |
| 12-pNCA | 141 | 284 | 316 | 555 | 233 | 596 | 517 | 480 |
| 10-pNCA | 339 | 92 | 207 | 106 | 52 | 150 | 103 | 171 |
| 8-pNCA | 15 | 2 | 69 | 16 | 13 | 3 | 6 | 4 |

| | | | | | | | | |
|---|---|---|---|---|---|---|---|---|
| ¹ Spezifische Aktivität: nmol/min/nmol P450. | | | | | | | | |

## Patentansprüche

1. Elektronendonorsystem für die Übertragung von Elektronen auf Enzyme mit Redox-Eigenschaften, **dadurch gekennzeichnet, daß** das System eine anorganische, nicht elektrodengebundene Elektronenquelle und einen Mediator umfaßt, der zur Übertragung von Elektronen von der Elektronenquelle auf das Enzym befähigt ist, wobei das Enzym eine Cytochrom P450-haltige Monooxygenase (E.C. 1.14.-.-) ist, und die Elektronenquelle ein Metall mit einem niedrigeren Standard-Normalpotential als der Mediator ist.

2. Elektronendonorsystem nach Anspruch 1, **dadurch gekennzeichnet, daß** der Mediator ein Standard-Normalpotential im Bereich von weniger als etwa -0,4 V besitzt.

3. Elektronendonorsystem nach einem der vorhergehenden Ansprüche, **dadurch gekennzeichnet, daß** der Mediator ausgewählt ist unter Kobalt(III)sepulchrat, Methylviologen, Neutralrot, Riboflavin, Rutheniumtriacetat, FMN und FAD.

4. Elektronendonorsystem nach Anspruch 1, **dadurch gekennzeichnet, daß** die Elektronenquelle metallisches Zink ist.

5. Elektronendonorsystem, nach einem der vorhergehenden Ansprüche, ausgewählt unter den Systemen:
- Zn/Kobalt(III)sepulchrat und
- Zn/Neutralrot.

6. Verfahren zur enzymatischen Übertragung von Sauerstoff auf ein Kohlenwasserstoff-haltiges Wasserstoffdonor-Molekül, **dadurch gekennzeichnet, daß** man das Wasserstoff-Donormolekül in einem Reaktionsmedium, umfassend das Sauerstoff-übertragenden Enzym und ein Elektronendonorsystem nach einem der Ansprüche 1 bis 5, in Gegenwart von Sauerstoff unter Reaktionsbedingungen inkubiert.

7. Verfahren nach Anspruch 6, **dadurch gekennzeichnet, daß** das Wasserstoff-Donormolekül ausgewählt ist unter Verbindungen der Formel
R-X
worin
R für einen Alkylrest mit 8 oder mehr Kohlenstoffatomen steht, und
X für eine polare, zur Ausbildung von Wasserstoffbrücken befähigte Gruppe, vorzugsweise eine Carboxy-, Amid-, Nitril-, Sulfat-, Sulfon-, Amin- oder Hydroxygruppe, steht.

8. Verfahren zur enzymatischen Herstellung terminal oder subterminal (Position ω-1 bis ω-4) hydroxylierter Fettsäuren, **dadurch gekennzeichnet, daß** man
a) eine hydroxylierbare Fettsäure oder Fettsäurederivat in Gegenwart eines Elektronendonorsystems nach einem der Ansprüche 1 bis 5 mit einer Cytochrom P450 Monooxygenase und Sauerstoff umsetzt; und
b) das (die) hydroxylierte(n) Produkt(e) isoliert.

9. Verfahren nach Anspruch 8, **dadurch gekennzeichnet, daß** das ω-hydroxylierbare Fettsäurederivat ausgewählt ist unter terminal gesättigten, verzweigten oder unverzweigten Fettsäuren mit mehr als 10 Kohlenstoffatomen, insbesondere C₁₂ - C₃₀-Fettsäuren.

10. Verfahren nach einem der Ansprüche 6 bis 9, **dadurch gekennzeichnet, daß** das Enzym eine Cytochrom P450 Monooxygenase ist, ausgewählt unter:
a) dem aus Bacillus megaterium (DSM 32T) isolierbaren Wildtypenzym; oder
b) einer durch Aminosäuresubstitution in wenigstens einer der Positionen 26, 47, 72, 74, 87, 188 und 354 erhältlichen Mutanten des Wildtypenzyms (SEQ ID NO:35).

11. Verfahren nach Anspruch 10, **dadurch gekennzeichnet, daß** eine Einfachmutante eingesetzt wird, die ausgewählt ist unter F87A, F87V, L188K, V26T, R47F, S72G, A74G und M354T.

12. Verfahren nach Anspruch 10, **dadurch gekennzeichnet, daß** die Mutante in Position 87 die Mutation F87A oder F87V und wenigstens eine weitere der folgenden Mutationen aufweist: L188K, A74G, R47F und V26T.

13. Verfahren nach einem der Ansprüche 8 bis 12, **dadurch gekennzeichnet, daß** das Elektronendonorsystem Zink/Co(III)sepulchrat ist.

14. Verfahren nach einem der Ansprüche 8 bis 13, **dadurch gekennzeichnet, daß** man zumindest Stufe a) in Gegenwart von Chloridionen durchführt.

15. Verfahren nach einem der Ansprüche 8 bis 14, **dadurch gekennzeichnet, daß** man zumindest Stufe a) in Gegenwart eines Wasserstoffperoxyd-spaltenden Enzyms durchführt.

16. Bioreaktor zur Verwendung bei der Herstellung ω-hydroxylierter Fettsäuren, **gekennzeichnet durch** immobilisierte Monooxygenase und ein Elektronendonorsystem nach einem der Ansprüche 1 bis 5 im flüssigen Reaktionsmedium.

17. Nachweisverfahren für Fettsäure-Monooxygenasen, **dadurch gekennzeichnet, daß** man
a) einen Analyten, in dem man Enzymaktivität vermutet, mit einer ω-hydroxylierbaren Fettsäure oder Fettsäurederivat, welche(s) einen terminalen, abspaltbaren Chromophor oder Fluorophor trägt, in Gegenwart eines Elektronendonorsystems nach einem der Ansprüche 1 bis 5 inkubiert; und
b) die Abspaltung des Chromophors oder Fluorophors qualitativ oder quantitativ bestimmt.

18. Verfahren nach Anspruch 17, **dadurch gekennzeichnet, daß** man die Umsetzung in Gegenwart eines Wasserstoffperoxyd-spaltenden Enzyms und gegebenenfalls in Gegenwart von Chloridionen durchführt.

19. Testkit, umfassend ein Elektronendonorsystem nach einem der Ansprüche 1 bis 5.

## Claims

1. An electron donor system for transferring electrons to enzymes with redox properties, wherein the system comprises an inorganic, non-electrode-bound source of electrons and a mediator which is able to transfer electrons from the source of electrons to the enzyme, the enzyme being a cytochrome P450-containing monooxygenase (E.C. 1.14.-.-), and the source of electrons being a metal with a lower standard normal potential than the mediator.

2. The electron donor system according to claim 1, wherein the mediator has a standard normal potential in the region of less than about -0.4 V.

3. The electron donor system according to any of the preceding claims, wherein the mediator is selected from cobalt(III) sepulchrate, methylviologen, neutral red, riboflavin, ruthenium triacetate, FMN and FAD.

4. The electron donor system according to claim 6, wherein the source of electrons is metallic zinc.

5. The electron donor system according to any of the preceding claims, selected from the systems:
- Zn/cobalt(III) sepulchrate and
- Zn/neutral red.

6. A method for the enzymatic transfer of oxygen to a hydrocarbon-containing hydrogen donor molecule, which comprises incubating the hydrogen donor molecule in a reaction medium comprising the oxygen-transferring enzyme and an electron donor system according to any of claims 1 to 5 in the presence of oxygen under reaction conditions.

7. The method according to claim 6, wherein the hydrogen donor molecule is selected from compounds of the formula
R-X
in which
R is an alkyl radical with 8 or more carbon atoms, and
X is a polar group capable of forming hydrogen bonds, preferably a carboxyl, amide, nitrile, sulfate, sulfone, amine or hydroxyl group.

8. A method for the enzymatic production of terminally or subterminally (position ω-1 to ω-4) hydroxylated fatty acids, which comprises
a) converting a hydroxylatable fatty acid or fatty acid derivative in the presence of an electron donor system according to any of claims 1 to 5 using a cytochrome P450 monooxygenase and oxygen; and
b) isolating the hydroxylated product(s).

9. The method according to claim 8, wherein the ω-hydroxylatable fatty acid derivative is selected from terminally saturated, branched or unbranched fatty acids with more than 10 carbon atoms, in particular C₁₂ - C₃₀ fatty acids.

10. The method according to any of claims 6 to 9, wherein the enzyme is a cytochrome P450 monooxygenase selected from:
a) the wild-type enzyme which can be isolated from Bacillus megaterium (DSM 32T); or
b) a mutant, which can be obtained by amino acid substitution in at least one of positions 26, 47, 72, 74, 87, 188 and 354, of the wild-type enzyme (SEQ ID NO: 35).

11. The method according to claim 10, wherein a single mutant selected from F87A, F87V, L188K, V26T, R47F, S27G, A74G and M354T is employed.

12. The method according to claim 10, wherein the mutant has in position 87 the mutation F87A or F87V and at least one other of the following mutations: L188K, A74G, R47F and V26T.

13. The method according to any of claims 8 to 12, wherein the electron donor system is zinc/Co(III) sepulchrate.

14. The method according to any of claims 8 to 13, wherein at least stage a) is carried out in the presence of chloride ions.

15. The method according to any of claims 8 to 14, wherein at least stage a) is carried out in the presence of a hydrogen peroxide-cleaving enzyme.

16. A bioreactor for use for producing ω-hydroxylated fatty acids, which comprises immobilized monooxygenase and an electron donor system according to any of claims 1 to 5 in a liquid reaction medium.

17. A detection method for fatty acid monooxygenases, which comprises
a) incubating an analyte suspected of having enzymic activity with an ω-hydroxylatable fatty acid or fatty acid derivative which has a terminal chromophore or fluorophore which can be eliminated, in the presence of an electron donor system according to any of claims 1 to 5; and
b) determining the elimination of the chromophore or fluorophore qualitatively or quantitatively.

18. The method according to claim 17, wherein the conversion is carried out in the presence of a hydrogen peroxide-cleaving enzyme and, where appropriate, in the presence of chloride ions.

19. A test kit comprising an electron donor system according to any of claims 1 to 5.

## Revendications

1. Système donneur d'électrons pour le transfert d'électrons à des enzymes ayant des propriétés redox, **caractérisé en ce que** le système comprend une source inorganique d'électrons non reliée à des électrodes et un médiateur, qui est capable de transférer des électrons de la source d'électrons à l'enzyme, l'enzyme étant une monooxygénase contenant un cytochrome P450 (E.C.1.14.-.-), et la source d'électrons est un métal ayant un potentiel normal/standard inférieur à celui du médiateur.

2. Système donneur d'électrons selon la revendication 1, **caractérisé en ce que** le médiateur possède un potentiel normal/standard dans la plage de valeurs inférieures à environ -0,4 V.

3. Système donneur d'électrons selon l'une quelconque des revendications précédentes, **caractérisé en ce que** le médiateur est choisi parmi le sépulcrate de cobalt (III), le viologène de méthyle, le rouge neutre, la riboflavine, le triacétate de ruthénium, la FMN et la FAD.

4. Système donneur d'électrons selon la revendication 1, **caractérisé en ce que** la source d'électrons est du zinc métallique.

5. Système donneur d'électrons selon l'une quelconque des revendications précédentes, choisi parmi les systèmes :
- Zn/sépulcrate de cobalt(III) et
- Zn/rouge neutre.

6. Procédé de transfert enzymatique d'oxygène à une molécule donneuse d'hydrogène contenant des hydrocarbures, **caractérisé en ce que** l'on incube la molécule donneuse d'hydrogène dans un milieu réactionnel comprenant l'enzyme de transfert d'oxygène et un système donneur d'électrons selon l'une quelconque des revendications 1 à 5, en présence d'oxygène dans des conditions réactionnelles.

7. Procédé selon la revendication 6, **caractérisé en ce que** la molécule donneuse d'hydrogène est choisie parmi les composés de formule
R-X
dans laquelle
R représente un radical alkyle ayant 8 atomes de carbone ou plus, et
X représente un groupement polaire capable de former des ponts d'hydrogène, de préférence, un groupement carboxy, amide, nitrile, sulfate, sulfone, amine ou hydroxy.

8. Procédé de fabrication enzymatique d'acides gras hydroxylés en position terminale ou subterminale (position ω-1 à ω-4), **caractérisé en ce que**
a) on fait réagir un acide gras ou un dérivé d'acide gras pouvant être hydroxylé en présence d'un système donneur d'électrons selon l'une quelconque des revendications 1 à 5, avec une monooxygénase contenant un cytochrome P450 et de l'oxygène ; et
b) on isole le ou les produits hydroxylés.

9. Procédé selon la revendication 8, **caractérisé en ce que** le dérivé d'acide gras pouvant être hydroxylé en position ω est choisi parmi des acides gras saturés en position terminale, ramifiés ou non ramifiés, ayant plus de 10 atomes de carbone, en particulier, des acides gras en C₁₂₋C₃₀.

10. Procédé selon l'une quelconque des revendications 6 à 9, **caractérisé en ce que** l'enzyme est une monooxygénase contenant un cytochrome P450 choisie parmi :
a) l'enzyme de type sauvage que l'on peut isoler du Bacillus megaterium (DSM 32T) ; ou
b) un mutant de l'enzyme de type sauvage que l'on peut obtenir par substitution d'acide aminé dans au moins l'une des positions 26, 47, 72, 74, 87, 188 et 354 (SEQ ID n° 35).

11. Procédé selon la revendication 10, **caractérisé en ce que** l'on utilise un mutant ponctuel qui est choisi parmi F87A, F87V, L188K, V26T, R47F, S72G, A74G et M354T.

12. Procédé selon la revendication 10, **caractérisé en ce que** le mutant en position 87 présente la mutation F87A ou F87V et au moins une autre des mutations suivantes : L188K, A74G, R47F et V26T.

13. Procédé selon l'une quelconque des revendications 8 à 12, **caractérisé en ce que** le système donneur d'électrons est le système zinc/sépulcrate de Co(III).

14. Procédé selon l'une quelconque des revendications 8 à 13, **caractérisé en ce que** l'on effectue au moins l'étape a) en présence d'ions chlorure.

15. Procédé selon l'une quelconque des revendications 8 à 14, **caractérisé en ce que** l'on effectue au moins l'étape a) en présence d'un enzyme clivant le peroxyde d'hydrogène.

16. Bioréacteur pour utilisation dans la fabrication d'acides gras hydroxylés en position ω, **caractérisé par** une monooxygénase immobilisée et un système donneur d'électrons selon l'une quelconque des revendications 1 à 5 dans le milieu réactionnel liquide.

17. Procédé de détection pour des monooxygénases d'acides gras, **caractérisé en ce que**
a) on incube un analyte, dans lequel on suppose une activité enzymatique, avec un acide gras ou un dérivé d'acide gras pouvant être hydroxylé en position ω, qui porte un chromophore ou un fluorophore terminal clivable, en présence d'un système donneur d'électrons selon l'une quelconque des revendications 1 à 5 ; et
b) on détermine le clivage du chromophore ou du fluorophore par voie qualitative ou quantitative.

18. Procédé selon la revendication 17, **caractérisé en ce que** l'on effectue la réaction en présence d'un enzyme clivant le peroxyde d'hydrogène et, éventuellement, en présence d'ions chlorure.

19. Trousse d'essai comprenant un système donneur d'électrons selon l'une quelconque des revendications 1 à 5.
